# EUROPEAN PATENT SPECIFICATION

(11) **EP 1 114 147 B1**
(45) Date of publication and mention of the grant of the patent: **15.11.2006**
(21) Application number: 99944276.7
(22) Date of filing: 17.09.1999
(51) Int. Cl.: C12N 9/24

(54) **A 2,6-BETA-D-FRUCTAN HYDROLASE ENZYME AND PROCESSES FOR USING THE ENZYME**
2,6-BETA-D-FRUKTANHYDROLASE ENZYM UND VERFAHREN ZU DESSEN VERWENDUNG
2,6-BETA-D-FRUCTOSANE HYDROLASE ET UTILISATIONS DE LADITE ENZYME

(30) Priority: 18.09.1998 DK 117398; 09.12.1998 DK 162398
(43) Date of publication of application: 11.07.2001
(73) Proprietor: Novozymes A/S, 2880 Bagsvaerd (DK)
(72) Inventor: M LLER, S ren, 2880 Bagsv rd (DK); JOHANSEN, Charlotte, 2880 Bagsv rd (DK); SCHÄFER, Thomas, 2880B agsv rd (DK); STERGAARD, Peter, Rahbek, 2880 Bagsv rd (DK); HOECK, Lisbeth, Hedegaard, 2880 Bagsv rd (DK)
(86) International application number: PCT/DK1999/000495
(87) International publication number: WO 2000/017331

(56) References cited:
- AT-B- 398 435
- DATABASE EMBL, Y12619, 16 October 1997 MIASNIKOV A.M.: 'Characterization of anovel endo-levonase from Bacillus sp. L7 and it's gene', XP002944376 & FEMS MICROBIOLOGY LETTERS, vol. 154, 1997, pages 23 - 28
- CHEMICAL ABSTRACTS, vol. 108, no. 11, 14 March 1988, Columbus, Ohio, US; abstract no. 90537F, IGARASHI TAKESHI ET AL.: 'Purification and Characterization of levanase from actinomyces viscosus ATCC 19246' XP002944380 & INFECT. IMMUN., vol. 55, no. 12, 1987, pages 3001 - 3005
- A. CHAUDHARY ET AL.: 'Levanases for control of slime in paper manufacture' BIOTECHNOLOGY ADVANCES, vol. 16, no. 5/6, 1998, pages 899 - 912, XP004138781
- CHEMICAL ABSTRACTS, vol. 124, no. 23, 1996, Columbus, Ohio, US; abstract no. 124:310895U, CHAUDHARY ANITA ET AL.: 'Purification and properties of levanase from Rhodotorual sp.' XP002925194 & J. BIOTECHNOL., vol. 46, no. 1, 1996, (ENG), pages 55 - 61
- CHEMICAL ABSTRACTS, vol. 129, no. 25, 1998, Columbus, Ohio, US; abstract no. 129:332274W, CHAUDHARY A. ET AL.: 'Levanases for control of slime in paper manufacture' XP002925195 & BIOTECHNOL. ADV., vol. 16, no. 5/6, pages 899 - 912

## Description

### TECHNICAL FIELD

The present invention relates to isolated polypeptides having 2,6-β-D-fructan hydrolase activity and isolated nucleic acid sequences encoding the polypeptides. The invention also relates to nucleic acid constructs, vectors, and host cells comprising the nucleic acid sequences as well as methods for producing and using the polypeptides as well as compositions comprising the polypeptides and the use thereof.

### BACKGROUND ART

2,6-β-D-fructans, such as levans of bacterial origin and phleins of plant origin, are substantially β-2,6-fructose polysaccharides consisting of a variable number of fructose units combined by β-(2→6)-glycosidic linkages.

In phlein, β-2,1 branching points also exist depending on the plant origin. Many plants, in particular grasses, store phlein as reserve polysaccharides in stems and leaves. As grasses are ubiquitous plants, phlein is an attractive resource as it is available in almost unlimited amounts.

Levans originate from bacteria. In nutrient limited ecosystems, bacteria have a marked tendency to adhere to surfaces and initiate the formation of a biofilm. A biofilm is a community of microbes, embedded in an organic polymer matrix, adhering to a surface. In natural and industrial ecosystems, especially in nutrient limited systems, biofilms will predominate and cause problems as increased frictional resistance to fluids in water conduits and on ship hulls (fouling), decreased heat transfer from heat exchangers, corrosion of metallic substrates and contamination in the food and biotechnology industry. Biofilms are also a severe problem in medical science and industry causing dental plaque, contaminated endoscopes and contact lenses, prosthetic device colonization and biofilm formation on medical implants.

A biofilm is a collection of microcolonies, typically with water channels in between, and an assortment of cells and extracellular polymers (polysaccharides, glycoproteins, proteins). Bacterial extracellular polysaccharides are composed of homo- and heteropolysaccharides of particularly glucose, fucose, mannose, galactose, fructose, pyruvate, mannuronic acid or glucuronic acid based complexes. The different bonds between the saccharides give rise to a multitude of different polysaccharides including levans, polymannans, dextrans, cellulose, amylopectin, glycogen and alginate.

Bacteria growing in biofilms are more resistant to antibiotics and disinfectants than planktonic cells and the resistance increases with the age of the biofilm. Bacterial biofilm also exhibits increased physical resistance towards desiccation, extreme temperatures or light. As mentioned, biofilm formation causes industrial, environmental and medical problems and the difficulties in cleaning and disinfection of bacterial biofilm with chemicals is a major concern in many industries. Furthermore, the trend towards milder disinfection and cleaning compositions may increase the insufficient cleaning of surfaces covered with biofilm.

Levanases are known e.g from *Fuchs et al.* [*Fuchs A, DeBruijn J M and Niedeveld C J;* Antonie van Leeuwenhoek, 1985 **52** (3) 333-343]. Levanases from bacteria have been reported such as from *Arthrobacter* species strain 7 by Zelikson R. and Hestrin S., Biochemical Journal (1961), 79 page 71-79, from S. *salivarius* by Takahishi N, Mizuno F, Takamori K (1983) Infection and Immunicity vol 42, pp 231-236 and from *A. viscosus* by Igarashi T., Takahashi M., Yamamoto A., Etoh Y., Takamori K., (1987), Infection and immunity, Vol 55, pp. 3001-3005.

A levanase from *Bacillus* sp. No. L7 was reported by Miasnikov A.N. (Characterization of a novel endo levanase and its gene from bacillus sp. L7; FEMS Microbiology Letters, 1997, vol. 154, pp. 23-28). An application of a Bacillus sp. No. 7 endo-levanase is described in WO 99/31020. A levanase from *Bacillus polymyxa* was reported by Bezzate et al. in J. Bacteriol. (1994), 176(8), 2177-83. This Bacillus strain was later reclassified as *Paenibacillus polymyxa*: Carol Ash et al., Antonie van Leeuwenhoek, 64, pp 253-260, 1993-4. The strain *paenibacillus amylolyticus* is known from Osamu Shida et al., Int. J. Bateriol., 47, pp 299-306, 1997. Further in Naummoff D.G., FEMS Microbiol. lett., 164, pp 227-228, 1997 a comparison of 7 specific levanses is given concluding that some regions in these levanases are conserved.

US 3,773,623 describes a composition for treating slime in industrial waters with dead cells of the yeast *Rhodotorula sp.* having levan hydrolase activity

WO 97/01669 describes a method to remove water binders such as slime on a press felt by spraying enzymes onto or into the press felt.

EP 129315 A1 describes a separately packed two component biocidal system.

A process for the manufacture of fructose is described in JP 52136929 A and JP 52136928 A.

US 4,927,757 describes the manufacture of fructose by use of a fructosyl transferase and a levanase.

KR 9301882 B describes the manufacture of a fructo-oligosaccharide preparation by treating a sucrose containing medium with a levanase.

### SUMMARY OF THE INVENTION

The present invention relates to an isolated polypeptide having 2,6-β-D-fructan hydrolases activity and having an amino acid sequence which has at least 90%, preferably at least 95%, more preferably 100% identity with amino acids 32 to 923 for the mature polypeptide of SEQ ID NO:1.

The present invention also relates to isolated nucleic acid sequences encoding the polypeptides and to nucleic acid constructs, vectors, and host cells comprising the nucleic acid sequences as well as methods for producing and using the polypeptides. Also the invention relates to a process for removing a microbial biofilm on a surface comprising contacting said biofilm with an endo-2,6-β-D-fructan hydrolase in an aqueous medium.

### BRIEF DESCRIPTION OF THE DRAWINGS

Figure 1 shows a measurement of the molecular weight of the 2,6-β-D-fructan hydrolase of the mature part of SEQ ID No:1 compared to a series of standards as a result of SDS-PAGE analysis.
Figure 2 shows the dose-response relation between dosed 2,6-β-D-fructan hydrolase of the mature part of SEQ ID No:1 and the response in the LRU assay.
Figure 3 shows the relation between activity of the 2,6-β-D-fructan hydrolase of the mature part of SEQ ID No:1 and the pH in the LRU assay medium at 37 °C.
Figure 4 shows the relation between activity of the 2,6-β-D-fructan hydrolase of the mature part of SEQ ID No:1 and the temperature in the LRU assay medium at pH 6.5.
Figure 5 shows microscopy photos of steel surfaces with biofilm, cleaned with or without the 2,6-β-D-fructan hydrolase of the mature part of SEQ ID No:1.
Figure 6 shows the number of removed cells from a biofilm on a steel surface when cleaned with i) no enzyme, II) 100 ppm Bio-Cip membrane, III) 1000 ppm Bio-Cip membrane and IV) 100 ppm Bio-Cip membrane and 10 mg/L 2,6-β-D-fructan hydrolase of the mature part of SEQ ID No:1.
Figure 7 shows activity of 2,6-β-D-fructan hydrolase in diluted tooth pastes.
Figure 8 shows activity of 2,6-β-D-fructan hydrolase undiluted tooth pastes.

### DETAILED DISCLOSURE OF THE INVENTION

The present invention provides novel enzymes, which hydrolyzes 2,6-β-D-fructo-furanosidic linkages in 2,6-β-D-fructans (levans or phleins). Accordingly the enzymes may be classified as a 2,6-β-D-fructan hydrolase or a levanase or a phleinase or a fructan β-fructosidase.

### Definitions

The term, "an isolated polypeptide" or "isolated 2,6-β-D-fructan hydrolase" as used herein about the enzyme of the invention, is a 2, 6-β-D-fructan hydrolase or 2,6-β-D-fructan hydrolase part, which is at least about 20 % pure, preferably at least about 40% pure, more preferably about 60% pure, even more preferably about 80% pure, most preferably about 90% pure, and even most preferably about 95% pure, as determined by SDS-PAGE.

The term "isolated polypeptide" may alternatively be termed "purified polypeptide".

The term "2,6-β-D-fructan hydrolase encoding part" as used herein in connection with a DNA sequence means the region of the DNA sequence which corresponds to the region which is translated into a polypeptide sequence. The translated polypeptide may comprise, in addition to the mature sequence exhibiting 2,6-β-D-fructan hydrolase activity, an N-terminal signal sequence. The signal sequence generally guides the secretion of the polypeptide. For further information see (Stryer, L., "Biochemistry" W.H., Freeman and Company/New York, ISBN 0-7167-1920-7). In the present context the term "2,6-β-D-fructan hydrolase encoding part" is intended to cover the translated polypeptide and the mature part thereof.

The term 2,6-β-D-fructan hydrolase as used herein is defined according to the Enzyme classification (EC), as having the EC-number: EC 3.2.1.65 in accordance with *Recommendations (1992) of the Nomenclature Committee of the International Union of Biochemistry and Molecular Biology,* Academic Press, Inc., 1992. The term 2,6-β-D-fructan hydrolase alternatively be termed levanase.

The term "surface" as used herein relates to any surface which is essentially non-permeable to microorganisms. Examples of surfaces are surfaces made from metal, e.g. stainless steel alloys, plastics/synthetic polymers, rubber, board, glass, wood, paper, textile, concrete, rock, marble, gypsum and ceramic materials which optionally may be coated, e.g. with paint, enamel, polymers and the like. The surface may however also be of biological origin such as mucous membranes, skin, teeth, hair, nails etc.

2,6-β-D-fructan hydrolases activity as used herein is defined as the ability of an enzyme of hydrolyzing 2,6-β-D-fructo-furanosidic linkages in 2,6-β-D-fructans. A measure of 2,6-β-D-fructan hydrolases activity is a Levan Reducing sugar Unit (LRU), where 1 LRU is defined as the amount of enzyme that generates an amount of reducing groups in a levan substrate equivalent to 1 µmol fructose per minute. The conditions for measuring 2,6-β-D-fructan hydrolases activity is given in the "Materials and methods" section, *infra.*

### The 2,6-β-D-fructan hydrolase

The isolated polypeptide provided by the invention is a polypeptide obtained from a strain of *Paenibacillus* exhibiting 2,6-β-D-fructan hydrolase activity.

### Properties

In a preferred embodiment the isolated polypeptide is further characterized by having one or more of the following properties:
a) a 2,6-β-D-fructan hydrolase activity optimum in the pH range of 3.5-9.5, measured at 37°C;
b) a molecular mass greater or equal to about 88 kDa;
c) a 2,6-β-D-fructan hydrolase activity optimum in the temperature range of 20-70°C;

A preferred pH-activity optimum range is from about 3.5 to about 8.5. A more preferred pH-activity optimum range is from about 5.5 to about 7.5, while a most preferred range is from about 6.5 to about 6.75 measured at in the 2,6-β-D-fructan hydrolase assay at 37°C as described *vide infra.*

Polypeptides of the invention is surprisingly quite large. Accordingly the molecular weight of a polypeptide of the invention greater or equal to about 88 as determined by SDS-PAGE gel electrophoresis as described in Sambrook et al.; ***Molecular Cloning: A Laboratory Manual***; 1989,; Cold Spring Harbor Lab.; Cold Spring Harbor; NY.

A preferred temperature-activity optimum range is from about 25°C to about 55°C, while a most preferred temperature-activity optimum range is from about 35°C to about 40°C, e.g. about 37°C, measured at in the 2,6-β-D-fructan hydrolase assay at pH 6.5 as described *vide infra.*

Preferred substrates for a polypeptide having 2,6-β-D-fructan hydrolase activity of the invention are compounds comprising 2,6-fructose furanosidic bonds, such as levans and phleins specifically, in which, the 2,6-β-D-fructan hydrolases cleave β-2,6-fructose furanosidic bonds.

The polypeptides of the present invention have at least 90%, and most preferably at least 100% of the 2,6-β-D-fructan hydrolase activity of the mature polypeptide of SEQ ID No:1.

### Structure

In further preferred embodiments, the isolated polypeptide is selected from one of the following groups:
a) An isolated polypeptide encoded by the nucleotide sequence inserted into plasmid pSJ1678 present in *E. coli* DSM 12406
b) An isolated polypeptide having 2,6-β-D-fructan hydrolase activity and having an amino acid sequence which has a degree of identity to amino acids 32 to 923 of SEQ ID NO: 1, (herein after "mature polypeptide") of at least 90%, even more preferably about at least 95% and most preferably about at least 97% (herein after "homologous polypeptides"). Preferred homologous polypeptides have an amino acid sequence which differs by five amino acids, preferably by four amino acids, more preferably by three amino acids, even more preferably by two amino acids, and most preferably by one amino acid from amino acids of said sequences.
c) A polypeptide comprising the amino acid sequence of SEQ ID No. 1 having 2,6-β-D-fructan hydrolase activity, preferably comprising the amino acid sequence of the mature polypeptide.
d) An isolated polypeptide comprising an allelic variant having an amino acid sequence which has at least 90%, preferably at least 95%, more preferably 100% identity with amino acids 32 to 923 for the mature polypeptide of SEQ ID NO:1 having 2,6-β-D-fructan hydrolase activity, preferably comprising the amino acid sequence of an allelic variant of the mature polypeptide.
e) An isolated polypeptide consisting of the amino acid sequence of SEQ ID No. 1, preferably consisting of the amino acid sequence of the mature polypeptide.
f) An isolated polypeptide consisting of an allelic variant having an amino acid sequence which has at least 90%, preferably at least 95%, more preferably 100% identity with amino acids 32 to 923 for the mature polypeptide of SEQ ID NO:1, preferably consisting of the amino acid sequence of an allelic variant of the mature polypeptide.
g) A fragment of the polypeptide of SEQ ID No:1 having an amino acid sequence which has at least 90%, preferably at least 95%, more preferably 100% identity with amino acids 32 to 923 for the mature polypeptide of SEQ ID NO:1 having 2,6-β-D-fructan hydrolase activity.

An allelic variant denotes any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

A fragment of a polypeptide of SEQ ID No:1 having 2,6-β-D-fructan hydrolase activity is a polypeptide having one or more amino acids deleted from the amino and/or carboxyl terminus of this amino acid sequence. It is presently contemplated that the first approximately 400 amino acids in the N-terminal of mature polypeptides having 2,6-β-D-fructan hydrolase activity from *Paenibacillus* strains constitutes one or more putative levan binding domains. It is also contemplated that for some of these polypeptides, e.g. from *Paenibacillus pabuli,* an additional putative levan binding domain are present in the C-terminal of the polypeptide. Accordingly 2,6-β-D-fructan hydrolases of the invention mat comprise one or more levan binding domains.

The amino acids in position 1-31 in SEQ ID No:1 are predicted to be signal peptides, whereas the amino acids in position 32 to 923 are predicted to be the mature 2,6-β-D-fructan hydrolase. The prediction was made by using the SignalP neural network computer program described by Nielsen H., Engelbrecht J., Brunak S., von Heijne G.; ***Identification of prokaryotic and eukaryotic signal peptides and prediction of their cleavage sites**;* Protein Engineering; 1997; vol. 10, pp. 1-6. However, surprisingly when the polypetide of SEQ ID No:1 was purified from a strain of *Bacillus subtilis* the N-terminal sequence was found to be different than the one predicted using SignalP neural network computer program. Accordingly a most preferred polypeptide or an allelic variant or a fracment thereof has the sequence of amino acids 54 to 923 of SEC ID No:1 (see examples).

For purposes of the present invention, the degree of homology (identity) between two protein sequences is determined by the Clustal method (Thompson, J.D., Higgins, D.G., and Gibson, T.J. , (1994), Nucleic Acids research 22, 4673-4680) with an PAM250 residue table, and the default settings of the Megalign program in the Lasergene package (DNAstart Inc., 1228 South Park Street, Madison, Wisconsin 53715). The settings for multiple alignment are; gap penalty of 10, and a gap length penalty of 10 while the pairwise alignment parameters are gap penalty of 3, Ktuple of 1, windows=5 and diagonals=5.

In an additional preferred embodiment the polypeptide is a variant of the polypeptide having an amino acid sequence of SEQ ID No:1 comprising a substitution, deletion, and/or insertion of one or more amino acids.

The amino acid sequences of the variant polypeptide may differ from the amino acid sequence of SEQ ID No:1 or the mature polypeptides thereof by an insertion or deletion of one or more amino acid residues and/or the substitution of one or more amino acid residues by different amino acid residues. Preferably, amino acid changes are of a minor nature, that is conservative amino acid substitutions that do not significantly affect the folding and/or activity of the protein; small deletions, typically of one to about 30 amino acids; small amino- or carboxyl-terminal extensions, such as an amino-terminal methionine residue; a small linker peptide of up to about 20-25 residues; or a small extension that facilitates purification by changing net charge or another function, such as a polyhistidine tract, an antigenic epitope or a binding domain.

Examples of conservative substitutions are within the group of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions which do not generally alter the specific activity are known in the art and are described, for example, by H. Neurath and R.L. Hill, 1979, In, The Proteins, Academic Press, New York. The most commonly occurring exchanges are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly as well as these in reverse.

As defined herein, an "isolated" polypeptide is a polypeptide which is essentially free of other non-[enzyme] polypeptides, e.g., at least about 20% pure, preferably at least about 40% pure, more preferably about 60% pure, even more preferably about 80% pure, most preferably about 90% pure, and even most preferably about 95% pure, as determined by SDS-PAGE.

Polypeptides encoded by nucleic acid sequences of the present invention also include fused polypeptides or cleavable fusion polypeptides in which another polypeptide is fused at the N-terminus or the C-terminus of the polypeptide or fragment thereof. A fused polypeptide is produced by fusing a nucleic acid sequence (or a portion thereof) encoding another polypeptide to a nucleic acid sequence (or a portion thereof) of the present invention. Techniques for producing fusion polypeptides are known in the art, and include ligating the coding sequences encoding the polypeptides so that they are in frame and that expression of the fused polypeptide is under control of the same promoter(s) and terminator.

### Nucleic acid sequences

The invention also relates to isolated nucleic acid sequences which encode a polypeptides of the present invention. The isolated nucleic acid sequence is preferably selected from one of the following groups:
a) The 2,6-β-D-fructan hydrolase polypeptide encoding parts of the nucleic acid sequences contained in plasmids pSJ1678 that are contained in *E. coli* strain DSM 12406.
b) The mature 2,6-β-D-fructan hydrolase polypeptide encoding parts of the nucleic acid sequences contained in plasmids pSJ1678 that are contained in *E. coli* strain DSM 12406.
c) The nucleic acid sequence shown in SEQ ID No:2 or more preferred the sequence of SEQ ID NO: 2 encoding the mature 2,6-β-D-fructan hydrolase polypeptides.
d) An isolated nucleic acid sequence which encode a 2,6-β-D-fructan hydrolase polypeptide having the amino acid sequence of SEQ ID NO:1 or the mature polypeptides thereof, which differ from SEQ ID NO:1 by virtue of the degeneracy of the genetic code.
e) An isolated subsequence of SEQ ID No:2 which encode fragments of SEQ ID NO:1 having an amino acid sequence which has at least 90%, preferably at least 95%, more preferably 100% identity with amino acids 32 to 923 for the mature polypeptide of SEQ ID NO:1 that have 2,6-β-D-fructan hydrolase activity. A subsequence of No:2 is a nucleic acid sequence encompassed by No:2 except that one or more nucleotides from the 5' and/or 3' end have been deleted. Preferably, a subsequence contains at least 2100 nucleotides, more preferably at least 1800 nucleotides, and most preferably at least 1500 nucleotides.
f) An isolated mutant nucleic acid sequence comprising at least one mutation in the mature polypeptide coding sequence of No:2, in which the mutant nucleic acid sequence encodes a polypeptide which consists of amino acids 32-923 of SEQ ID NO:1.
g) An isolated nucleic acid sequence encoding a polypeptide of the present invention, which hybridize under high stringency conditions, and most preferably very high stringency conditions with a nucleic acid probe which hybridizes under the same conditions with the nucleic acid sequence of SEQ ID No:2 or its complementary strands; or allelic variants and subsequences thereof (J. Sambrook, E.F. Fritsch, and T. Maniatus, 1989, Molecular Cloning, A Laboratory Manual, 2d edition, Cold Spring Harbor, New York), as defined herein.
h) An isolated nucleic acid sequences produced by (a) hybridizing a DNA under high, or very high stringency conditions with (i) nucleotides 94 to 2769 of SEQ ID No:2, (ii) the cDNA sequence comprised or contained in nucleotides 94 to 2769 of SEQ ID No:2 or (iii) a complementary strand of (i) or (ii); and (b) isolating the nucleic acid sequence.

The techniques used to isolate or clone a nucleic acid sequence encoding a polypeptide are known in the art and include isolation from genomic DNA, preparation from cDNA, or a combination thereof. The cloning of the nucleic acid sequences of the present invention from such genomic DNA can be effected, e.g., by using the well known polymerase chain reaction (PCR) or antibody screening of expression libraries to detect cloned DNA fragments with shared structural features. See, e.g., Innis et al., 1990, PCR: A Guide to Methods and Application, Academic Press, New York. Other nucleic acid amplification procedures such as ligase chain reaction (LCR), ligated activated transcription (LAT) and nucleic acid sequence-based amplification (NASBA) may be used. The nucleic acid sequence may be cloned from a strain of Paenibacillus, or another or related organism and thus, for example, may be an allelic or species variant of the polypeptide encoding region of the nucleic acid sequence.

The term "isolated nucleic acid sequence" as used herein refers to a nucleic acid sequence which is essentially free of other nucleic acid sequences, e.g., at least about 20% pure, preferably at least about 40% pure, more preferably at least about 60% pure, even more preferably at least about 80% pure, and most preferably at least about 90% pure as determined by agarose electrophoresis. For example, an isolated nucleic acid sequence can be obtained by standard cloning procedures used in genetic engineering to relocate the nucleic acid sequence from its natural location to a different site where it will be reproduced. The cloning procedures may involve excision and isolation of a desired nucleic acid fragment comprising the nucleic acid sequence encoding the polypeptide, insertion of the fragment into a vector molecule, and incorporation of the recombinant vector into a host cell where multiple copies or clones of the nucleic acid sequence will be replicated. The nucleic acid sequence may be of genomic, cDNA, RNA, semisynthetic, synthetic origin, or any combinations thereof.

For purposes of the present invention, the degree of homology (identity) between two nucleic acid sequences is determined by the Clustal method (Thompson, J.D., Higgins, D.G., and Gibson, T.J. , (1994), Nucleic Acids research 22, 4673-4680) with an PAM250 residue table, and the default settings of the Megalign program in the Lasergene package (DNAstart Inc., 1228 South Park Street, Madison, Wisconsin 53715). The settings for multiple alignment are; gap penalty of 10, and a gap length penalty of 10 while the pairwise alignment parameters are gap penalty of 5 and Ktuple of 2, windows=4 and diagonals=4.

The nucleic acids in position 1-93 in SEQ ID No:2, is predicted to encode signal peptides, whereas the nucleic acids in position 94 to 2769 of SEQ ID No:2, is predicted to encode mature 2,6-β-D-fructan hydrolase. The prediction was made by using the SignalP neural network computer program described by Nielsen H., Engelbrecht J., Brunak S., von Heijne G.; ***Identification of prokaryotic and eukaryotic signal peptides and prediction of their cleavage sites***; Protein Engineering; 1997; vol. 10, pp. 1-6.

Modification of a nucleic acid sequence encoding a polypeptide of the present invention may be necessary for the synthesis of polypeptides substantially similar to the polypeptide. The term "substantially similar" to the polypeptide refers to non-naturally occurring forms of the polypeptide. These polypeptides may differ in some engineered way from the polypeptide isolated from its native source, e.g., variants that differ in specific activity, thermostability, pH optimum, or the like. The variant sequence may be constructed on the basis of the nucleic acid sequence presented as the polypeptide encoding part of SEQ ID No:2, e.g., a subsequence thereof, and/or by introduction of nucleotide substitutions which do not give rise to another amino acid sequence of the polypeptide encoded by the nucleic acid sequence, but which correspond to the codon usage of the host organism intended for production of the enzyme, or by introduction of nucleotide substitutions which may give rise to a different amino acid sequence. For a general description of nucleotide substitution, see, e.g., Ford et al., 1991, Protein Expression and Purification 2: 95-107.

It will be apparent to those skilled in the art that such substitutions can be made outside the regions critical to the function of the molecule and still result in an active polypeptide. Amino acid residues essential to the activity of the polypeptide encoded by the isolated nucleic acid sequence of the invention, and therefore preferably not subject to substitution, may be identified according to procedures known in the art, such as site-directed mutagenesis or alanine-scanning mutagenesis (see, e.g., Cunningham and Wells, 1989, Science 244: 1081-1085). In the latter technique, mutations are introduced at every positively charged residue in the molecule, and the resultant mutant molecules are tested for 2,6-β-D-fructan hydrolase activity to identify amino acid residues that are critical to the activity of the molecule. Sites of substrate-enzyme interaction can also be determined by analysis of the three-dimensional structure as determined by such techniques as nuclear magnetic resonance analysis, crystallography or photoaffinity labelling (see, e.g., de Vos et al., 1992, Science 255: 306-312; Smith et al., 1992, Journal of Molecular Biology 224: 899-904; Wlodaver et al., 1992, FEBS Letters 309: 59-64).

The nucleic acid sequence of SEQ ID No:2 or a subsequence thereof, as well as the amino acid sequences of SEQ ID No:1 or a fragment thereof, may be used to design a nucleic acid probe to identify and clone DNA encoding polypeptides having 2,6-β-D-fructan hydrolase activity from strains of different genera or species according to methods well known in the art. In particular, such probes can be used for hybridization with the genomic or cDNA of the genus or species of interest, following standard Southern blotting procedures, in order to identify and isolate the corresponding gene therein. Such probes can be considerably shorter than the entire sequence, but should be at least 15, preferably at least 25, and more preferably at least 35 nucleotides in length. Longer probes can also be used. Both DNA and RNA probes can be used. The probes are typically labeled for detecting the corresponding gene (for example, with ³²P, ³H, ³⁵S, biotin, or avidin). Such probes are encompassed by the present invention.

Thus, a genomic DNA or cDNA library prepared from such other organisms may be screened for DNA which hybridizes with the probes described above and which encodes a polypeptide having 2,6-β-D-fructan hydrolase activity. Genomic or other DNA from such other organisms may be separated by agarose or polyacrylamide gel electrophoresis, or other separation techniques. DNA from the libraries or the separated DNA may be transferred to and immobilized on nitrocellulose or other suitable carrier material. In order to identify a clone or DNA which is homologous with SEQ ID No:2 or a subsequence thereof, the carrier material is used in a Southern blot. For purposes of the present invention, hybridization indicates that the nucleic acid sequence hybridizes to a labeled nucleic acid probe corresponding to the nucleic acid sequences shown in SEQ ID No:2 its complementary strands, or a subsequence thereof, under high to very high stringency conditions. Molecules to which the nucleic acid probe hybridizes under these conditions are detected using X-ray film.

In a preferred embodiment, the nucleic acid probe is a nucleic acid sequence which encodes the polypeptide of SEQ ID No:1 or a subsequence thereof. In another preferred embodiment, the nucleic acid probe is SEQ ID No:2. In another preferred embodiment, the nucleic acid probe is the mature polypeptide coding region of SEQ ID No:2. In another preferred embodiment, the nucleic acid probe is the nucleic acid sequence contained in plasmid pSJ1678 which is contained in *E. coli* strain DSM 12406, wherein the nucleic acid sequence encodes a polypeptide having 2,6-β-D-fructan hydrolase activity. In another preferred embodiment, the nucleic acid probe is the mature polypeptide coding region contained in plasmid pSJ1678 which is contained in *E. coli* strain DSM 12406.

For long probes of at least 100 nucleotides in length, very low to very high stringency conditions are defined as prehybridization and hybridization at 42°C in 5X SSPE, 0.3% SDS, 200 µg/ml sheared and denatured salmon sperm DNA, and either 25% formamide for very low and low stringencies, 35% formamide for medium and medium-high stringencies, or 50% formamide for high and very high stringencies, following standard Southern blotting procedures.

For long probes of at least 100 nucleotides in length, the carrier material is finally washed three times each for 15 minutes using 2 x SSC, 0.2% SDS preferably at least at 45°C (very low stringency), more preferably at least at 50°C (low stringency), more preferably at least at 55°C (medium stringency), more preferably at least at 60°C (medium-high stringency), even more preferably at least at 65°C (high stringency), and most preferably at least at 70°C (very high stringency).

For short probes which are about 15 nucleotides to about 70 nucleotides in length, stringency conditions are defined as prehybridization, hybridization, and washing post-hybridization at 5°C to 10°C below the calculated Tₘ using the calculation according to Bolton and McCarthy (1962, *Proceedings of the National Academy of Sciences USA* 48:1390) in 0.9 M NaCl, 0.09 M Tris-HCl pH 7.6, 6 mM EDTA, 0.5% NP-40, 1X Denhardt's solution, 1 mM sodium pyrophosphate, 1 mM sodium monobasic phosphate, 0.1 mM ATP, and 0.2 mg of yeast RNA per ml following standard Southern blotting procedures.

For short probes which are about 15 nucleotides to about 70 nucleotides in length, the carrier material is washed once in 6X SCC plus 0.1% SDS for 15 minutes and twice each for 15 minutes using 6X SSC at 5°C to 10°C below the calculated Tₘ.

### Microbial sources

A polypeptide of the present invention may be obtained from microorganisms of any genus. For purposes of the present invention, the term "obtained from" as used herein in connection with a given source shall mean that the polypeptide encoded by the nucleic acid sequence is produced by the source or by a cell in which the nucleic acid sequence from the source has been inserted. In a preferred embodiment, the polypeptide is secreted extracellularly.

The 2,6-β-D-fructan hydrolase as shown in SEQ ID No:1 are obtained from strains of *Paenibacillus.* More specifically the 2,6-β-D-fructan hydrolase producing strains is Paenibacillus amylolyticus,. A sample of this microorganism was deposited by the applicant according to the Budapest Treaty on the International Recognition of the Deposits of Microorganisms for the Purpose of Patent Procedures at Deutsche Sammlung von Mikroorganismen und Zellkulturen GmbH, under under Accession No. DSM 12458 for the *Paenibacillus amylolyticus.* The strain was acquired from commercially available strain collections.

These deposited examples of isolated substantially pure biological cultures of *Paenibacillus* (any mutant of said *Paenibacillus* strains having retained the 2,6-β-D-fructan hydrolase encoding capability is considered to be included in the present invention), was used to obtain the DNA sequences encoding 2,6-β-D-fructan hydrolases of the invention.

In accordance with Jones D. and Collins M.D.; ***Bergey's manual of Systematic Bacteriology;*** Sneath P.H.A., Mair N.S., Sharpe M.E., Holt J.G. (Eds.); Williams & Wilkins; Baltimore/London; 1986, Vol.2, p. 1105-1139, the microorganisms of the invention are aerobic endospore forming bacterias belonging to the genus *Paenibacillus.* The optimum temperature for growth is about 30-40°C. The microorganism is a common gram positive soil bacteria.

### Methods for Producing Mutant Nucleic Acid Sequences

The present invention further relates to methods for producing a mutant nucleic acid sequence, comprising introducing at least one mutation into the mature polypeptide coding sequence of SEQ ID No:2 or a subsequence thereof, wherein the mutant nucleic acid sequence encodes a polypeptide which consists of amino acids 32 to 923 of SEQ ID No:1 or a fragment thereof which has 2,6-β-D-fructan hydrolase activity.

The introduction of a mutation into the nucleic acid sequence to exchange one nucleotide for another nucleotide may be accomplished by site-directed mutagenesis using any of the methods known in the art. Particularly useful is the procedure which utilizes a supercoiled, double stranded DNA vector with an insert of interest and two synthetic primers containing the desired mutation. The oligonucleotide primers, each complementary to opposite strands of the vector, extend during temperature cycling by means of Pfu DNA polymerase. On incorporation of the primers, a mutated plasmid containing staggered nicks is generated. Following temperature cycling, the product is treated with *Dpn*I which is specific for methylated and hemimethylated DNA to digest the parental DNA template and to select for mutation-containing synthesized DNA. Other procedures known in the art may also be used.

### Nucleic Acid Constructs

The present invention also relates to nucleic acid constructs comprising a nucleic acid sequence of the present invention operably linked to one or more control sequences which direct the expression of the coding sequence in a suitable host cell under conditions compatible with the control sequences. Expression will be understood to include any step involved in the production of the polypeptide including, but not limited to, transcription, post-transcriptional modification, translation, post-translational modification, and secretion.

"Nucleic acid construct" is defined herein as a nucleic acid molecule, either single- or double-stranded, which is isolated from a naturally occurring gene or which has been modified to contain segments of nucleic acid combined and juxtaposed in a manner that would not otherwise exist in nature. The term nucleic acid construct is synonymous with the term expression cassette when the nucleic acid construct contains all the control sequences required for expression of a coding sequence of the present invention. The term "coding sequence" is defined herein as a nucleic acid sequence which directly specifies the amino acid sequence of its protein product. The boundaries of the coding sequence are generally determined by a ribosome binding site (prokaryotes) or by the ATG start codon (eukaryotes) located just upstream of the open reading frame at the 5' end of the mRNA and a transcription terminator sequence located just downstream of the open reading frame at the 3' end of the mRNA. A coding sequence can include, but is not limited to, DNA, cDNA, and recombinant nucleic acid sequences.

An isolated nucleic acid sequence encoding a polypeptide of the present invention may be manipulated in a variety of ways to provide for expression of the polypeptide. Manipulation of the nucleic acid sequence prior to its insertion into a vector may be desirable or necessary depending on the expression vector. The techniques for modifying nucleic acid sequences utilizing recombinant DNA methods are well known in the art.

The term "control sequences" is defined herein to include all components which are necessary or advantageous for the expression of a polypeptide of the present invention. Each control sequence may be native or foreign to the nucleic acid sequence encoding the polypeptide. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleic acid sequence encoding a polypeptide. The term "operably linked" is defined herein as a configuration in which a control sequence is appropriately placed at a position relative to the coding sequence of the DNA sequence such that the control sequence directs the expression of a polypeptide.

The control sequence may be an appropriate promoter sequence, a nucleic acid sequence which is recognized by a host cell for expression of the nucleic acid sequence. The promoter sequence contains transcriptional control sequences which mediate the expression of the polypeptide. The promoter may be any nucleic acid sequence which shows transcriptional activity in the host cell of choice including mutant, truncated, and hybrid promoters, and may be obtained from genes encoding extracellular or intracellular polypeptides either homologous or heterologous to the host cell.

Examples of suitable promoters for directing the transcription of the nucleic acid constructs of the present invention, especially in a bacterial host cell, are the promoters obtained from the *E. coli lac* operon, *Streptomyces coelicolor* agarase gene *(dagA), Bacillus subtilis* levansucrase gene *(sacB), Bacillus licheniformis* alpha-amylase gene *(amyL), Bacillus stearothermophilus* maltogenic amylase gene *(amyM), Bacillus amyloliquefaciens* alpha-amylase gene *(amyQ), Bacillus licheniformis* penicillinase gene *(penP), Bacillus subtilis xylA* and *xylB* genes, and prokaryotic beta-lactamase gene (Villa-Kamaroff *et al.,* 1978, *Proceedings of the National Academy of Sciences USA* 75: 3727-3731), as well as the tac promoter (DeBoer et *al.,* 1983, *Proceedings of the National Academy of Sciences USA* 80: 21-25). Further promoters are described in "Useful proteins from recombinant bacteria" in *Scientific American,* 1980, 242: 74-94; and in Sambrook *et al.,* 1989, *supra.*

The control sequence may also be a suitable transcription terminator sequence, a sequence recognized by a host cell to terminate transcription. The terminator sequence is operably linked to the 3' terminus of the nucleic acid sequence encoding the polypeptide. Any terminator which is functional in the host cell of choice may be used in the present invention.

The control sequence may also be a suitable leader sequence, a nontranslated region of an mRNA which is important for translation by the host cell. The leader sequence is operably linked to the 5' terminus of the nucleic acid sequence encoding the polypeptide. Any leader sequence that is functional in the host cell of choice may be used in the present invention.

The control sequence may also be a polyadenylation sequence, a sequence operably linked to the 3' terminus of the nucleic acid sequence and which, when transcribed, is recognized by the host cell as a signal to add polyadenosine residues to transcribed mRNA. Any polyadenylation sequence which is functional in the host cell of choice may be used in the present invention.

The control sequence may also be a signal peptide coding region that codes for an amino acid sequence linked to the amino terminus of a polypeptide and directs the encoded polypeptide into the cell's secretory pathway. The 5' end of the coding sequence of the nucleic acid sequence may inherently contain a signal peptide coding region naturally linked in translation reading frame with the segment of the coding region which encodes the secreted polypeptide. Alternatively, the 5' end of the coding sequence may contain a signal peptide coding region which is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the polypeptide. However, any signal peptide coding region which directs the expressed polypeptide into the secretory pathway of a host cell of choice may be used in the present invention.

Effective signal peptide coding regions for bacterial host cells are the signal peptide coding regions obtained from the genes for *Bacillus* NCIB 11837 maltogenic amylase, *Bacillus stearothermophilus* alpha-amylase, *Bacillus licheniformis* subtilisin, *Bacillus licheniformis* beta-lactamase, *Bacillus stearothermophilus* neutral proteases (*nprT*, *nprS*, *nprM*), and *Bacillus subtilis prsA.* Further signal peptides are described by Simonen and Palva, 1993, *Microbiological Reviews* 57: 109-137.

In a preferred embodiment, the signal peptide coding region is nucleotides 1 to 95 of SEQ ID No:2 which encode amino acids 1 to 31 of SEQ ID NO:1.

The control sequence may also be a propeptide coding region that codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for *Bacillus subtilis* alkaline protease *(aprE), Bacillus subtilis* neutral protease *(nprT), Saccharomyces cerevisiae* alpha-factor, *Rhizomucor miehei* aspartic proteinase, and *Myceliophthora thermophila* laccase (WO 95/33836).

Where both signal peptide and propeptide regions are present at the amino terminus of a polypeptide, the propeptide region is positioned next to the amino terminus of a polypeptide and the signal peptide region is positioned next to the amino terminus of the propeptide region.

It may also be desirable to add regulatory sequences which allow the regulation of the expression of the polypeptide relative to the growth of the host cell. Examples of regulatory systems are those which cause the expression of the gene to be turned on or off in response to a chemical or physical stimulus, including the presence of a regulatory compound. Regulatory systems in prokaryotic systems include the *lac, tac,* and *trp* operator systems.

### Expression Vectors

The present invention also relates to recombinant expression vectors comprising a nucleic acid sequence of the present invention, a promoter, and transcriptional and translational stop signals. The various nucleic acid and control sequences described above may be joined together to produce a recombinant expression vector which may include one or more convenient restriction sites to allow for insertion or substitution of the nucleic acid sequence encoding the polypeptide at such sites. Alternatively, the nucleic acid sequence of the present invention may be expressed by inserting the nucleic acid sequence or a nucleic acid construct comprising the sequence into an appropriate vector for expression. In creating the expression vector, the coding sequence is located in the vector so that the coding sequence is operably linked with the appropriate control sequences for expression.

The recombinant expression vector may be any vector (*e.g.*, a plasmid or virus) which can be conveniently subjected to recombinant DNA procedures and can bring about the expression of the nucleic acid sequence. The choice of the vector will typically depend on the compatibility of the vector with the host cell into which the vector is to be introduced. The vectors may be linear or closed circular plasmids.

The vector may be an autonomously replicating vector, *i.e.*, a vector which exists as an extrachromosomal entity, the replication of which is independent of chromosomal replication, *e.g.*, a plasmid, an extrachromosomal element, a minichromosome, or an artificial chromosome. The vector may contain any means for assuring self-replication. Alternatively, the vector may be one which, when introduced into the host cell, is integrated into the genome and replicated together with the chromosome(s) into which it has been integrated. Furthermore, a single vector or plasmid or two or more vectors or plasmids which together contain the total DNA to be introduced into the genome of the host cell, or a transposon may be used.

The vectors of the present invention preferably contain one or more selectable markers which permit easy selection of transformed cells. A selectable marker is a gene the product of which provides for biocide or viral resistance, resistance to heavy metals, prototrophy to auxotrophs, and the like. Examples of bacterial selectable markers are the *dal* genes from *Bacillus subtilis* or *Bacillus licheniformis,* or markers which confer antibiotic resistance such as ampicillin, kanamycin, chloramphenicol or tetracycline resistance. Suitable markers for yeast host cells are ADE2, HIS3, LEU2, LYS2, MET3, TRP1, and URA3. Selectable markers for use in a filamentous fungal host cell include, but are not limited to, *amdS* (acetamidase), *argB* (ornithine carbamoyltransferase), *bar* (phosphinothricin acetyltransferase), *hygB* (hygromycin phosphotransferase), *niaD* (nitrate reductase), *pyrG* (orotidine-5'-phosphate decarboxylase), *sC* (sulfate adenyltransferase), *trpC* (anthranilate synthase), as well as equivalents thereof. Preferred for use in an *Aspergillus* cell are the *amdS* and *pyrG* genes of *Aspergillus nidulans* or *Aspergillus oryzae* and the *bar* gene of *Streptomyces hygroscopicus.*

The vectors of the present invention preferably contain an element(s) that permits stable integration of the vector into the host cell's genome or autonomous replication of the vector in the cell independent of the genome.

For integration into the host cell genome, the vector may rely on the nucleic acid sequence encoding the polypeptide or any other element of the vector for stable integration of the vector into the genome by homologous or nonhomologous recombination. Alternatively, the vector may contain additional nucleic acid sequences for directing integration by homologous recombination into the genome of the host cell. The additional nucleic acid sequences enable the vector to be integrated into the host cell genome at a precise location(s) in the chromosome(s). To increase the likelihood of integration at a precise location, the integrational elements should preferably contain a sufficient number of nucleic acids, such as 100 to 1,500 base pairs, preferably 400 to 1,500 base pairs, and most preferably 800 to 1,500 base pairs, which are highly homologous with the corresponding target sequence to enhance the probability of homologous recombination. The integrational elements may be any sequence that is homologous with the target sequence in the genome of the host cell. Furthermore, the integrational elements may be non-encoding or encoding nucleic acid sequences. On the other hand, the vector may be integrated into the genome of the host cell by non-homologous recombination.

For autonomous replication, the vector may further comprise an origin of replication enabling the vector to replicate autonomously in the host cell in question. Examples of bacterial origins of replication are the origins of replication of plasmids pBR322, pUC19, pACYC177, and pACYC184 permitting replication in *E. coli,* and pUB110, pE194, pTA1060, and pAMß1 permitting replication in *Bacillus.* Examples of origins of replication for use in a yeast host cell are the 2 micron origin of replication, ARS1, ARS4, the combination of ARS1 and CEN3, and the combination of ARS4 and CEN6. The origin of replication may be one having a mutation which makes its functioning temperature-sensitive in the host cell (see, e.g., Ehrlich, 1978, *Proceedings of the National Academy of Sciences USA* 75: 1433).

More than one copy of a nucleic acid sequence of the present invention may be inserted into the host cell to increase production of the gene product. An increase in the copy number of the nucleic acid sequence can be obtained by integrating at least one additional copy of the sequence into the host cell genome or by including an amplifiable selectable marker gene with the nucleic acid sequence where cells containing amplified copies of the selectable marker gene, and thereby additional copies of the nucleic acid sequence, can be selected for by cultivating the cells in the presence of the appropriate selectable agent.

The procedures used to ligate the elements described above to construct the recombinant expression vectors of the present invention are well known to one skilled in the art (see, *e.g.,* Sambrook *et al.,* 1989, *supra*)*.*

### Host Cells

The present invention also relates to recombinant host cells, comprising a nucleic acid sequence of the invention, which are advantageously used in the recombinant production of the polypeptides. A vector comprising a nucleic acid sequence of the present invention is introduced into a host cell so that the vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be a unicellular microorganism, *e.g.,* a prokaryote, or a non-unicellular microorganism, *e.g.*, a eukaryote.

Useful unicellular cells are bacterial cells such as gram positive bacteria including, but not limited to, a *Bacillus* cell, *e.g., Bacillus alkalophilus, Bacillus amyloliquefaciens, Bacillus brevis, Bacillus circulans, Bacillus clausii, Bacillus coagulans, Bacillus lautus, Bacillus lentus, Bacillus licheniformis, Bacillus megaterium, Bacillus stearothermophilus, Bacillus subtilis,* and *Bacillus thuringiensis;* or a *Streptomyces* cell, *e.g., Streptomyces lividans* or *Streptomyces murinus,* or gram negative bacteria such as *E. coli* and *Pseudomonas* sp. In a preferred embodiment, the bacterial host cell is a *Bacillus lentus, Bacillus licheniformis, Bacillus stearothermophilus,* or *Bacillus subtilis* cell. In another preferred embodiment, the *Bacillus* cell is an alkalophilic *Bacillus.*

The introduction of a vector into a bacterial host cell may, for instance, be effected by protoplast transformation (see, *e.g.,* Chang and Cohen, 1979, *Molecular General Genetics* 168: 111-115), using competent cells (see, *e.g.,* Young and Spizizin, 1961, *Journal of Bacteriology* 81: 823-829, or Dubnau and Davidoff-Abelson, 1971, *Journal of Molecular Biology* 56: 209-221), electroporation (see, e.g., Shigekawa and Dower, 1988, *Biotechniques* 6: 742-751), or conjugation (see, *e.g.*, Koehler and Thorne, 1987, *Journal of Bacteriology* 169: 5771-5278).

The host cell may be a eukaryote, such as a mammalian, insect, plant, or fungal cell.

In a preferred embodiment, the host cell is a fungal cell. "Fungi" as used herein includes the phyla Ascomycota, Basidiomycota, Chytridiomycota, and Zygomycota (as defined by Hawksworth *et al., In, Ainsworth and Bisby's Dictionary of The Fungi,* 8th edition, 1995, CAB International, University Press, Cambridge, UK) as well as the Oomycota (as cited in Hawksworth et *al.,* 1995, *supra,* page 171) and all mitosporic fungi (Hawksworth *et al.*, 1995, *supra*)*.*

In a more preferred embodiment, the fungal host cell is a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in *Biology and Activities of Yeast* (Skinner, F.A., Passmore, S.M., and Davenport, R.R., eds, *Soc. App. Bacteriol. Symposium Series* No. 9, 1980).

In an even more preferred embodiment, the yeast host cell is a *Candida, Hansenula, Kluyveromyces, Pichia, Saccharomyces, Schizosaccharomyces,* or *Yarrowia* cell.

In a most preferred embodiment, the yeast host cell is a *Saccharomyces carlsbergensis, Saccharomyces cerevisiae, Saccharomyces diastaticus, Saccharomyces douglasii, Saccharomyces kluyveri, Saccharomyces norbensis* or *Saccharomyces oviformis* cell. In another most preferred embodiment, the yeast host cell is a *Kluyveromyces lactis* cell. In another most preferred embodiment, the yeast host cell is a *Yarrowia lipolytica* cell.

In another more preferred embodiment, the fungal host cell is a filamentous fungal cell. "Filamentous fungi" include all filamentous forms of the subdivision Eumycota and Oomycota (as defined by Hawksworth et *al.,* 1995, *supra).* The filamentous fungi are characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

In an even more preferred embodiment, the filamentous fungal host cell is a cell of a species of, but not limited to, *Acremonium, Aspergillus, Fusarium, Humicola, Mucor, Myceliophthora, Neurospora, Penicillium, Thielavia, Tolypocladium,* or *Trichoderma.*

In a most preferred embodiment, the filamentous fungal host cell is an *Aspergillus awamori, Aspergillus foetidus, Aspergillus japonicus, Aspergillus nidulans, Aspergillus niger* or *Aspergillus oryzae* cell. In another most preferred embodiment, the filamentous fungal host cell is a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell. In an even most preferred embodiment, the filamentous fungal parent cell is a *Fusarium venenatum* (Nirenberg sp. nov.) cell. In another most preferred embodiment, the filamentous fungal host cell is a *Humicola insolens, Humicola lanuginosa, Mucor miehei, Myceliophthora thermophila, Neurospora crassa, Penicillium purpurogenum, Thielavia terrestris, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* host cells are described in EP 238 023 and Yelton et *al.,* 1984, *Proceedings of the National Academy of Sciences USA* 81: 1470-1474. Suitable methods for transforming *Fusarium* species are described by Malardier et *al.,* 1989, Gene 78: 147-156 and WO 96/00787. Yeast may be transformed using the procedures described by Becker and Guarente, In Abelson, J.N. and Simon, M.I., editors, *Guide to Yeast Genetics and Molecular Biology, Methods in Enzymology,* Volume 194, pp 182-187, Academic Press, Inc., New York; Ito et *al.,* 1983, *Journal of Bacteriology* 153: 163; and Hinnen et *al.,* 1978, *Proceedings of the National Academy of Sciences USA* 75: 1920.

### Methods of Production

The present invention also relates to methods for producing a polypeptide of the present invention comprising (a) cultivating a strain, which in its wild-type form is capable of producing the polypeptide, to produce a supernatant comprising the polypeptide; and (b) recovering the polypeptide. Preferably, the strain is of the genus *Paenibacillus,* and more preferably *Paenibacillus amylolyticus or Paenibacillus pabuli or Paenibacillus macerans.*

The present invention also relates to methods for producing a polypeptide of the present invention comprising (a) cultivating a host cell under conditions conducive for production of the polypeptide; and (b) recovering the polypeptide.

The present invention also relates to methods for producing a polypeptide of the present invention comprising (a) cultivating a host cell under conditions conducive for production of the polypeptide, wherein the host cell comprises a mutant nucleic acid sequence having at least one mutation in the mature polypeptide coding region of SEQ ID No:2, wherein the mutant nucleic acid sequence encodes a polypeptide which consists of amino acids 32 to 923 of SEQ ID No:1, and (b) recovering the polypeptide.

In the production methods of the present invention, the cells are cultivated in a nutrient medium suitable for production of the polypeptide using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.,* in catalogues of the American Type Culture Collection). If the polypeptide is secreted into the nutrient medium, the polypeptide can be recovered directly from the medium. If the polypeptide is not secreted, it can be recovered from cell lysates.

The polypeptides may be detected using methods known in the art that are specific for the polypeptides. These detection methods may include use of specific antibodies, formation of an enzyme product, or disappearance of an enzyme substrate. For example, an enzyme assay may be used to determine the activity of the polypeptide as described herein.

The resulting polypeptide may be recovered by methods known in the art. For example, the polypeptide may be recovered from the nutrient medium by conventional procedures including, but not limited to, centrifugation, filtration, extraction, spray-drying, evaporation, or precipitation.

The polypeptides of the present invention may be purified by a variety of procedures known in the art including, but not limited to, chromatography (*e.g.*, ion exchange, affinity, hydrophobic, chromatofocusing, and size exclusion), electrophoretic procedures (*e.g.*, preparative isoelectric focusing), differential solubility (*e.g.*, ammonium sulfate precipitation), SDS-PAGE, or extraction (see, *e.g., Protein Purification,* J.-C. Janson and Lars Ryden, editors, VCH Publishers, New York, 1989).

### Plants

The present invention also relates to a transgenic plant, plant part, or plant cell which has been transformed with a nucleic acid sequence encoding a polypeptide having 2,6-β-D-fructan hydrolase activity of the present invention so as to express and produce the polypeptide in recoverable quantities. The polypeptide may be recovered from the plant or plant part. Alternatively, the plant or plant part containing the recombinant polypeptide may be used as such for improving the quality of a food or feed, *e.g.*, improving nutritional value, palatability, and rheological properties, or to destroy an antinutritive factor.

The transgenic plant can be dicotyledonous (a dicot) or monocotyledonous (a monocot). Examples of monocot plants are grasses, such as meadow grass (blue grass, Poa), forage grass such as festuca, lolium, temperate grass, such as Agrostis, and cereals, *e.g.*, wheat, oats, rye, barley, rice, sorghum, and maize (corn).

Examples of dicot plants are tobacco, legumes, such as lupins, potato, sugar beet, pea, bean and soybean, and cruciferous plants (family Brassicaceae), such as cauliflower, rape seed, and the closely related model organism *Arabidopsis thaliana.*

Examples of plant parts are stem, callus, leaves, root, fruits, seeds, and tubers. Also specific plant tissues, such as chloroplast, apoplast, mitochondria, vacuole, peroxisomes, and cytoplasm are considered to be a plant part. Furthermore, any plant cell, whatever the tissue origin, is considered to be a plant part.

Also included within the scope of the present invention are the progeny of such plants, plant parts and plant cells.

The transgenic plant or plant cell expressing a polypeptide of the present invention may be constructed in accordance with methods known in the art. Briefly, the plant or plant cell is constructed by incorporating one or more expression constructs encoding a polypeptide of the present invention into the plant host genome and propagating the resulting modified plant or plant cell into a transgenic plant or plant cell.

Conveniently, the expression construct is a nucleic acid construct which comprises a nucleic acid sequence encoding a polypeptide of the present invention operably linked with appropriate regulatory sequences required for expression of the nucleic acid sequence in the plant or plant part of choice. Furthermore, the expression construct may comprise a selectable marker useful for identifying host cells into which the expression construct has been integrated and DNA sequences necessary for introduction of the construct into the plant in question (the latter depends on the DNA introduction method to be used).

The choice of regulatory sequences, such as promoter and terminator sequences and optionally signal or transit sequences is determined, for example, on the basis of when, where, and how the polypeptide is desired to be expressed. For instance, the expression of the gene encoding a polypeptide of the present invention may be constitutive or inducible, or may be developmental, stage or tissue specific, and the gene product may be targeted to a specific tissue or plant part such as seeds or leaves. Regulatory sequences are, for example, described by Tague *et al.,* 1988, *Plant Physiology* 86: 506.

For constitutive expression, the 35S-CaMV promoter may be used (Franck *et al.,* 1980, *Cell* 21: 285-294). Organ-specific promoters may be, for example, a promoter from storage sink tissues such as seeds, potato tubers, and fruits (Edwards & Coruzzi, 1990, *Ann. Rev. Genet.* 24: 275-303), or from metabolic sink tissues such as meristems (Ito et *al.,* 1994, *Plant Mol. Biol.* 24: 863-878), a seed specific promoter such as the glutelin, prolamin, globulin, or albumin promoter from rice (Wu et *al.,* 1998, *Plant and Cell Physiology* 39: 885-889), a *Vicia faba* promoter from the legumin B4 and the unknown seed protein gene from *Vicia faba* (Conrad *et al.,* 1998, *Journal of Plant Physiology* 152: 708-711), a promoter from a seed oil body protein (Chen *et al.,* 1998, Plant and Cell Physiology 39: 935-941), the storage protein napA promoter from *Brassica napus,* or any other seed specific promoter known in the art, *e.g.*, as described in WO 91/14772. Furthermore, the promoter may be a leaf specific promoter such as the *rbcs* promoter from rice or tomato (Kyozuka *et al.*, 1993, *Plant Physiology* 102: 991-1000, the chlorella virus adenine methyltransferase gene promoter (Mitra and Higgins, 1994, *Plant Molecular Biology* 26: 85-93), or the *aldP* gene promoter from rice (Kagaya *et al.*, 1995, *Molecular and General Genetics* 248: 668-674), or a wound inducible promoter such as the potato pin2 promoter (Xu *et al.*, 1993, *Plant Molecular Biology* 22: 573-588).

A promoter enhancer element may also be used to achieve higher expression of the enzyme in the plant. For instance, the promoter enhancer element may be an intron which is placed between the promoter and the nucleotide sequence encoding a polypeptide of the present invention. For instance, Xu *et al.,* 1993, *supra* disclose the use of the first intron of the rice actin 1 gene to enhance expression.

The selectable marker gene and any other parts of the expression construct may be chosen from those available in the art.

The nucleic acid construct is incorporated into the plant genome according to conventional techniques known in the art, including Agrobacterium-mediated transformation, virus-mediated transformation, microinjection, particle bombardment, biolistic transformation, and electroporation (Gasser *et al.*, 1990, *Science* 244: 1293; Potrykus, 1990, *Bio*/*Technology* 8: 535; Shimamoto *et al.*, 1989, Nature 338: 274).

Presently, *Agrobacterium* tumefaciens-mediated gene transfer is the method of choice for generating transgenic dicots (for a review, see Hooykas and Schilperoort, 1992, *Plant Molecular Biology* 19: 15-38). However it can also be used for transforming monocots, although other transformation methods are generally preferred for these plants. Presently, the method of choice for generating transgenic monocots is particle bombardment (microscopic gold or tungsten particles coated with the transforming DNA) of embryonic calli or developing embryos (Christou, 1992, *Plant Journal* 2: 275-281; Shimamoto, 1994, *Current Opinion Biotechnology* 5: 158-162; Vasil *et al.,* 1992, *Bio*/*Technology* 10: 667-674). An alternative method for transformation of monocots is based on protoplast transformation as described by Omirulleh *et al.*, 1993, *Plant Molecular Biology* 21: 415-428.

Following transformation, the transformants having incorporated therein the expression construct are selected and regenerated into whole plants according to methods well-known in the art.

The present invention also relates to methods for producing a polypeptide of the present invention comprising (a) cultivating a transgenic plant or a plant cell comprising a nucleic acid sequence encoding a polypeptide having 2,6-β-D-fructan hydrolase activity of the present invention under conditions conducive for production of the polypeptide; and (b) recovering the polypeptide.

### Compositions

In a still further aspect, the present invention relates to compositions comprising a polypeptide of the present invention. Preferably, the compositions are enriched in a polypeptide of the present invention. In the present context, the term "enriched" indicates that the 2,6-β-D-fructan hydrolase activity of the composition has been increased, e.g., with an enrichment factor of 1.1.

The composition may comprise a polypeptide of the invention as the major enzymatic component, *e.g.,* a mono-component composition. Alternatively, the composition may comprise multiple enzymatic activities, such as an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, invertase, laccase, lipase, mannosidase, oxidase, pectinolytic enzyme, peptidoglutaminase, peroxidase, phytase, polyphenoloxidase, proteolytic enzyme, ribonuclease, transglutaminase, or xylanase. The additional enzyme(s) may be producible by means of a microorganism belonging to the genus *Aspergillus,* preferably *Aspergillus aculeatus, Aspergillus awamori, Aspergillus niger,* or *Aspergillus oryzae,* or *Trichoderma,* Humicola, preferably *Humicola insolens,* or *Fusarium,* preferably *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sulphureum, Fusarium toruloseum, Fusarium trichothecioides,* or *Fusarium venenatum.*

The polypeptide compositions may be prepared in accordance with methods known in the art and may be in the form of a liquid or a dry composition. For instance, the polypeptide composition may be in the form of a granulate or a microgranulate. The polypeptide to be included in the composition may be stabilized in accordance with methods known in the art.

Examples are given below of preferred uses of the polypeptide compositions of the invention. The dosage of the polypeptide composition of the invention and other conditions under which the composition is used may be determined on the basis of methods known in the art.

### Applications

The 2,6-β-D-fructan hydrolases of the invention may be applied in various processes. Said 2,6-β-D-fructan hydrolases may be as described *vide supra* be comprised in a liquid or solid composition, which ever is suitable for the intended application.

### Disintegration/removal of biofilm

One preferred process is disintegration and/or removal of biofilm. The term "disintegration" as used herein is to be understood as hydrolysis of polysaccharides in a biofilm matrix connecting and binding together individual microbial cells in the biofilm whereby the microbial cells can be released and removed from the biofilm. The biofilm is preferably present at a surface and the disintegration of the biofilm is preferably achieved by bringing the surface in contact, e.g. by immersing, covering or splashing the surface with an aqueous medium comprising a 2,6-β-D-fructan hydrolase, thus disintegrating and removing the biofilm from the surface whereon to it is attached. It is presently contemplated that the 2,6-β-D-fructan hydrolase of the invention is an endo-2,6-β-D-fructan hydrolase, which surprisingly may be produced in quantities sufficient to test on biofilm matrices. Accordingly the invention relates to a process for disintegrating a microbial biofilm on a surface comprising contacting said biofilm with an endo-2,6-β-D-fructan hydrolase in an aqueous medium as well as a process for disintegrating a microbial biofilm comprising contacting said biofilm with a 2,6-β-D-fructan hydrolase of the invention. A 2,6-β-D-fructan hydrolase of the invention may also be used to hydrolyse slime e.g. in white waters in the pulping and paper industry. As opposed to a biofilm as defined, supra, slime is solubilized polysaccharides, which increases the viscosity of the aqueous medium in which it is dissolved.

The 2,6-β-D-fructan hydrolase may be present in the aqueous medium in amounts of 0.0001 to 10000 mg/l, preferably 0.001-1000 mg/l more preferably 0.01-100 mg/l, most preferably 0.1-10 mg/l.

The process may suitably be performed at temperatures from about ambient temperature to about 70°C. A more preferred temperature range is from about 30°C to about 60°C, e.g. about 40°C to about 50°C.

A suitable pH for the process lies within from about 3.5 to about 8.5 A more preferred pH range is from about 5.5 to about 8, e.g. from about 6.5 to about 7.5. The contact time or reaction time for the 2,6-ß-D-fructan hydrolase to effectively removing a biofilm may vary considerably, depending on the biofilm properties and the frequency of which a surface is treated with the 2,6-ß-D-fructan hydrolase, but preferably a suitable reaction time lies within 0.25-25 hours, more preferred 1-10 hours, e.g. 2 hours.

A biofilm may also suitably be removed by contacting the biofilm with the 2,6-β-D-fructan hydrolases of the invention in combination with one or more other enzymes and/or active compounds. Thus the 2,6-β-D-fructan hydrolases may be combined with on or more suitable hydrolases such as cellulases, hemicellulases, xylanases, amylases, lipases, proteases and/or pectinases. The 2,6-β-D-fructan hydrolases of the invention may further be combined with antimicrobial agents such as enzymatic or non-enzymatic biocides. An enzymatic biocide may e.g. be a composition comprising an oxidoreductase, e.g. a laccase or a peroxidase, especially haloperoxidase, and optionally an enhancing agent such as an alkyl syringate as described in patent applications WO97/42825 and DK97/1273 (not published at the filing date).

The surface from which a biofilm is to be removed and/or cleaned off is in one preferred embodiment a hard surface, which by definition relates to any surface which is essentially non-permeable to microorganisms. Examples of surfaces are surfaces made from metal, e.g. stainless steel alloys, plastics/synthetic polymers, rubber, board, glass, wood, paper, textile, concrete, rock, marble, gypsum and ceramic materials which optionally may be coated, e.g. with paint, enamel, polymers and the like. Accordingly the surface may be a member of a system holding, transporting, processing or in contact with aqueous solutions such as water supply systems, food processing systems, cooling systems, chemical processing systems or pharmaceutical processing systems. The invention is particularly useful for removing biofilm in the wood processing industry, such as the pulp and/or paper industry. Accordingly, the invention is useful in a conventional cleaning-in-place (C-I-P) system. The surface may a member of a system unit such as pipes, tanks, pumps, membranes, filters, heat exchangers, centrifuges, evaporators, mixers, spray towers, valves and reactors. The surface may also be or be a part of utensils used in the medical science and industry such as contaminated endoscopes, prosthetic devices or medical implants.

An important embodiment of the invention is, that the 2,6-β-D-fructan hydrolase of the invention is useful for prevention of so-called bio-corrosion occurring when a metal surface, e.g. a pipeline, is attacked by a microbial biofilm, that is by disintegrating the biofilm the 2,6-β-D-fructan hydrolase prevents the microbial cells of the biofilm from creating a biofilm environment which corrode the metal surface to which it is attached.

### Oral care applications

Also contaminated contact lenses are encompasses as surfaces suitable for applying treatment with the 2,6-β-D-fructan hydrolase of the invention.

The surface may however also be of biological origin such as mucous membranes, skin, teeth, hair, nails etc., and hence the invention also includes 2,6-β-D-fructan hydrolase of the invention for the treatment of a surface of biological origin, preferably mucous membranes, skin, teeth, hair or nails. In particular the invention includes 2,6-β-D-fructan hydrolase of the invention, e.g. by incorporating the enzyme of the invention into toothpaste, for the disintegration of plaque present on a human or animal tooth or for disintegration biofilm in lungs in patients suffering from cystic fibrosis.

Moreover the invention includes a process for preparation of a medicament comprising a 2,6-β-D-fructan hydrolase of the invention for disintegration of plaque present on a human or animal tooth. Also the invention includes a process for preparation of a medicament comprising a 2,6-β-D-fructan hydrolase of the invention for disintegration of a biofilm present on a human or animal mucous membrane.

Accordingly in a still further aspect the present invention relates to an oral care composition comprising a recombinant 2,6-β-D-fructan hydrolase of the invention or a wild-type 2,6-β-D-fructan hydrolases of the invention, preferably purified and essentially free of any active contaminants. An oral care composition of the invention may suitably comprise an amount of a recombinant 2,6-β-D-fructan hydrolase equivalent to an enzyme activity, calculated as enzyme activity units in the final oral care product, in the range from 0.001 LRU to 1000 LRU/ml, preferably from 0.01 LRU/ml to 500 LRU/ml, especially from 0.1 LRU/ml to 100 LRU/ml or 0.5 LRU/ml to 100 LRU LRU/ml.

It is also contemplated according to the invention to include other enzyme activities than 2,6-β-D-fructan hydrolase activity in the oral care composition. Contemplated enzyme activities include activities from the group of enzymes comprising dextranase, mutanases, oxidases, such as glucose oxidase, L-amino acid oxidase, peroxidases, such as *e.g.* the *Coprinus sp.* peroxidases described in WO 95/10602 (from Novo Nordisk A/S) or lactoperoxidase, haloperoxidases, especially haloperoxidase derivable from *Curvularia* sp., in particular *C. verruculosa* and *C. inaequalis.,* laccases, proteases, such as papain, acidic protease (*e.g.* the acidic proteases described in WO 95/02044 (Novo Nordisk A/S)), endoglucosidases, lipases, amylases, including amyloglucosidases, such as AMG (from Novo Nordisk A/S), anti-microbial enzymes, and mixtures thereof.

The oral care composition may have any suitable physical form (*i.e.* powder, paste, gel, liquid, ointment, tablet etc.). An "oral care composition" can be defined as a composition which can be used for maintaining or improving the oral hygiene in the mouth of humans and animals, by preventing dental caries, preventing the formation of dental plaque and tartar, removing dental plaque and tartar, preventing and/or treating dental diseases etc. At least in the context of the present invention oral care compositions do also encompass products for cleaning dentures, artificial teeth and the like. Examples of such oral care compositions includes toothpaste, dental cream, gel or tooth powder, odontic mouth washes, pre- or post brushing rinse formulations, chewing gum, lozenges, and candy. Tooth pastes and tooth gels typically include abrasive polishing materials, foaming agents, flavoring agents, humectants, binders, thickeners, sweetening agents, whitening/bleaching/stain removing agents, water, and optionally enzymes.

Mouth washes, including plaque removing liquids, typically comprise a water/alcohol solution, flavor, humectant, sweetener, foaming agent, colorant, and optionally enzymes.

Abrasive polishing material might also be incorporated into the oral care composition of the invention such as a dentifrice. According to the invention said abrasive polishing material includes alumina and hydrates thereof, such as alpha alumina trihydrate, magnesium trisilicate, magnesium carbonate, kaolin, aluminosilicates, such as calcined aluminum silicate and aluminum silicate, calcium carbonate, zirconium silicate, and also powdered plastics, such as polyvinyl chloride, polyamides, polymethyl methacrylate, polystyrene, phenol-formaldehyde resins, melamine-formaldehyde resins, urea-formaldehyde resins, epoxy resins, powdered polyethylene, silica xerogels, hydrogels and aerogels and the like. Also suitable as abrasive agents are calcium pyrophosphate, water-insoluble alkali metaphosphates, dicalcium phosphate and/or its dihydrate, dicalcium orthophosphate, tricalcium phosphate, particulate hydroxyapatite and the like. It is also possible to employ mixtures of these substances.

Dependent on the oral care composition the abrasive product may be present in from 0 to 70% by weight, preferably from 1% to 70%. For tooth pastes the abrasive material content typically lies in the range of from 10% to 70% by weight of the final toothpaste.

Humectants are employed to prevent loss of water from e.g. tooth pastes. Suitable humectants for use in oral care compositions according to the invention include the following compounds and mixtures thereof: glycerol, polyol, sorbitol, polyethylene glycols (PEG), propylene glycol, 1,3-propanediol, 1,4-butanediol, hydrogenated partially hydrolysed polysaccharides and the like. Humectants are in general present in from 0% to 80%, preferably 5 to 70% by weight in toothpaste.

Silica, starch, tragacanth gum, xanthan gum, extracts of Irish moss, alginates, pectin, cellulose derivatives, such as hydroxyethyl cellulose, sodium carboxymethyl cellulose and hydroxypropyl cellulose, polyacrylic acid and its salts, polyvinylpyrrolidone, can be mentioned as examples of suitable thickeners and binders, which helps stabilising a dentifrice product. Thickeners may be present in toothpaste creams and gels in an amount of from 0.1 to 20% by weight, and binders to the extent of from 0.01 to 10% by weight of the final product.

As foaming agent soap, anionic, cationic, non-ionic, amphoteric and/or zwitterionic surfactants can be used. These may be present at levels of from 0% to 15%, preferably from 0.1 to 13%, more preferably from 0.25 to 10% by weight of the final product.

Surfactants are only suitable to the extent that they do not exert an inactivation effect on the present enzymes. Surfactants include fatty alcohol sulphates, salts of sulphonated mono-glycerides or fatty acids having 10 to 20 carbon atoms, fatty acid-albumen condensation products, salts of fatty acids amides and taurines and/or salts of fatty acid esters of isethionic acid.

Suitable sweeteners include saccharin.

Flavours, such as spearmint, are usually present in low amounts, such as from 0.01% to about 5% by weight, especially from 0.1% to 5%.

Whitening/bleaching agents include H₂O₂ and may be added in amounts less that 5%, preferably from 0.25 to 4%, calculated on the basis of the weight of the final product.

The whitening/bleaching agents may be an enzyme, such as an oxidoreductase. Examples of suitable teeth bleaching enzymes are described in WO 97/06775 (from Novo Nordisk A/S).

Water is usually added in an amount giving e.g. toothpaste a flowable form.

Further water-soluble anti-bacterial agents, such as chlorhexidine digluconate, hexetidine, alexidine, Triclosan®, quaternary ammonium anti-bacterial compounds and water-soluble sources of certain metal ions such as zinc, copper, silver and stannous (*e.g.* zinc, copper and stannous chloride, and silver nitrate) may also be included.

Also contemplated according to the invention is the addition of compounds which can be used as fluoride source, dyes/colorants, preservatives, vitamins, pH-adjusting agents, anti-caries agents, desensitizing agents etc.

Other components useful in oral care compositions of the invention are enzymes as described supra. Enzymes are biological catalysts of chemical reactions in living systems. Enzymes combine with the substrates on which they act forming an intermediate enzyme-substrate complex. This complex is then converted to a reaction product and a liberated enzyme which continue its specific enzymatic function.

Enzymes provide several benefits when used for cleansing of the oral cavity. Proteases break down salivary proteins, which are adsorbed onto the tooth surface and form the pellicle, the first layer of resulting plaque. Proteases along with lipases destroy bacteria by lysing proteins and lipids which form the structural components of bacterial cell walls and membranes.

Dextranase and other carbohydrases such as the 2,6-β-D-fructan hydrolase of the invention breaks down the organic skeletal structure produced by bacteria that forms a matrix for bacterial adhesion. Proteases and amylases, not only prevents plaque formation, but also prevents the development of calculus by breaking-up the carbohydrate-protein complex that binds calcium, preventing mineralization.

A toothpaste of the invention may typically comprise the following ingredients (in weight % of the final toothpaste composition):

| | |
|---|---|
| Abrasive material | 10 to 70% |
| Humectant | 0 to 80% |
| Thickener | 0.1 to 20% |
| Binder | 0.01 to 10% |
| Sweetener | 0.1% to 5% |
| Foaming agent | 0 to 15% |
| Whitener | 0 to 5% |
| Enzymes | 0.0001% to 20% |

In a specific embodiment a tooth paste of the invention has a pH in the range from 6.0 to about 8.0, and comprises:

| | | |
|---|---|---|
| a) | 10% to 70% | Abrasive material |
| b) | 0 to 80% | Humectant |
| c) | 0.1 to 20% | Thickener |
| d) | 0.01 to 10% | Binder |
| e) | 0.1% to 5% | Sweetener |
| f) | 0 to 15% | Foaming agent |
| g) | 0 to 5% | Whitener |
| i) | 0.0001% to 20% | Enzymes. |

Said enzymes referred to under i) include a recombinant 2,6-β-D-fructan hydrolase of the invention, and optionally other types of enzymes mentioned above known to be used in toothpastes and the like.

A mouth wash of the invention may typically comprise the following ingredients (in weight % of the final mouth wash composition):

| | |
|---|---|
| 0-20% | Humectant |
| 0-2% | Surfactant |
| 0-5% | Enzymes |
| 0-20% | Ethanol |
| 0-2% | Other ingredients (e.g. flavour, sweetener active ingredients such as florides). |
| 0-70% | Water |

The mouth wash composition may be buffered with an appropriate buffer e.g. sodium citrate or phosphate in the pH-range 6-7.5.

The mouth wash may be in none-diluted form (i.e. must be diluted before use).

The oral care compositions of the invention may be produced using any conventional method known to the art of oral care.

### Detergent applications

In another preferred embodiment the 2,6-β-D-fructan hydrolase of the invention may also be incorporated in detergent compositions and used for removal/cleaning of biofilm present on household and/or industrial textile/laundry. Accordingly the 2,6-β-D-fructan hydrolase of the invention may be added to and thus become a component of a detergent composition.

The detergent composition of the invention may for example be formulated as a hand or machine laundry detergent composition including a laundry additive composition suitable for pretreatment of stained fabrics and a rinse added fabric softener composition, or be formulated as a detergent composition for use in general household hard surface cleaning operations, or be formulated for hand or machine dishwashing operations.

In a specific aspect, the invention provides a detergent additive comprising the 2,6-β-D-fructan hydrolase of the invention. The detergent additive as well as the detergent composition may comprise one or more other enzymes such as a protease, a lipase, a cutinase, an amylase, a carbohydrase, a cellulase, a pectinase, a mannanase, an arabinase, a galactanase, a xylanase, an oxidase, e.g., a laccase, and/or a peroxidase.

In general the properties of the chosen enzyme(s) should be compatible with the selected detergent, (i.e. pH-optimum, compatibility with other enzymatic and non-enzymatic ingredients, etc.), and the enzyme(s) should be present in effective amounts. Proteases: Suitable proteases include those of animal, vegetable or microbial origin. Microbial origin is preferred. Chemically modified or protein engineered mutants are included. The protease may be a serine protease or a metallo protease, preferably an alkaline microbial protease or a trypsin-like protease. Examples of alkaline proteases are subtilisins, especially those derived from *Bacillus,* e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the *Fusarium* protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235 and 274.

Preferred commercially available protease enzymes include Alcalase^{™}, Savinase^{™}, Primase^{™}, Duralase^{™}, Esperase^{™}, and Kannase^{™} (Novo Nordisk A/S), Maxatase^{™}, Maxacal^{™}, Maxapem^{™}, Properase^{™}, Purafect^{™}, Purafect OxP^{™}, FN2^{™}, and FN3^{™} (Genencor International Inc.).

Lipases: Suitable lipases include those of bacterial or fungal origin. Chemically modified or protein engineered mutants are included. Examples of useful lipases include lipases from *Humicola* (synonym *Thermomyces*)*,* e.g. from H. *lanuginosa* (*T. lanuginosus*) as described in EP 258 068 and EP 305 216 or from *H. insolens* as described in WO 96/13580, a *Pseudomonas* lipase, e.g. from *P. alcaligenes* or *P. pseudoalcaligenes* (EP 218 272), *P. cepacia* (EP 331 376), *P. stutzeri* (GB 1,372,034), *P. fluorescens, Pseudomonas sp.* strain SD 705 (WO 95/06720 and WO 96/27002), *P. wisconsinensis* (WO 96/12012), a *Bacillus* lipase, e.g. from *B. subtilis* (Dartois et al. (1993), Biochemica et Biophysica Acta, 1131, 253-360), B. *stearothermophilus* (JP 64/744992) or *B. pumilus* (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202.

Preferred commercially available lipase enzymes include Lipolase^{™} and Lipolase Ultra^{™} (Novo Nordisk A/S). Amylases: Suitable amylases (α and/or β) include those of bacterial or fungal origin. Chemically modified or protein engineered mutants are included. Amylases include, for example, α-amylases obtained from *Bacillus,* e.g. a special strain of *B*. *licheniformis,* described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are Duramyl^{™} , Termamyl^{™}, Fungamyl^{™} and BAN^{™} (Novo Nordisk A/S), Rapidase^{™} and Purastar^{™} (from Genencor International Inc.). Cellulases: Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g. the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme^{™}, and Carezyme^{™} (Novo Nordisk A/S), Clazinase^{™}, and Puradax HA^{™} (Genencor International Inc.), and KAC-500(B)^{™} (Kao Corporation).

Peroxidases/Oxidases: Suitable peroxidases/oxidases include those of plant, bacterial or fungal origin. Chemically modified or protein engineered mutants are included. Examples of useful peroxidases include peroxidases from *Coprinus,* e.g. from *C. cinereus,* and variants thereof as those described in WO 93/24618, WO 95/10602, and WO 98/15257.

Commercially available peroxidases include Guardzyme^{™} (Novo Nordisk A/S).

The detergent enzyme(s) may be included in a detergent composition by adding separate additives containing one or more enzymes, or by adding a combined additive comprising all of these enzymes. A detergent additive of the invention, i.e. a separate additive or a combined additive, can be formulated e.g. as a granulate, a liquid, a slurry, etc. Preferred detergent additive formulations are granulates, in particular non-dusting granulates, liquids, in particular stabilized liquids, or slurries.

Non-dusting granulates may be produced, e.g., as disclosed in US 4,106,991 and 4,661,452 and may optionally be coated by methods known in the art. Examples of waxy coating materials are poly(ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

The detergent composition of the invention may be in any convenient form, e.g., a bar, a tablet, a powder, a granule, a paste or a liquid. A liquid detergent may be aqueous, typically containing up to 70 % water and 0-30 % organic solvent, or non-aqueous.

The detergent composition comprises one or more surfactants, which may be non-ionic including semi-polar and/or anionic and/or cationic and/or zwitterionic. The surfactants are typically present at a level of from 0.1% to 60% by weight.

When included therein the detergent will usually contain from about 1% to about 40% of an anionic surfactant such as linear alkylbenzenesulfonate, alpha-olefinsulfonate, alkyl sulfate (fatty alcohol sulfate), alcohol ethoxysulfate, secondary alkanesulfonate, alpha-sulfo fatty acid methyl ester, alkyl- or alkenylsuccinic acid or soap.

When included therein the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl N-alkyl derivatives of glucosamine ("glucamides").

The detergent may contain 0-65 % of a detergent builder or complexing agent such as zeolite, diphosphate, triphosphate, phosphonate, carbonate, citrate, nitrilotriacetic acid, ethylenediaminetetraacetic acid, diethylenetriaminepentaacetic acid, alkyl- or alkenylsuccinic acid, soluble silicates or layered silicates (e.g. SKS-6 from Hoechst).

The detergent may comprise one or more polymers. Examples are carboxymethylcellulose, poly(vinylpyrrolidone), poly (ethylene glycol), poly(vinyl alcohol), poly(vinylpyridine-N-oxide), poly(vinylimidazole), polycarboxylates such as polyacrylates, maleic/acrylic acid copolymers and lauryl methacrylate/acrylic acid copolymers.

The detergent may contain a bleaching system which may comprise a H₂O₂ source such as perborate or percarbonate which may be combined with a peracid-forming bleach activator such as tetraacetylethylenediamine or nonanoyloxybenzenesulfonate. Alternatively, the bleaching system may comprise peroxyacids of e.g. the amide, imide, or sulfone type.

The enzyme(s) of the detergent composition of the invention may be stabilized using conventional stabilizing agents, e.g., a polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in e.g. WO 92/19709 and WO 92/19708.

The detergent may also contain other conventional detergent ingredients such as e.g. fabric conditioners including clays, foam boosters, suds suppressors, anti-corrosion agents, soil-suspending agents, anti-soil redeposition agents, dyes, bactericides, optical brighteners, hydrotropes, tarnish inhibitors, or perfumes.

It is at present contemplated that in the detergent compositions any enzyme, in particular the enzyme of the invention, may be added in an amount corresponding to 0.01-100 mg of enzyme protein per liter of wash liqour, preferably 0.05-5 mg of enzyme protein per liter of wash liqour, in particular 0.1-1 mg of enzyme protein per liter of wash liqour.

The enzyme of the invention may additionally be incorporated in the detergent formulations disclosed in WO 97/07202 which is hereby incorporated as reference.

### Production of 2,6-β-D-fructose oligosaccharide sweeteners

The 2,6-β-D-fructan hydrolase of the invention is also useful in a hydrolysis process of levan and phlein, with the purpose of generating β-2,6-fructose oligosaccharides. β-2,6-fructose oligosaccharides are useful as sweeteners in foods and exhibit or have additionally positive health effects. The benefits of oligosaccharide ingestion such as a) proliferation of Bifidobacteria and reduction of detrimental bacteria, b) reduction of toxic metabolites and detrimental enzymes, c) prevention of pathogenic and autogenous diarrhea, d) prevention of constipation, e) protecktion of liver function, f) reduction of blood pressure, g) anti cancer effect, h) production of nutrients and i) replacement of antibiotics in feed are described in Tomomatsu H; Food technology; 1994; vol. 48, pp. 61-65.

Accordingly an embodiment of the invention is a process for production of β-2,6-fructose oligosaccharides comprising contacting a material comprising a substrate selected from the group consisting of levan and phlein with the 2,6-β-D-fructan hydrolase of the invention in an aqueous medium, the 2,6-β-D-fructan hydrolase being present in an amount sufficient to hydrolyze the substrate.

A suitable phlein comprising material may be a plant material suitable for the process or purified phlein recovered from processing such a plant material. A preferred plant material is a grass, preferably of the order of graminales (Poales), e.g. *Dactylis glomerata* (orchard grass), *Festuca arundinacea, Lolium temulentum, Lolium multiflorum, Lolium perenne* (Perennial ryegrass), *Phleum pratense* as well as Rye (Secale), Oat (Aveneae), Wheat (Triticeae), Barley (Hordeum) and rice (Oryzeae) and bamboo (Bambuseaea). Both stems and/or leafs of grasses contains phlein. Grasses has so far found little applicability in producing food additives.

The plant material comprising the phlein may be preprocessed prior to the enzyme reaction, i.e. after harvesting it may be cut into small pieces (shredded), followed by maceration to open the cells. Maceration may be accomplished by using conventional physical methods and/or cell wall degrading enzymes known to the art. The material may be further processed to recover a purified phlein, e.g. separating the soluble phlein from insoluble fractions of the material by extraction. The phlein may be further purified by separating phlein from proteins by precipitating the proteins e.g. by acid or salt precipitation followed by centrifugation.

The phlein may be even further purified by precipitating the phlein, e.g. by adding an alcohol, e.g. ethanol, to the phlein solution.

The phlein comprised in the plant material or the purified phlein is then subjected to hydrolysis with a 2,6-β-D-fructan hydrolase of the invention optionally in combination one or more other hydolases such as inulinases (EC 3.2.1.7), fructan β-fructosidases (EC 3.2.1.80), and levanbiohydrolases (EC 3.2.1.64.)

The hydrolysis reaction producing β-2,6-fructose oligosaccharides should be carried out at conditions allowing a suitable activity level of the 2,6-β-D-fructan hydrolase:

Accordingly the hydrolysis reaction should preferably be carried out at a pH in the range of from about pH 4 to about pH 8, in particular of from about pH 5 to about pH 7.

Further the hydrolysis reaction should preferably be carried out at a temperature of from about 10°C to about 70 °C, more preferred of from about 20°C to about 50°C, most preferred of from about 30°C to about 40°C, e.g. 37°C.

The reaction time depends on the material comprising the levan or phlein and may be any time suitable for obtaining the desired degree of hydrolysis. Usually a reaction time within about 1 to 72 hours is suitable. A preferred reaction time is about 10 to 48 hours, more preferred about 15-35 hours most preferred 20-30 hour, e.g. 24 hours overnight reaction.

The 2,6-β-D-fructan hydrolase of the invention should be dosed in amounts sufficient for achieving the desired degree of hydrolysis within the desired reaction time. It is at present contemplated that a suitable enzyme dosage is in the range of from 0,00001-100 is suitable depending on the amount, origin and purity of the substrate. A more preferred dosage is 0,0001-10 mg/l, while 0,001-1 mg/l, e.g. 0,1 mg/l is most preferred.

### Production of fructose

Fructose may be produced by as a first step degrading a substrate selected from phlein or levan with the enzyme of the invention, and as a second step either subsequently or simultaneously contacting the generated 2,6-β-D-fructose oligosaccharides with an 2,6-β-D-fructan exo-hydrolase, such as sacC exo-levanase from *Bacillus subtillis* e.g. as described in Wanker E., Huber A., Schwab H.; ***Purification and characterization of Bacillus subtillis levanase produced in Escherichia coli,*** Applied and environmental microbiology, 1995, vol. 61, no. 5, pp. 1953-1958.

### Materials and methods

### General molecular biology methods:

Unless otherwise mentioned the DNA manipulations and transformations were performed using standard methods of molecular biology (Sambrook et al. (1989) *supra;* Ausubel, F. M. et al. (eds.); ***Current protocols in Molecular Biology;*** 1995; John Wiley and Sons.; Harwood C. R., and Cutting S. M. (eds.); **Molecular Biological Methods for Bacillus**; 1990; John Wiley and Sons.

### Media

TY medium was prepared as described in Ausubel et al. (1995), supra.

Diluted TY-medium was prepared by diluting TY medium 1:10 with demineralized water.

Diluted TY medium supplemented with stained levan. This medium was prepared by adding a 10% w/w stained levan solution to the diluted TY medium at a ratio of 20 ml stained levan solution to 500 ml diluted TY medium. The levan was stained according to the description in Rinderknecht H., Wilding P., Haverback B.J. Experientia, 1967, vol. 23, p 805.

B-medium was prepared as described in Bertani G.; J. Bacteriol.; 1951; vol. 62; pp. 293-300.
BPX medium was prepared in accordance with EP 0 506 780 (WO 91/09129).

BPG medium was prepared by mixing 500 ml of LB medium with 5 ml phosphate buffer, 1 M, adjusted to pH 7 and 10 ml 20% w/w aqueous glucose solution.

### Deposited organisms:

The following microorganisms was deposited by the inventors according to the Budapest Treaty on the International Recognition of the Deposit of Microorganisms for the Purposes of Patent Procedure at the Deutsche Sammlung von Mikroorganismen und Zellkulturen GmbH, Mascheroder Weg 1b, D-38124 Braunschweig, Federal Republic of Germany:
Deposition number DSM 12458; *Paenibacillus amylolyticus;* October 23, 1998.

Deposition number DSM 12406; *E. coli* harboring strain transformed with plasmid pSJ1678 containing a nucleic acid sequence encoding a 2,6-β-D-fructan hydrolase polypeptide; October 23, 1998.

### Other organisms

Host cells were cells of *E. coli* SJ2 (Diderichsen B., Wedsted U., Hedegaard L., Jensen B. R., Sjøholm C.; ***Cloning of aldB, which encodes alpha-acetolactate decarboxylase, an exoenzyme from Bacillus brevis*;** J. Bacteriol., 1990, vol. 172, pp. 4315-4321).

Bacillus subtilis strain SHa273 disclosed in WO 95/10603 was used. Competent cells were prepared and transformed as described by Yasbin, R.E., Wilson, G.A. and Young, F.E. (1975) Transformation and transfection in lysogenic strains of Bacillus subtilis: evidence for selective induction of prophage in competent cells. J. Bacteriol, 121:296-304.

### Plasmids:

SJ1678 as described in the International Patent Application published as WO 94/19454.

pLi937 is pSJ1678 with an insert containing SEQ ID No:2.

pMOL944. This plasmid is a pUB110 derivative essentially containing elements making the plasmid propagatable in Bacillus subtilis, kanamycin resistance gene and having a strong promoter and signal peptide cloned from the amyL gene of *B. licheniformis* ATCC14580. The signal peptide contains a SacII site making it convenient to clone the DNA encoding the mature part of a protein in-fusion with the signal peptide. This results in the expression of a Pre-protein which is directed towards the exterior of the cell. The plasmid was constructed by means of ordinary genetic engineering and is briefly described in the following. The pUB110 plasmid (McKenzie, T. et al., 1986, Plasmid 15:93-103) was digested with the unique restriction enzyme NciI. A PCR fragment amplified from the amyL promoter encoded on the plasmid pDN1981 (P.L. Jørgensen et al., 1990, Gene, 96, p37-41.) was digested with NciI and inserted in the NciI digested pUB110 to give the plasmid pSJ2624.

The two PCR primers used have the following sequences:
# LWN54945'-GTCGCCGGGGCGGCCGCTATCAATTGGTAACTGTATCTCAGC -3'
# LWN54955'-GTCGCCCGGGAGCTCTGATCAGGTACCAAGCTTGTCGACCTGCAGAA TGAGGCAGCAAGAAGAT -3'
The primer #LWN5494 inserts a NotI site in the plasmid. The plasmid pSJ2624 was then digested with SacI and NotI and a new PCR fragment amplified on amyL promoter encoded on the pDN1981 was digested with SacI and NotI and this DNA fragment was inserted in the SacI-NotI digested pSJ2624 to give the plasmid pSJ2670. This cloning replaces the first amyL promoter cloning with the same promoter but in the opposite direction. The two primers used for PCR amplification have the following sequences:
#LWN59385'-GTCGGCGGCCGCTGATCACGTACCAAGCTTGTCGACCTGCAGAATG AGGCAGCAAGAAGAT -3'
#LWN59395'-GTCGGAGCTCTATCAATTGGTAACTGTATCTCAGC -3'

The plasmid pSJ2670 was digested with the restriction enzymes PstI and BclI and a PCR fragment amplified from a cloned DNA sequence encoding the alkaline amylase SP722 (Patent # WO9526397-A1) was digested with PstI and BclI and inserted to give the plasmid pMOL944. The two primers used for PCR amplification have the following sequence:
#LWN78645' -AACAGCTGATCACGACTGATCTTTTAGCTTGGCAC-3'
#LWN7901 5' -AACTGCAGCCGCGGCACATCATAATGGGACAAATGGG -3'
The primer #LWN7901 inserts a SacII site in the plasmid.

### Isolation of genomic DNA

The strain of *Paenibacillus* DSM 12458 was propagated in liquid TY medium. After 16 hours incubation at 37°C and 300 rpm, the cells were harvested and genomic DNA isolated by the method described by Pitcher D. G., Saunders N. A., Owen R. J.; ***Rapid extraction of bacterial genomic DNA with guanidium thiocyanate;*** Lett. Appl. Microbiol.; 1989; vol. 8; pp. 151-156.

### Genomic library construction

Genomic DNA was partially digested with restriction enzyme Sau3A, and size-fractionated by electrophoresis on a 0.7 % agarose gel. Fragments between 2 and 10 kb in size was isolated by electrophoresis onto DEAE-cellulose paper (Dretzen G., Bellard M., Sassone-Corsi P., Chambon P.; ***A reliable method for the recovery of DNA fragments from agarose and acrylamide gels;*** Anal. Biochem.; 1981; vol. 112; pp. 295-298).

Isolated DNA fragments were ligated to BamHI digested pSJ1678 plasmid DNA, and the ligation mixture was used to transform E. *coli* SJ2.

### Transformation

*E. coli* SJ2 host cells were prepared for and transformed by electroporation using a gene PULSER^{™} electroporator from BIO-RAD as described by the supplier.

### Identification of positive transformant:

A DNA library in E. *coli* SJ2, constructed as described above, was screened on agar plates made of ten times diluted TY-medium or undiluted LB medium, supplemented with stained levan and incubated overnight at 37°C. For clones/colonies expressing the 2,6-β-D-fructan hydrolase clearing zones (decoloration of the stained levan) appeared.

### Isolation of the DNA sequence shown in SEQ ID No: 2:

Positive *E. coli* transformant found, DSM 12406, and the nucleic acid sequences shown in SEQ ID NO: 2 encoding the 2,6-β-D-fructan hydrolase of the invention were obtained by extraction of plasmid DNA by methods known in the art (Sambrook et al. (1989) supra.

### Sequencing the DNA encoding the 2,6-β-D-fructan hydrolase

The DNA encoding 2,6-β-D-fructan hydrolase of the invention was sequenced by conventional methods known to the art, i.e. by DNA sequencing of the cloned Sau3A DNA fragment. The DNA was characterised by DNA sequencing using the Taq deoxy-terminal cycle sequencing kit (Perkin-Elmer, USA), fluorescent labelled terminators and appropriate oligonucleotides as primers.

### SDS-PAGE gel electrophoresis

The SDS-PAGE characterization of the 2,6-β-D-fructan hydrolase was performed according to methods known to the art (Sambrook et al. (1989) supra). The electrophoresis was performed on a 4-20% gradient Tris-Glycine precast gel (catalog no. EC60255; NOVEX Electrophoresis GmbH, D-65929 Frankfurt/M.) according to the instructions enclosed with the product on a Laemmli-type, Tris-glycine, SDS-PAGE, denaturing and reducing gel. After electrophoresis, the gel is stained with GelCode Blue Stain Reagent (catalog no. 24590; Pierce Chemical Company, Rockford Il, USA) according to the instructions enclosed with the reagent. As molecular weight standards on the SDS-PAGE gel, an Electrophoresis Calibration Kit (catalog no. 17-0446-01; Amersham Pharmacia, Uppsala, Sweden) was used with molecular weights: 94,000; 67,000; 43,000; 30,000; 20,100 and 14,400.

### Determination of protein by BCA protein assay:

This assay is identical to PIERCE cat. No. 23225 Pierce Chemical Company, Rockford IL, USA: BCA protein assay kit, and the assay was performed according to the description provided with the kit. The BCA working solution was made by mixing 50 parts of reagent A with 1 part reagent B. 200 µl enzyme sample and standard solutions of BSA was each mixed with 2.0 ml BCA working solution. After 30 minutes at 37°C, the sample and standards was cooled to room temperature and OD₄₉₀ was read. OD₄₉₀ of the enzyme sample was compared with the OD₄₉₀ of the BSA standards by interpolation as a measure of the protein concentration (in mg enzyme per ml) in the sample.

### 2,6-β-D-fructan hydrolase activity assay.

The following assay was used for characterization of the 2,6-β-D-fructan hydrolase. The principle of the assay is that 2,6-β-D-fructan hydrolase hydrolyzes levan thereby generating reducing groups. The increase in reducing ends generated after an incubation, which is a measure of the 2,6-β-D-fructan hydrolase activity, is monitored by adding a reagent forming a color upon reaction with the reducing groups. To correlate the color formation to the amount of generated reducing groups the optical density or absorbance is compared to the color formation of standard samples of fructose with a predetermined number of reducing groups.

1 LRU (Levan Reducing sugar Unit) is defined as the amount of enzyme that generates an amount of reducing groups in a levan substrate equivalent to 1 µmol fructose per minute.

### Procedure

A buffer (**A**) is prepared consisting of 50 mM CH₃COOH, 50 mM KH₂PO₄, 50 mM H₃BO₃, 1 mM CaCl₂, 1 mM MgCl₂ and 0.01% Triton X-100. The solution adjusted to pH-value 6.5 with a solution of hydrochloric acid or sodium hydroxide.

A levan substrate solution (**B**) is prepared by dissolving 15 mg/ml levan (Sigma, L-8647) in the buffer (**A**).

A sample solution (**C**) is prepared by dissolving/diluting a sample of recovered 2,6-β-D-fructan hydrolase in the buffer (**B**).

A CNC-reagent (**D**) is prepared by mixing in the following order: 1.8 ml demineralized water, 200 µl 40mM CuSO₄, 6.0 ml 0.67 mg/ml Neocuproin-HCl (Merck 2964) and 2.67 ml 2.0 M Na₂CO₃. The CNC-reagent was made just before use.

A mixture of 50 µl levan substrate (**B**) and 25 µl enzyme sample (**C**), a mixture of 50 µl levan substrate (**B**) and 25 µl buffer (**A**) and fructose standard solutions are transferred to ice cooled Eppendorf tubes. Incubation is initiated by placing the tubes in an Eppendorf thermomixer at 37 °C. The tubes is incubated for 15 minutes on the Eppendorf thermomixer with shaking. The incubation is stopped by ice cooling the tubes, adding 500 µl ice-cold CNC-reagent (**D**), and mixing the solutions in the tubes by sucking back and forth the solutions with a pipette. To develop the color, the tubes is placed in an Eppendorf thermomixer and incubated for 30 minutes at 25°C without exposing the tubes to light. 200 µl of the colored solutions is transferred to a microtiter plate and OD₄₅₀ is read. OD₄₅₀ for the solution containing both enzyme and levan minus OD₄₅₀ for the solution containing only levan is compared to the OD₄₅₀ for the standards containing fructose only by interpolation.

### EXAMPLES

The invention is further illustrated with the following examples which are not, in any way, intended to be limiting to the scope of the invention as claimed.

### EXAMPLE 1

### Culturing of Paenibacillus amylolyticus DSM 12458

DSM 12458 was grown in a TY medium, pH 7.0, supplemented with 0.2% w/w levan at 30°C. Culture supernatant was harvested on day 1, 2, and 5, respectively and centrifuged 10 minutes in Eppendorf Cups. An agarose gel plates containing 20 ml 10% w/w stained levan solution per 500 ml agarose gel was casted. 20 µl cell free supernatant samples were applied to 4 mm holes which were punched out of the agarose gel plates. The agarose gel plates were incubated at 37°C for 2 hours. For all harvested samples clearing zones appeared around the holes showing presence of the 2,6-β-D-fructan hydrolase in the supernatant already from the first harvesting day.

### EXAMPLE 2

### Degradation of levan with the 2,6-ß-D-fructan hydrolase

Enzyme was recovered from the culture supernatant of example 1 by gel filtration of the supernatant on a Sephadex G25 PD-10 column kit at conditions according to instruction by supplier (Pharmacia). The enzyme was eluted using a 0.02 M MOPS buffer at pH 7.0. 0.4 ml of the recovered enzyme preparation was mixed with 0.4 ml of a 2% w/w levan solution and 0.2 ml buffer (0.1 M Na-acetate buffer pH 5.5). One sample of 0.5 ml was taken immediately after preparation and the enzyme reaction was stopped by boiling for 20 minutes and centrifugation. Another sample of 0.5 ml was incubated overnight at 30°C, where after the enzyme reaction was stopped. Both samples were analyzed by Dionex HPLC analysis.

The analysis showed that during the incubation overnight the levan was almost totally degraded into 2,6-β-fructose oligo saccharides and fructose indicating that the 2,6-ß-D-fructan hydrolase is an endo-hydrolase.

### EXAMPLE 3

### Culturing of E. coli DSM 12406 and recovery and purification of cloned 2,6-β-D-fructan hydrolase.

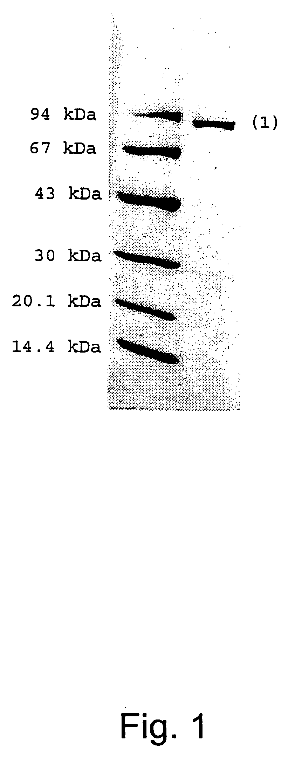

DSM 12406 grown in a TY medium, pH 7.0, supplemented with 0.2% w/w levan at 30°C for 24 hours. 5 litres of supernatant was filtered through a Seitz EKS depth filter plate to give a germ free filtrate. The pH in the germ free filtrate was adjusted to pH 7.0 with CH₃COOH and diluted to 50 L with demineralized water. The conductivity was 2.3 mS/cm. The enzyme solution was applied to a 1 litre Q-sepharose FF column equilibrated in 10 mM KH₂PO₄/NaOH, pH 7.0. After washing the column with the equilibration buffer, the 2,6-β-D-fructan hydrolase was eluted with a linear NaCl gradient (0 → 0.5M). 2,6-β-D-fructan hydrolase containing fractions were pooled and (NH₄)₂SO₄ was added to 2.0M final concentration. The enzyme was applied to a 100 ml Butyl Toyopearl 650S column equilibrated in 10 mM KH₂PO₄/NaOH, pH 7.0, 2M (NH₄)₂SO₄, pH 7.0. After washing the column with the equilibration buffer, the 2,6-β-D-fructan hydrolase was eluted with a linear (NH₄)₂SO₄ gradient (2 → 0M). 2,6-β-D-fructan hydrolase containing fractions were pooled and the buffer was exchanged with 10 mM KH₂PO₄/NaOH, pH 7.0 on a Sephadex G25 column. The G25 filtrate was applied to a 40 ml SOURCE 30Q column equilibrated in 10mM KH₂PO₄/NaOH, pH 7.0. The column was washed with the equilibration buffer and the 2,6-β-D-fructan hydrolase was eluted with a linear NaCl gradient (0 → 0.3M). 2,6-β-D-fructan hydrolase containing fractions were analysed by SDS-PAGE as shown in figure 1 and the molecular weight of the levanase (band (1)) was determined to be about Mᵣ = 88 kDa by interpolation. The N-terminus of the prepared 2,6-β-D-fructan hydrolase was found to be blocked. Less than 1 mg 2,6-β-D-fructan hydrolase was purified from the 5 litres of culture supernatant.

### EXAMPLE 4

### Determination of dose-response

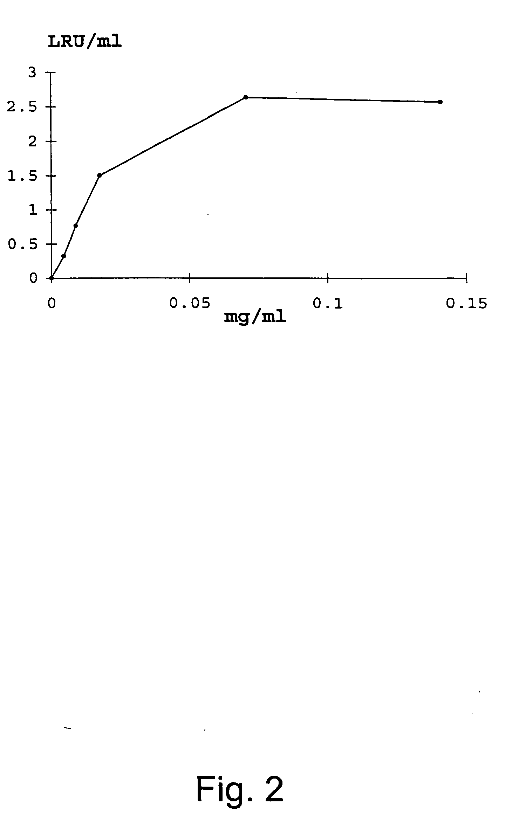

The relation between amount of 2,6-β-D-fructan hydrolase enzyme protein and the measured response in the 2,6-β-D-fructan hydrolase assay was determined. Determination of the dose-response relation was determined by subjecting samples of various enzyme protein concentration to the 2,6-β-D-fructan hydrolase assay, and the result is shown in figure 2. It is observed that the 2,6-β-D-fructan hydrolase assay is linear between 0 and approx. 1.5 LRU/ml, which range was used throughout further analyses.

### EXAMPLE 5

### Determination of pH-activity range

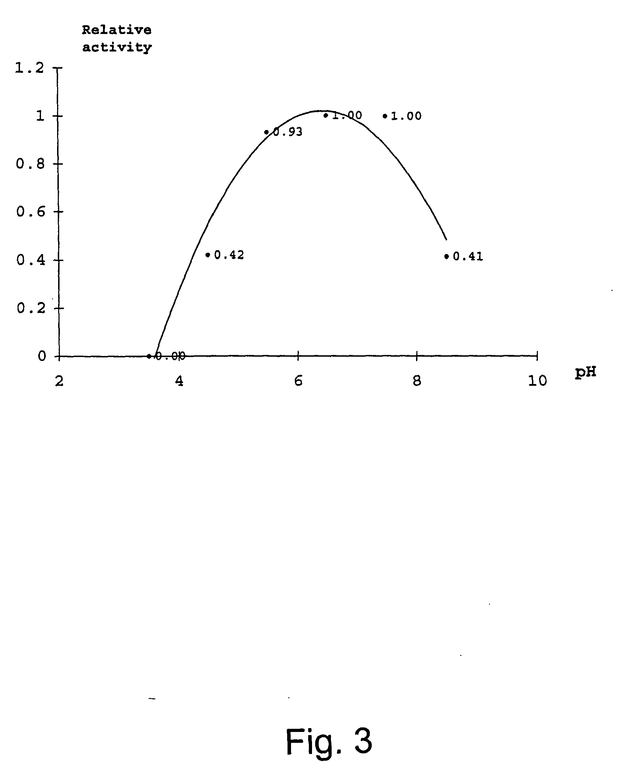

2,6-β-D-fructan hydrolase activities were determined in the 2,6-β-D-fructan hydrolase assay at 37°C and at pH-values 3.5 to 8.5, buffers being pH adjusted with either HCl or NaOH. The reults were plotted as activities relative to the maximum activity against the pH-value as shown in figure 3. It was observed from the result that the 2,6-β-D-fructan hydrolase has a near neutral optimum between pH 6.5 and 7.0. In addition, 2,6-β-D-fructan hydrolase samples were preincubated for 2 hours at 37°C at the different pH-values with less than 10% reduction in activity. Accordingly 2,6-β-D-fructan hydrolase is found fairly stable in the pH range 4.5 to 8.5.

### EXAMPLE 6

### Determination of temperature-activity range

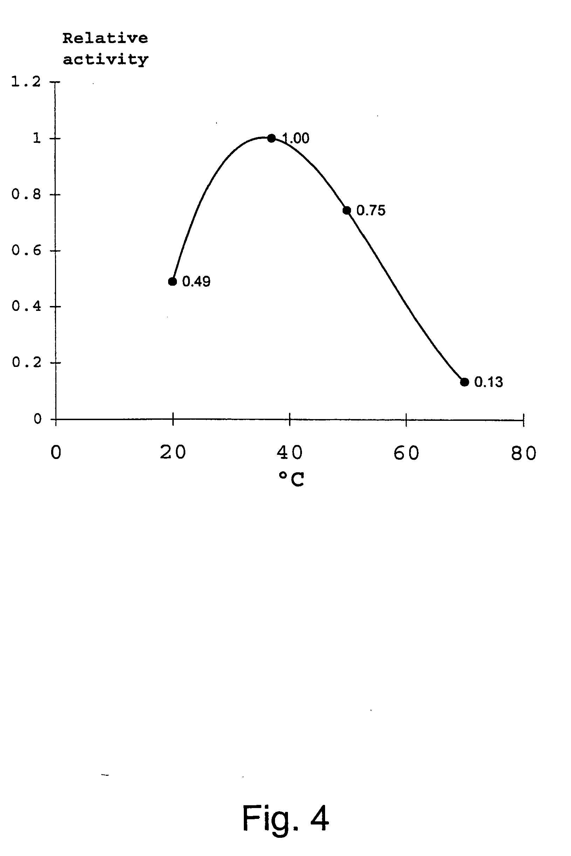

2,6-β-D-fructan hydrolase activities were determined in the 2,6-β-D-fructan hydrolase assay at pH 6.5 and at temperatures 20°C, 37°C, 50°C, and 70°C. The results were plotted as activities relative to the maximum activity against the temperature as shown in figure 4. It was observed that the 2,6-β-D-fructan hydrolase has a temperature optimum at about 37°C.

### EXAMPLE 7

### Biofilm disintegration by 2,6-b-D-fructan hydrolase and combined systems containing 2,6-b-D-fructan hydrolase.

Biofilm was made on stainless steel, and activity of enzymes against microbial biofilm was determined as a release of biofilm from the surface.

A preculture is made by mixing 220 ml tap-water with 10 ml Tryptone Soya Broth (TSB, Oxoid CM129), the water was incubated for 48 hours at approximately 20°C. The culture was then re-circulated, by use of a peristaltic pump, through a modified Robbins Device with 25 steel plugs holding 25 steel discs. After 3 days, diluted substrate (1/7 TSB + glucose 1 g/L) was added continuously to the culture during 48 hours as the culture was circulated trough the Robbins Device.

Biofilm with microorganisms from tap-water was formed on the steel discs in the Robbins Device. The effects of enzymes against this biofilm was evaluated in two ways: a) by removing all discs from the Robbins Device and performing the enzyme treatment in a micro-titer plate (24 wells, pH 6.5, 50°C for 2 hours) or b) by making a biofilm in two Robbins devices with a serial connection, so that an identical biofilm was made in both devices. The Robbins devices was kept assembled and the enzyme solution (pH 6.5, 50°C for 2 hours), was circulated through one of the Devices and a control solution without enzyme is circulated through the other device. After the enzyme treatment all discs were removed from the Robbins devices and the enzyme effect was evaluated.

The amount of biofilm left on the steel surface was evaluated either by fluorescent microscopy where all microbial cells on the surface were stained with a fluorescent dye (DAPI), or by conductance measurements.

*Fluorescence microscopy:* The DNA-binding fluorochrome DAPI (4',6-diamidino-2-phenylindole, Sigma D-9542) was used as an indicator for the total cell number. The discs with biofilm were after enzyme treatment incubated in the dark for 5 min with DAPI (3 mM). The stained cells were examined under a 100 x magnification oil immersion fluorescence objective on an Olympus model BX50 microscope equipped with a 200 W mercury burner. The filter combination used for viewing DAPI-stained cells was a 330-385 nm excitation filter and a 420 barrier filter (Olympus cube model U-MWU).

*Conductance measurements:* Indirect Malthus measurements were used when enumerating adherent cells on the substrata (Johnston M. and Jones M.V.; ***Disinfection tests with intact* biofilms: combined use of the modified Robbins *Device* with *impedance detection;*** J. Microbiol.; 1995; Methods 21; pp.15-26; Johansen C., Falholt P. and Gram L.; ***Enzymatic removal and disinfection of bacterial** biofilms;* Appl. Environ. Microbiol.; 1997; vol. 63; pp. 3724-3728). The discs were after incubation with enzymes transferred to Malthus tubes containing 3 ml of growth media (TSB) in the outer tube and 0.5 ml 0.1 M KOH in the inner tube (Dezenclos T., Ascon-Cabrera M., Ascon D., Lebeault J.-M. and Pauss A.; ***Optimisation of the indirect impedancemetry technique; a handy technique for microbial growth measurements;*** Appl. Microbiol. Biotechnol.; 1994; vol. 42; pp. 232-238). Tubes were placed in a Malthus 2000 (Malthus Flexi 2000, Malthus Instrument FLimited) and incubated at 25°C.

Carbon dioxide produced by the bacteria were absorbed by the KOH and thereby altering the conductivity. Changes in conductance were plotted against time and the detection time (DT) was determined as the time taken from start of the measurement until a rapid change in conductance was detecable by the Malthus. The DT was related to the number of cells present at the start of the test by use of a calibration curve, which was constructed for each organism by inoculating Malthus tubes with a tenfold dilution series (Johansen *et al.* 1997, supra).

Biofilm removal by the recombinant 2,6-β-D-fructan hydrolase was evaluated both for the 2,6-β-D-fructan hydrolase alone and in combination with other enzyme activities by combining with BioCip-membrane. BioCip-membrane (Novo Nordisk A/S, Denmark) is a commercially available multicomponent enzyme preparation containing protease activity and a wide range of carbo-hydrases including cellulase, arabanase, hemi-cellulase, β-glucanase, pectinases and xylanase activities.

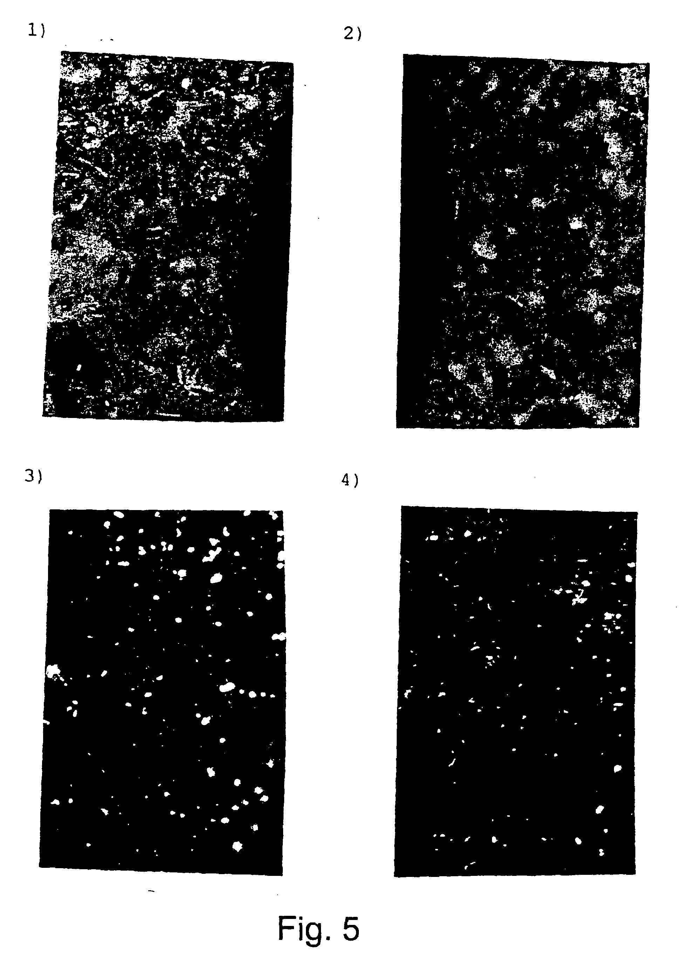

A significant removal of biofilm was observed by microscopy (Figure 5), both when 2,6-β-D-fructan hydrolase was used alone and when used in combination with Bio-Cip membrane. Figure 5 shows DAPI staining of microbial cells on steel surfaces. 1) and 2) are the control biofilms which are cleaned without enzymes, and 3) and 4) are the same biofilm cleaned with 2,6-b-D-fructan hydrolase (10 mg/L) for 2 hours at 50°C and pH 6.5.

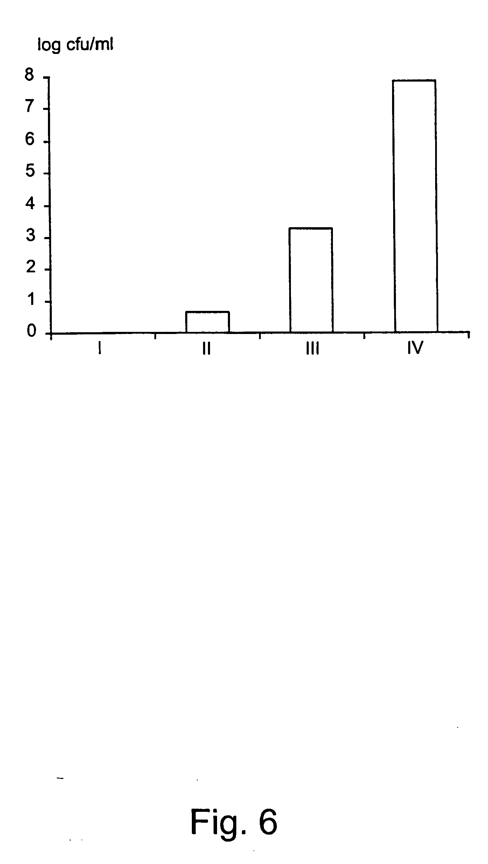

BioCip-membrane also removes biofilm from the steel surface, but when combining BioCip-membrane with 2,6-ß-D-fructan hydrolase, the removal was significantly increased. Figure 6 shows log cfu/ml, i.e. the logarithm to the number of cells per ml cleaning solution removed/released from a biofilm evaluated by conductance measurements on the biofilm cells remaining on the steel surface after cleaning the biofilm with I) no enzyme, II) 100 ppm Bio-Cip membrane, III) 1000 ppm Bio-Cip membrane and IV) 100 ppm Bio-Cip membrane and 10 mg/l 2,6-β-D-fructan hydrolase. A huge synergistic effect was seen when cleaning with both Bio-Cip membrane and 2,6-β-D-fructan hydrolase.

The 2,6-β-D-fructan hydrolase has shown to be an important enzyme activity for biofilm degradation, and the overall biofilm degradation of complex microbial biofilms can be further increased if 2,6-β-D-fructan hydrolase is combined with a mix of different enzymes.

### EXAMPLE 8

Levanase activity of *Paenibacillus* species was evaluated on agar plates made of ten times diluted TY-medium supplemented with stained levan and incubated overnight at 37°C. For colonies expressing the 2,6-β-D-fructan hydrolase clearing zones (decoloration of the stained levan) appeared. The following results were obtained:

| | |
|---|---|
| Organism | levanase activity |
| Paenibacillus amylolyticus DSM | + |
| 12458 | |

the strain of *Paenibacillus* amylolyticus expressed polypeptides having 2,6-β-D-fructan hydrolase activity.

### EXAMPLE 9

### Cloning of the SEQ ID No:2 encoding 2,6-β-D-fructan hydrolase into Bacillus subtilis and purification of the 2,6-β-D-fructan hydrolase.

The 2,6-β-D-fructan hydrolase encoding DNA sequence SEQ ID No:2 was PCR amplified using the PCR primer set consisting of these two oligo nucleotides:
Lev-amy.upper.PstI
   5'-CAT TCT GCA GCA GCG GCG GCT ATG GCT GTA CTT GC-3'
Lev-amy.lower.BglII
   5'-CGC GGA TCC AGA TCT TAC ATG GAG TCC AAG CTT TC -3'

Restriction sites PstI and BglII are underlined Chromosomal DNA isolated from *Paenibacillus amylolyticus* DSM 12458 was used as template in a PCR reaction using Amplitaq DNA Polymerase (Perkin Elmer) according to manufacturers instructions. The PCR reaction was set up in PCR buffer (10 mM Tris-HCl, pH 8.3, 50 mM KCl, 1.5 mM MgCl₂, 0.01 % (w/v) gelatin) containing 200 µM of each dNTP, 2.5 units of AmpliTaq polymerase (Perkin-Elmer, Cetus, USA) and 100 pmol of each primer. The PCR reactions was performed using a DNA thermal cycler (Landgraf, Germany). One incubation at 94°C for 1 min followed by thirty cycles of PCR performed using a cycle profile of denaturation at 94°C for 30 sec, annealing at 60°C for 1 min, and extension at 72 °C for 2 min. Five-µl aliquots of the amplification product was analysed by electrophoresis in 0.7 % agarose gels (NuSieve, FMC) .

The PCR products generated as described above were purified using QIAquick PCR purification kit (Qiagen, USA) according to the manufacturer's instructions. The purified DNA was eluted in 50 µl of 10mM Tris-HCl, pH 8.5. pMOL944 (described *supra*) was digested with PstI and Bcl1, the purified PCR fragment was digested with PstI and HindIII (this fragment covering the coding sequence until the HindIII site) and plasmid pLiH937 from strain DSM 12406 digested with HindIII-BglII (this fragment covering the rest of the encoding sequence) electrophoresed in 0.8 % low gelling temperature agarose (SeaPlaque GTG, FMC) gels, the relevant fragments were excised from the gels, and purified using QIAquick Gel extraction Kit (Qiagen, USA) according to the manufacturer's instructions. The isolated PCR DNA fragment and the 2.3 kb HindIII-BglII fragment from pLiH938 (see DNA sequence of invention) were then ligated to the PstI-BclI digested and purified pMOL944. The ligation was performed overnight at 16°C using 0.5 µg of each DNA fragment, 1 U of T4 DNA ligase and T4 ligase buffer (Boehringer Mannheim, Germany).

The ligation mixture was used to transform competent B. subtilis SHa273. The transformed cells were plated onto LBPG-10 µg/ml of Kanamycin-agar plates. After 18 hours incubation at 37°C colonies were seen on plates. Several clones were analyzed by isolating plasmid DNA from overnight culture broth.

One such positive clone was restreaked several times on agar plates as used above, this clone was called LiH1528 The clone LiH1528 was grown overnight in TY-10µg/ml Kanamycin at 37°C, and next day 1 ml of cells were used to isolate plasmid from the cells using the Qiaprep Spin Plasmid Miniprep Kit #27106 according to the manufacturers recommendations for B.subtilis plasmid preparations.

The clone LiH 1528 was grown in 200ml BPX media with 15 µg/ml of Kanamycin in 500ml two baffled shakeflasks for 4-5 days at 37°C at 300 rpm.

200 ml of the shake flask culture fluid of the clone LiH1528 was collected and centrifuged at 15000 rpm for 20 minutes in 50 ml tubes.

The supernatants were filtered through a Seitz K250 filter plate followed by a Seitz EKS filter plate to remove the production organism completely. Solid ammonium sulfate was added to the filtrate to 1.8M final concentration and the filtrate was applied to a SOURCE Phenyl column (Amersham Pharmacia Biotech) equilibrated in 10mM KH₂PO₄/NaOH, 1.8 M (NH₄)₂SO₄, pH 7. After washing the column with the equilibration buffer, the column was eluted with a linear decreasing ammonium sulfate gradient (1.8 → 0 M). Fractions from the column were checked for levanase activity and active fractions were pooled and concentrated on a Amicon ultrafiltration cell equipped with a 10kDa cut-off regenerated cellulose membrane. The concentrated enzyme was fractionated on a Superdex200 HR16/60 column (Amersham Pharmacia Biotech) equilibrated in 25mM HEPES/NaOH, 100 mM NaCl, pH 8.0. Fractions from the column were checked for levanase activity and active fractions were purity checked on a SDS-PAGE gel. Pure fractions were pooled as purified levanase. The following N-terminal sequence of the purified protein was determined: Ser (54)-Ala-Ser-Lys-Ser-Asn-Thr-Asn-Leu-Ile-Gly-Trp-Gln-Val-Lys-Gly-Lys-Gly-; by Edman degradation of the electroblottet enzyme as known in the art.

### EXAMPLE 10

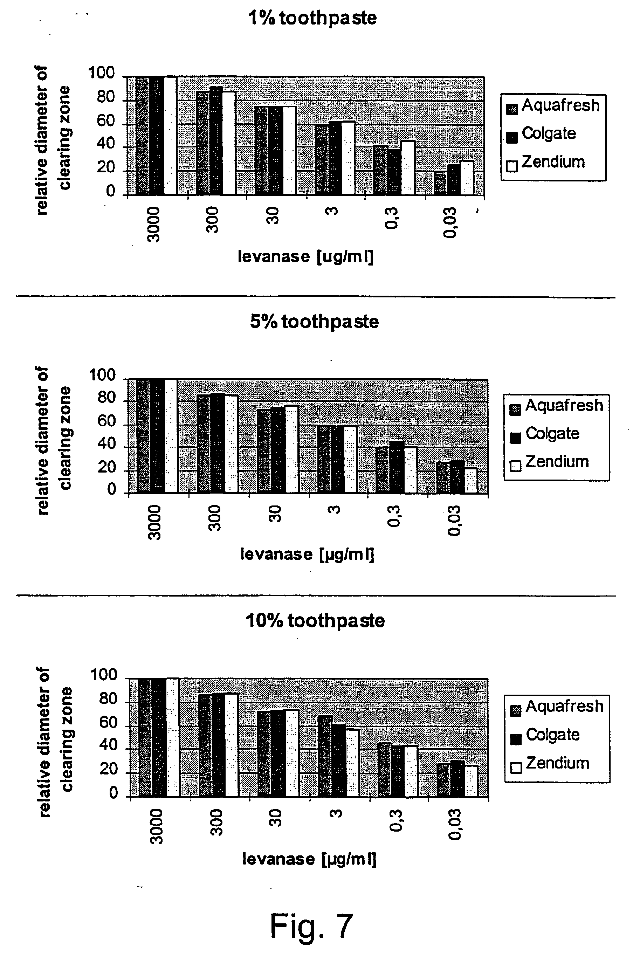

The 2,6-β-D-fructan hydrolase of SEQ ID No:1 was tested for activity in toothpaste using a spot test on plates containing stained levan. Samples of different tooth pastes in different concentrations containing difference amounts of 2,6-β-D-fructan hydrolase was places on the stained levan plates and clearing zone was measured after 7 hours incubation at 37°C showing presence of active 2,6-β-D-fructan hydrolase. The size of the clearing zone was dependent on the amount of 2,6-β-D-fructan hydrolase in the sample. The activity is independent of the concentration of toothpaste in the samples (i.e. similar diameter with increasing toothpaste concentration). The levanase is thus active in a toothpaste formulation. The following tooth pastes was used:
Commercial Aquafresh 'Fresh mint / triple protection' (SmithKlime Beecham Consumer HealthCare Ballerup, Denmark).
Commercial Colgate 'Micro-formula / tandsten kontrol' (Colgate Palmolive Dk-2600 Glostrup, Denmark).
Commercial Zendium: 'Cool Mint med xylitol' (Blumøller A/S Dk-5100 Odense C, Denmark). The results are shown in figure 7.

### EXAMPLE 11

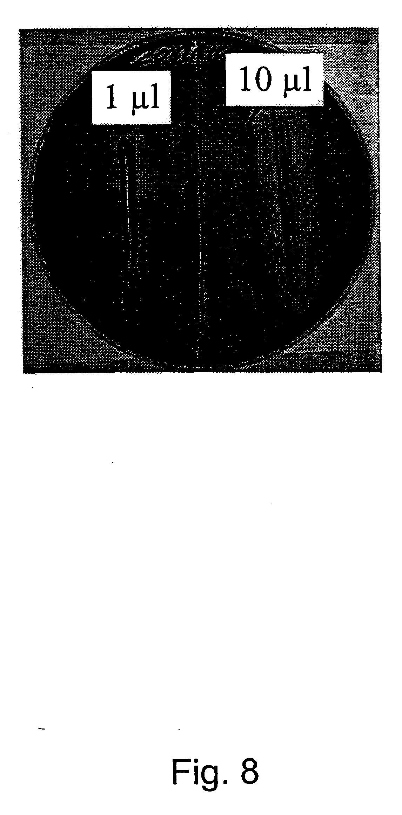

2,5 g of the undiluted Zendium tooth paste of example 10 was mixed with 1 µl or 10 µl 2,6-β-D-fructan hydrolase of the mature part of SEQ ID No:1, and applied to plates containing stained levan and kept overnight at 37°C temperature. The clearing zones that appeared surrounding the smear of toothpaste showed in figure 8, shows that the 2,6-β-D-fructan hydrolase is active after overnight incubation, and shows that the enzyme is stabile and active in an undiluted toothpaste formulation.

## Claims

1. An isolated polypeptide having endo-2,6-β-D-fructan hydrolases activity and having an amino acid sequence which has at least 90%, preferably at least 95%, more preferably 100% identity with amino acids 32 to 923 for the mature polypeptide of SEQ ID NO:1.

2. The polypeptide of claim 1, comprising amino acids 32 to 923 of SEQ ID NO:1.

3. The polypeptide of claim 1, consisting of amino acids 32 to 923 of SEQ ID NO:1.

4. The polypeptide of claim 1, which is encoded by the nucleic acid sequence inserted into plasmid pSJ1678 present in *E. coli* DSM 12406.

5. The polypeptide of any of claims 1-4 which has at least 20% of the 2,6-β-D-fructan hydrolase activity of SEQ ID No:1.

6. An isolated nucleic acid sequence comprising a nucleic acid sequence which encodes the polypeptide of any of the preceding claims.

7. The isolated nucleic acid sequence of claim 6 comprising a nucleic acid sequence having at least one mutation in the mature polypeptide coding sequence of SEQ ID No:2, in which the mutant nucleic acid sequence encodes a polypeptide consisting of amino acids 32 to 923 of SEQ ID NO:1.

8. The isolated nucleic acid sequence of claim 6 produced by (a) hybridizing a DNA under high stringency conditions with (i) nucleotides 94 to 2769 of SEQ ID No:2, (ii) the cDNA sequence Comprised in nucleotides 94 to 2769 of SEQ ID No:2 or (iii) a complementary strand of (i) or (ii); and (b) isolating the nucleic acid sequence.

9. The nucleic acid sequence of claim 6, wherein said sequence consists of nucleotides 94 to 2769 of SEQ ID No:2.

10. A nucleic acid construct comprising a nucleic acid sequence of claim 6 operably linked to one or more control sequences that direct the production of the polypeptide in a suitable expression host.

11. A recombinant expression vector comprising the nucleic acid construct of claim 10.

12. A recombinant host cell comprising the nucleic acid construct of claim 10.

13. A method for producing a mutant nucleic acid sequence, comprising (a) introducing at least one mutation into the mature polypeptide coding sequence of SEQ ID No:2, wherein the mutant nucleic acid sequence encodes a polypeptide consisting of amino acids 32 to 923 of SEQ ID NO:1 and (b) recovering the mutant nucleic acid sequence.

14. A method for producing the polypeptide of any of claims 1-5 comprising (a) cultivating a strain under conditions suitable for production of the polypeptide to produce a supernatant comprising the polypeptide; and (b) recovering the polypeptide.

15. The method of claim 14, wherein the strain comprises the mutant nucleic acid sequence of claim 7.

16. The method of claim 14, wherein the strain is a host cell comprising the nucleic acid construct of claim 10.

17. The method of claim 16, wherein the nucleic acid sequence is a mutant nucleic acid sequence according to claim 7.

18. A composition comprising the 2,6-β-D-fructan hydrolase obtained from the method of claim 14.

19. A non-therapeutic process for disintegrating a microbial biofilm comprising contacting said biofilm with a endo-2,6-β-D-fructan hydrolase of claims 1-5 in an aqueous medium.

20. The process of claim 19, wherein said biofilm is present on a hard surface.

21. The process of claim 20, wherein the hard surface is essentially non-permeable to microorganisms.

22. The process of claim 21, wherein the hard surface is selected from the group consisting of metal, synthetic polymers, rubber, board, glass, wood, paper, textile, concrete, rock, marble, gypsum, ceramics, paint, enamel.

23. The process of claim 22, wherein the hard surface is a member of a system holding, transporting, processing or is in contact with aqueous solutions.

24. The process of claim 23, wherein the hard surface is a member of a system selected from the group consisting of water supply systems, food processing systems, cooling systems, chemical processing systems, pharmaceutical processing systems and wood processing systems.

25. The process of claim 24, wherein the hard surface is a member of system unit selected from the group consisting if pipes, tanks, pumps, membranes, filters, heat exchangers, centrifuges, evaporators, mixers, spray towers, valves and reactors.

26. The process of claim 22, wherein the hard surface is a member of a utensil selected from the group consisting of endoscopes, prosthetic devices and medical implants.

27. The process of claims 19-25, wherein the process is a Cleaning In Place (CIP) process.

28. The polypeptide of claim 1 for disintegrating a microbial biofilm on a hard surface of biological origin.

29. The polypeptide of claim 28, wherein the hard surface is selected from the group consisting of mucous membranes, skin, teeth, hair and nails.

30. The polypeptide of claim 29, wherein the hard surface is the surface of a tooth and the biofilm is dental plaque.

31. The polypeptide of claim 29, wherein the hard surface is the surface of a mucous membrane tooth and the biofilm is present in a human lung.

32. A process for reducing bio-corrosion of a hard surface whereon a biofilm is present comprising applying the process of claim 19.

33. A non-therapeutic process for hydrolyzing slime comprising contacting the slime with a 2,6-β-D-fructan hydrolase of any of claims 1-5 in an aqueous medium.

34. The process of any of the claims 19-27, 32 and 33, wherein the aqueous medium further comprises one or more components selected from the group consisting of hydrolase enzymes and antimicrobial agents.

35. The process of claim 34, wherein the hydrolase enzyme is selected from the group consisting of cellulases, hemicellulases, xylanases, amylases, lipases, proteases and pectinases.

36. The process of claim 34, wherein the antimicrobial agent is selected from the group consisting of enzymatic and non-enzymatic biocides.

37. The process of claim 36, wherein the enzymatic biocide is an oxidoreductase selected from the group consisting of laccases and peroxidases.

38. A process for producing 2,6-β-D-fructose oligomers comprising contacting a substrate selected from the group consisting of levan and phlein with a 2,6-β-D-fructan hydrolase of any of claims 1-5.

39. A process for producing fructose comprising
a) contacting a substrate selected from the group consisting of phlein and levan with a 2,6-ß-D-fructan hydrolase of any of the claims 1-5 whereby 2,6-ß-D-fructose oligo saccharides are generated, and
b) contacting the generated 2,6-β-D-fructose oligosaccharides with a 2,6-β-D-fructan exo-hydrolase.

40. The composition of claim 18, wherein the composition is an oral care composition comprising an amount of 2,6-β-D-fructan hydrolase in the range of 0.001 LRU to 1000 LRU/ml.

41. The oral care composition of claim 40, wherein the composition is in a form selected from the group consisting of powder, paste, gel, liquid, ointment and tablet.

42. The oral care composition of claim 41, wherein the composition is selected from the group consisting of toothpaste, dental cream, gel, tooth powder, odontic mouth wash, pre brushing rinse formulations, post brushing rinse formulations, chewing gum, lozenges, and candy.

43. The oral care composition of claim 42, further comprising one or more enzymes from the group consisting of dextranase, mutanases, oxidases, peroxidases, laccases, proteases, endoglucosidases, lipases and amylases.

44. The oral care composition according to claim 43, further comprising one or more components selected from the group consisting of abrasives, humectants, thickeners, binders, foaming agents, soaps, surfactants, sweeteners, flavours, whitening/bleaching agents, anti-bacterial agents, fluoride sources, dyes/colorants, preservatives, vitamins, pH-adjusting agents, anti-caries agents and desensitizing agents.

45. The composition of claim 18, wherein the composition is a detergent additive comprising a 2,6-ß-D-fructan hydrolase of any of the claims 1-5.

46. The detergent additive of claim 45 further comprising an enzyme selected from the group consisting of protease, a lipase, a cutinase, an amylase, a carbohydrase, a cellulase, a pectinase, a mannanase, an arabinase, a galactanase, a xylanase, an oxidase, a laccase, and a peroxidase.

47. The composition of claim 18, wherein the composition is a detergent composition comprising a surfactant.

48. The detergent composition of claim 47, further comprising an enzyme selected from the group consisting of protease, a lipase, a cutinase, an amylase, a carbohydrase, a cellulase, a pectinase, a mannanase, an arabinase, a galactanase, a xylanase, an oxidase, a laccase, and a peroxidase.

49. A process for preparation of a medicament comprising a 2,6-β-D-fructan hydrolase of claims 1-5 for disintegration of plaque present on a human or animal tooth.

50. A process for preparation of a medicament comprising a 2,6-β-D-fructan hydrolase of claims 1-5 for disintegration of a biofilm present on a human or animal mucous membrane.

## Patentansprüche

1. Ein isoliertes Polypeptid mit einer endo-2,6-β-D-Fructanhydrolase-Aktivität und mit einer Aminosäuresequenz, die zu mindestens 90 %, vorzugsweise mindestens 95 %, bevorzugter mindestens 100 % identisch mit den Aminosäuren 32 bis 923 des reifen Polypeptides der SEQ ID Nr. 1 ist.

2. Das Polypeptid nach Anspruch 1, das die Aminosäuren 32 bis 923 der SEQ ID Nr. 1 umfasst.

3. Das Polypeptid nach Anspruch 1, das aus den Aminosäuren 32 bis 923 der SEQ ID Nr. 1 besteht.

4. Das Polypeptid nach Anspruch 1, das durch die Nukleinsäuresequenz kodiert wird, die in das Plasmid pSJ1678 inseriert ist, das in *E. coli* DSM 12406 vorliegt.

5. Das Polypeptid nach einem der Ansprüche 1 bis 4, das mindestens 20 % der endo-2,6-β-D-Fructanhydrolase-Aktivität der SEQ ID Nr. 1 besitzt.

6. Eine isolierte Nucleinsäuresequenz, die eine Nucleinsäuresequenz umfasst, die für das Polypeptid nach einem der vorhergehenden Ansprüchen kodiert.

7. Die isolierte Nucleinsäuresequenz nach Anspruch 6, die eine Nucleinsäuresequenz mit mindestens einer Mutation in der für das reife Polypeptid kodierende Sequenz der SEQ ID Nr. 2 besitzt, in der die Mutantennucleinsäuresequenz ein Polypeptid kodiert, das aus den Aminosäuren 32 bis 923 der SEQ ID Nr. 1 besteht.

8. Die Isolierte Nucleinsäuresequenz nach Anspruch 6, hergestellt durch (a) Hybridisieren einer DNA unter stringenten Bedingungen mit (i) Nucleotiden 94 bis 2769 der SEQ ID Nr. 2, (ii) der cDNA-Sequenz, die in den Nucleotiden 94 bis 2769 der SEQ ID Nr. 2 enthalten ist, oder (iii) einem komplementären Strang von (i) oder (ii) und (b) Isolieren der Nucleinsäuresequenz.

9. Die Nucleinsäuresequenz nach Anspruch 6, wobei die Sequenz aus den Nucleotiden 94 bis 2769 der SEQ ID Nr. 2 besteht.

10. Ein Nucleinsäurekonstrukt, das eine Nucleinsäuresequenz nach Anspruch 6 umfasst, die operativ an eine oder mehrer Kontrollsequenz(en) verbunden ist/sind, die die Produktion des Polypeptides in einem geeigneten Expressionswirt steuert.

11. Ein rekombinanter Expressionsvektor, der das Nucleinsäurekonstrukt nach Anspruch 10 umfasst.

12. Ein rekombinante Wirtszelle, die das Nucleinsäurekonstrukt von Anspruch 10 umfasst.

13. Ein Verfahren zur Herstellung einer Mutantennucleinsäuresequenz, die umfasst (a) den Einbau mindestens einer Mutation in die reife Polypeptid-kodierende Sequenz der SEQ ID Nr. 2, wobei die Mutantennucleinsäuresequenz ein Polypeptid kodiert, das aus den Aminosäuren 32 bis 923 der SEQ ID Nr. 1 besteht, und (b) Wiedergewinnen der Mutantennucleinsäuresequenz.

14. Ein Verfahren zur Herstellung des Polypeptides nach einem der Ansprüche 1 bis 5, umfassend (a) das Kultivieren eines Stammes unter Bedingungen, die zur Herstellung des Polypeptides geeignet sind, wodurch ein Überstand hergestellt wird, der das Polypeptid umfasst, und (b) Wiedergewinnen des Polypeptides.

15. Das Verfahren nach Anspruch 14, wobei der Stamm die Mutantennucleinsäure von Anspruch 7 umfasst.

16. Das Verfahren nach Anspruch 14, wobei der Stamm eine Wirtszelle ist, die das Nucleinsäurekonstrukt von Anspruch 10 umfasst.

17. Das Verfahren nach Anspruch 16, wobei die Nucleinsäuresequenz eine Mutantennucleinsäuresequenz nach Anspruch 7 ist.

18. Eine Zusammensetzung, umfassend die 2,6-β-D-Fructanhydrolase, die nach dem Verfahren von Anspruch 14 erhalten wird.

19. Ein nicht-therapeutisches Verfahren zum Abbau eines mikrobiellen Biofilm, das das in Kontakt bringen des Biofilms mit einer endo-2,6-β-D-Fructanhydrolase nach Anspruch 1 bis 5 in einem wässrigen Medium umfasst.

20. Das Verfahren nach Anspruch 19, wobei der Biofilm auf einer harten Oberfläche vorliegt.

21. Das Verfahren nach Anspruch 20, wobei die harte Oberfläche im wesentlichen nicht permeabel für Mikroorganismen ist.

22. Das Verfahren nach Anspruch 21, wobei die harte Oberfläche aus der Gruppe bestehend aus Metall, synthetischen Polymeren, Gummi, Karton, Glass, Holz, Papier, Textilien, Beton, Stein, Marmor, Gips, Keramik, Anstrich und Email ausgewählt ist.

23. Das Verfahren nach Anspruch 22, wobei die harte Oberfläche ein Teil eines Halte-, Transport- oder Verarbeitungssystems ist oder in Kontakt mit wässrigen Lösungen steht.

24. Das Verfahren nach Anspruch 23, wobei die harte Oberfläche ein Teil eines Systems ist, das aus der Gruppe von Wasserzuführsystemen, Lebensmittel-verarbeitenden Systemen, Kühlsystemen, Chemikalien-verarbeitenden Systemen, Arzneimittel-verarbeitenden Systemen und Holz-verarbeitenden Systemen ausgewählt ist.

25. Das Verfahren nach Anspruch 24, wobei die harte Oberfläche ein Teil einer Systemeinheit ist, ausgewählt aus der Gruppe bestehend aus Rohren, Tanks, Pumpen, Membranen, Filter, Wärmeaustauscher, Zentrifugen, Verdampfer, Mischer, Sprühtürmen, Ventile und Reaktoren.

26. Das Verfahren nach Anspruch 22, wobei die harte Oberfläche ein Teil einer Utensilie ist, die aus der Gruppe ausgewählt ist, bestehend aus Endoskopen, prosthetischen Vorrichtungen und medizinischen Implantaten.

27. Das Verfahren nach den Ansprüchen 19 bis 25, wobei das Verfahren ein Verfahren zum Reinigen an Ort und Stelle ("Cleaning-In-Place"(CIP)) ist.

28. Das Polypeptid nach Anspruch 1, wobei der Abbau eines mikrobiellen Biofilms auf einer harten Oberfläche von biologischem Ursprung ist.

29. Das Polypeptid nach Anspruch 28, wobei die harte Oberfläche aus der Gruppe bestehend aus Mukosamembranen, Haut, Zähne, Haare und Nägel ausgewählt ist.

30. Das Polypeptid nach Anspruch 29, wobei die harte Oberfläche die Oberfläche eine Zahns ist, und der Biofilm ein Zahnbelag ist.

31. Das Polypeptid nach Anspruch 29, wobei die harte Oberfläche die Oberfläche eines Schleimhautzahns ("mucous membrane tooth") ist, und er Biofilm liegt in der Lunge vor.

32. Ein Verfahren zum Vermindern der Biokorrosion einer harten Oberfläche, auf der ein Biofilm vorliegt, das das Anwenden des Verfahrens nach Anspruch 19 umfasst.

33. Ein nicht-therapeutisches Verfahren zum Hydrolysieren von Schleim, umfassend das in Kontakt bringen des Schleims mit einer -2,6-β-D-Fructanhydrolase nach einem der Ansprüche 1 bis 5 in wässrigem Medium.

34. Das Verfahren nach einem der Ansprüche 19 bis 27, 32 und 33, wobei das wässrige Medium weiterhin eine oder mehrere Komponente(n) umfasst, ausgewählt aus der Gruppe bestehend aus Hydrolasenzymen und antimikrobiellen Agenzien.

35. Das Verfahren nach Anspruch 34, wobei das Hydrolaseenzym aus der Gruppe bestehend aus Cellulasen, Hemicellulasen, Xylanasen, Amylasen, Lipasen, Proteasen und Pektinasen ausgewählt ist.

36. Das Verfahren nach Anspruch 34, wobei das antimikrobielle Agens aus der Gruppe bestehend aus enzymatischen und nicht-enzymatischen Bioziden ausgewählt ist.

37. Das Verfahren nach Anspruch 36, wobei das enzymatische Biozid eine Oxidoreduktase ist, die aus der Gruppe bestehend aus Laccase und Peroxidase ausgewählt ist.

38. Ein Verfahren zur Herstellung von 2,6-β-D-Fructose-Oligomeren, umfassend das in Kontakt bringen eines Substrates ausgewählt aus der Gruppe bestehend aus Lävan und Phlein mit einer 2,6-β-D-Fructanhydrolase nach einem der Ansprüche 1 bis 5.

39. Ein Verfahren zur Herstellung von Fructose, umfassend
(a) das in Kontakt bringen eines Substrates ausgewählt aus der Gruppe bestehend aus Phlein und Lävan mit einer 2,6-β-D-Fructanhydrolase nach einem der Ansprüche 1 bis 5, wobei 2,6-β-D-Fructose-Oligosaccaride erzeugt werden, und
(b) das in Kontakt bringen der erzeugten 2,6-β-D-Fructose-Oligosaccaride mit einer 2,6-β-D-Fructan-exo-hydrolase.

40. Die Zusammensetzung nach Anspruch 18, wobei die Zusammensetzung eine Zahnpflegezusammensetzung ist, die eine Menge der 2,6-β-D-Fructanhydrolase im Bereich von 0,001 LRU bis 1 000 LRU/ml umfasst.

41. Die Zahnpflegezusammensetzung nach Anspruch 40, wobei die Zusammensetzung in einer Form vorliegt, die ausgewählt ist aus der Gruppe bestehend aus Pulver, Paste, Gel, Flüssigkeit, Salbe und Tablette.

42. Die Zahnpflegezusammensetzung nach Anspruch 41, wobei die Zusammensetzung ausgewählt ist aus der Gruppe bestehend aus Zahnpasta, Zahncremes, Gele, Zahnpulver, Zahnmundspülungen, Spülungen vor dem Bürsten, Spülungen nach dem Bürsten, Kaugummi, Pastillen und Süßigkeiten.

43. Die Zahnpflegezusammensetzung nach Anspruch 42, die weiterhin ein oder mehrere Enzym(e) aus der Gruppe bestehend aus Dextranasen, Mutanasen, Oxidasen, Peroxidasen, Laccasen, Proteasen, Endoglucosidasen, Lipasen und Amylasen.

44. Die Zahnpflegezusammensetzung nach Anspruch 43, die weiterhin eine oder mehrere Komponente(n) umfasst, ausgewählt aus der Gruppe bestehend aus Schleifmittel, Benetzungsmittel, Verdickungsmittel, Binder, Schaummittel, Seifen, Surfactanten, Süssungsmittel, Geschmacksmittel, Weißmittel/Bleichmittel, antibakterielle Mittel, Fluoridquellen, Farbstoffe/Färbemittel, Konservierungsmittel, Mittel zum pH-Einstellen, Antikariesmittel und Desensibilisierungsmittel.

45. Die Zusammensetzung nach Anspruch 18, wobei die Zusammensetzung ein Detergenzadditiv ist, das 2,6-β-D-Fructanhydrolase nach einem der Ansprüche 1 bis 5 enthält.

46. Das Detergenzadditiv nach Anspruch 45, das weiterhin ein Enzym umfasst, ausgewählt aus der Gruppe bestehend aus Protease, Lipase, Cutinase, Amylase, Carbohydrase, Cellulase, Pektinase, Mannase, Arabinase, Galactanase, Xylanase, Oxidase, Laccase und Peroxidase.

47. Die Zusammensetzung nach Anspruch 18, wobei die Zusammensetzung eine Detergenzzusammensetzung ist, die ein Surfactant umfasst.

48. Die Detergenzzusammensetzung nach Anspruch 47, die weiterhin ein Enzym enthält, ausgewählt aus der Gruppe bestehend aus Protease, Lipase, Cutinase, Amylase, Carbohydrase, Cellulase, Pektinase, Mannase, Arabinase, Galactanase, Xylanase, Oxidase, Laccase und Peroxidase.

49. Ein Verfahren zur Herstellung eines Arzneimittels, das eine 2,6-β-D-Fructanhydrolase nach den Ansprüchen 1 bis 5 enthält, zum Abbau eines Belags, der auf einem menschlichen oder tierischen Zahn vorliegt.

50. Ein Verfahren zur Herstellung eines Arzneimittel, das eine 2,6-β-D-Fructanhydrolase nach den Ansprüchen 1 bis 5 enthält, zum Abbau eines Biofilm, der auf einer menschlichen oder tierischen Schleimhaut vorliegt.

## Revendications

1. Polypeptide isolé ayant une activité endo-2,6-β-D-fructosane hydrolase et ayant une séquence d'acides aminés qui a une identité d'au moins 90 %, de manière préférée d'au moins 95 %, plus préférentiellement d'au moins 100 % avec les acides aminés 32 à 923 du polypeptide mature de SEQ ID NO:1.

2. Polypeptide selon la revendication 1, comprenant les acides aminés 32 à 923 de SEQ ID NO:1.

3. Polypeptide selon la revendication 1, consistant en les acides aminés 32 à 923 de SEQ ID NO:1.

4. Polypeptide selon la revendication 1, qui est codé par la séquence d'acides nucléiques insérée dans le plasmide pSJ1678 présent chez *E. coli* DSM 12406.

5. Polypeptide selon l'une quelconque des revendications 1-4 qui a au moins 20 % de l'activité 2,6-β-D-fructosane hydrolase de SEQ ID NO:1.

6. Séquence d'acides nucléiques isolée comprenant une séquence d'acides nucléiques qui code pour le polypeptide de l'une quelconque des revendications précédentes.

7. Séquence d'acides nucléiques isolée selon la revendication 6, comprenant une séquence d'acides nucléiques ayant au moins une mutation dans la séquence codant pour le polypeptide mature de SEQ ID NO:2, dans laquelle la séquence d'acides nucléiques mutante code pour un polypeptide consistant en les acides aminés 32 à 923 de SEQ ID NO:1.

8. Séquence d'acides nucléiques isolée selon la revendication 6 produite par (a) hybridation d'un ADN dans des conditions de forte stringence (i) aux nucléotides 94 à 2769 de SEQ ID NO:2, (ii) à la séquence d'ADNc comprise entre les nucléotides 94 et 2769 de SEQ ID NO:2 ou (iii) à un brin complémentaire de (i) ou de (ii) ; et (b) isolation de la séquence d'acides nucléiques.

9. Séquence d'acides nucléiques selon la revendication 6, dans laquelle ladite séquence consiste en les nucléotides 94 à 2769 de SEQ ID NO:2.

10. Construction d'acides nucléiques comprenant une séquence d'acides nucléiques selon la revendication 6 liée de manière utilisable à une ou plusieurs séquences contrôles qui dirigent la production du polypeptide dans un hôte d'expression adéquat.

11. Vecteur d'expression recombinant comprenant la construction d'acides nucléiques selon la revendication 10.

12. Cellule hôte recombinante comprenant la construction d'acides nucléiques selon la revendication 10.

13. Méthode de production d'une séquence d'acides nucléiques mutante, comprenant (a) l'introduction d'au moins une mutation dans la séquence codant pour le polypeptide mature de SEQ ID NO:2, dans laquelle la séquence d'acides nucléiques mutante code pour un polypeptide consistant en les acides aminés 32 à 923 de SEQ ID NO:1 et (b) récupération de la séquence d'acides nucléiques mutante.

14. Méthode de production du polypeptide selon l'une quelconque des revendications 1-5 comprenant (a) la culture d'une souche dans des conditions adéquates pour la production du polypeptide pour produire un surnageant comprenant le polypeptide ; et (b) récupération du polypeptide.

15. Méthode selon la revendication 14, dans laquelle la souche comprend la séquence d'acides nucléiques mutante selon la revendication 7.

16. Méthode selon la revendication 14, dans laquelle la souche est une cellule hôte comprenant la construction d'acides nucléiques selon la revendication 10.

17. Méthode selon la revendication 16, dans laquelle la séquence d'acides nucléiques est une séquence d'acides nucléiques mutante selon la revendication 7.

18. Composition comprenant la 2,6-β-D-fructosane hydrolase obtenue par la méthode selon la revendication 14.

19. Procédé non-thérapeutique de désintégration d'un biofilm microbien comprenant la mise en contact dudit biofilm avec une endo-2,6-β-D-fructosane hydrolase selon les revendications 1-5 dans un milieu aqueux.

20. Procédé selon la revendication 19, dans lequel ledit biofilm est présent sur une surface dure.

21. Procédé selon la revendication 20, dans lequel la surface dure est essentiellement non-perméable aux microorganismes.

22. Procédé selon la revendication 21, dans lequel la surface dure est choisie parmi le groupe constitué par le métal, les polymères synthétiques, le caoutchouc, le carton, le verre, le bois, le papier, la matière textile, le béton, la pierre, le marbre, le gypse, la céramique, la peinture, l'émail.

23. Procédé selon la revendication 22, dans lequel la surface dure est un membre d'un système de maintien, de transport, de traitement ou est en contact avec des solutions aqueuses.

24. Procédé selon la revendication 23, dans lequel la surface dure est un membre d'un système choisi parmi le groupe constitué par les systèmes d'approvisionnement en eau, les systèmes de transformation des aliments, les systèmes de refroidissement, les systèmes de traitement chimique, les systèmes de traitement pharmaceutique et les systèmes de traitement du bois.

25. Procédé selon la revendication 24, dans lequel la surface dure est un membre d'une unité de système choisie parmi le groupe constitué par les tuyaux, les cuves, les pompes, les membranes, les filtres, les échangeurs thermiques, les centrifugeuses, les évaporateurs, les mélangeurs, les tours d'atomisation, les valves et les réacteurs.

26. Procédé selon la revendication 22, dans lequel la surface dure est un membre d'un accessoire choisi parmi le groupe constitué par les endoscopes, les dispositifs prothétiques et les implants médicaux.

27. Procédé selon les revendications 19-25, dans lequel le procédé est un procédé de Nettoyage En Place (NEP).

28. Polypeptide selon la revendication 1 pour la désintégration d'un biofilm microbien sur une surface dure d'origine biologique.

29. Polypeptide selon la revendication 28, dans lequel la surface dure est choisie parmi le groupe constitué par les muqueuses, la peau, les dents, les cheveux et les ongles.

30. Polypeptide selon la revendication 29, dans lequel la surface dure est la surface d'une dent et le biofilm est la plaque dentaire.

31. Polypeptide selon la revendication 29, dans lequel la surface dure est la surface d'une muqueuse bucco-dentaire et le biofilm est présent dans un poumon humain.

32. Procédé pour réduire la bio-corrosion d'une surface dure sur laquelle un biofilm est présent comprenant l'application du procédé selon la revendication 19.

33. Procédé non thérapeutique d'hydrolyse des boues comprenant la mise en contact des boues avec une 2,6-β-D-fructosane hydrolase selon l'une quelconque des revendications 1-5 dans un milieu aqueux.

34. Procédé selon l'une quelconque des revendications 19-27, 32 et 33, dans lequel le milieu aqueux comprend en outre un ou plusieurs composants choisis parmi le groupe constitué par les enzymes hydrolases et les agents antimicrobiens.

35. Procédé selon la revendication 34, dans lequel l'enzyme hydrolase est choisie parmi le groupe constitué par les cellulases, les hémicellulases, les xylanases, les amylases, les lipases, les protéases et les pectinases.

36. Procédé selon la revendication 34, dans lequel l'agent antimicrobien est choisi parmi le groupe constitué par des biocides enzymatiques et non-enzymatiques.

37. Procédé selon la revendication 36, dans lequel le biocide enzymatique est une oxydoréductase choisie parmi le groupe constitué par les laccases et les peroxydases.

38. Procédé de production d'oligomères 2,6-β-D-fructose comprenant la mise en contact d'un substrat choisi parmi le groupe constitué par le lévane et la phléine avec une 2,6-β-D-fructosane hydrolase selon l'une quelconque des revendications 1-5.

39. Procédé de production du fructose comprenant :
a) mise en contact d'un substrat choisi parmi le groupe constitué par la phléine et le lévane avec une 2,6-β-D-fructosane hydrolase selon l'une quelconque des revendications 1-5 moyennant quoi des 2,6-β-D-fructose oligo saccharides sont générés, et
b) mise en contact des 2,6-β-D-fructose oligosaccharides avec une 2,6-β-D-fructosane exo-hydrolase.

40. Composition selon la revendication 18, dans laquelle la composition est une composition de soin buccal comprenant une quantité de 2,6-β-D-fructosane hydrolase comprise dans la plage allant de 0,001 LRU à 1000 LRU/ml.

41. Composition de soin buccal selon la revendication 40, dans laquelle la composition est sous une forme choisie parmi le groupe constitué par la poudre, la pâte, le gel, le liquide, la pommade et le comprimé.

42. Composition de soin buccal selon la revendication 41, dans laquelle la composition est choisie parmi le groupe constitué par la pâte dentifrice, la crème dentaire, le gel, la poudre dentaire, le bain de bouche orthodontique, le bain de bouche avant brossage, le bain de bouche après brossage, le chewing-gum, la pastille, et le bonbon.

43. Composition de soin buccal selon la revendication 42, comprenant en outre une ou plusieurs enzymes issues du groupe constituée par la dextranase, les mutanases, les oxydases, les peroxydases, les laccases, les protéases, les endoglucosidases, les lipases et les amylases.

44. Composition de soin buccal selon la revendication 43, comprenant en outre un ou plusieurs composants choisis parmi le groupe constitué par les abrasifs, les humidifiants, les épaississants, les liants, les agents moussants, les savons, les agents tensioactifs, les édulcorants, les arômes, les agents blanchissants/décolorants, les agents anti-bactériens, les sources fluorure, les teintes/colorants, les conservateurs, les vitamines, les agents ajusteurs de pH, les agents anti-caries et les agents désensibilisants.

45. Composition selon la revendication 18, dans laquelle la composition est un additif détergent comprenant une 2,6-β-D-fructosane hydrolase selon l'une quelconque des revendications 1-5.

46. Additif détergent selon la revendication 45 comprenant en outre une enzyme choisie parmi le groupe constitué par une protéase, une lipase, une cutinase, une amylase, une carbohydrase, une cellulase, une pectinase, une mannanase, une arabinase, une galactanase, une xylanase, une oxydase, une laccase, et une peroxydase.

47. Composition selon la revendication 18, dans laquelle la composition est une composition détergente comprenant un tensioactif.

48. Composition détergente selon la revendication 47, comprenant en outre une enzyme choisie parmi le groupe constitué par une protéase, une lipase, une cutinase, une amylase, une carbohydrase, une cellulase, une pectinase, une mannanase, une arabinase, une galactanase, une xylanase, une oxydase, une laccase, et une peroxydase.

49. Procédé de préparation d'un médicament comprenant une 2,6-β-D-fructosane hydrolase selon les revendications 1-5 pour la désintégration de la plaque présente sur les dents humaines ou animales.

50. Procédé de préparation d'un médicament comprenant une 2,6-β-D-fructosane hydrolase selon les revendications 1-5 pour la désintégration d'un biofilm présent sur une muqueuse humaine ou animale.


### SEQUENCE LISTING

<110> NOVO NORDISK A/S
<120> A 2,6-β-D-FRUCTAN HYDROLASE ENZYME AND PROCESSES FOR USING THE ENZYME
<130> seqIDs
<140>
   <141>
<160> 6
<170> PatentIn Ver. 2.1
<210> 1
   <211> 923
   <212> PRT
   <213> Paenibacillus amylolyticus
<400> 1
   Met Lys Asp Lys Ser Lys Phe Ala Ile Cys Leu Leu Met Ala Thr Gly
   1               5                   10                  15
   Leu Thr Leu Thr Ser Asn Gly Ile Phe Thr Ser Lys Ser Ile Ala Ala
               20                  25                  30
   Ser Ala Val Leu Ala Asp Gln Glu Thr Asn Lys Gln Ala Lys Glu Glu
           35                  40                  45
   Leu Asp Ile Phe Glu Ser Ala Ser Lys Ser Asn Thr Asn Leu Ile Gly
       50                  55                  60
   Trp Gln Val Lys Gly Lys Gly Gly Leu Glu Asp Thr Ser Glu Gly Ile
   65                  70                  75                  80
   Leu Leu Thr Ser Gln Pro Lys Glu Asn Val Met Ala Ile Ser Glu Thr
                   85                  90                  95
   Val Ser Asp Asp Phe Ile Tyr Glu Ala Asp Val Met Ile Arg Asp Thr
               100                 105                 110
   Lys Ala Asp Ala Thr Leu Leu Phe Arg Ser Asn Glu Asp Gly Phe Ser
           115                 120                 125
   Ser Tyr Met Leu Gln Ile Val Pro Asp Ala Gly Leu Ile Arg Leu Arg
       130                 135                 140
   Asp Ala Arg Thr Gly Asp Gly Lys Leu Lys Glu Glu Arg Lys Val Ser
   145                 150                 155                 160
   Val Glu Met Gly Gln Ile Tyr His Leu Lys Val Lys Ala Val Gly Ser
                   165                 170                 175
   Ser Leu Lys Val Tyr Trp Gly Asn Gln Tyr Lys Pro Leu Ile Asp Val
               180                 185                 190
   Gln Asp Ile Ser Tyr Gln Ser Gly Lys Leu Gly Leu His Val Trp Asp
           195                 200                 205
   Gly Ser Ala Leu Phe Ser Asn Ile Val Val Ser Asp Leu Lys Gly Asn
   210 215 220
   Leu Gly Thr Val Leu Ser Ser Lys Gly Lys Trp Gln Pro Asp Ile Asn
   225 230 235 240
   Gly Lys Arg Gly Thr Val Glu Gln Gly Ser Ile Ala Gln Gln Ile Tyr
   245 250 255
   Asn Lys Glu Ala Thr Asp Met Val Tyr Glu Gly Asn Ile Thr Leu Arg
   260 265 270
   Pro Asp Ser Ile Ala Ala Leu Ala Phe Arg Ser Ser Thr Asp Gly Ala
   275 280 285
   Glu Gly Tyr Glu Ala Thr Leu Thr Lys Glu Gly Asp Gln Val Arg Val
   290 295 300
   Ser Leu Thr Asn Thr Lys Gly Thr Val Ile Ala Ser Ser Gln Arg Thr
   305 310 315 320
   Tyr Pro Ser Gln Met Gly Ala Lys His His Val Glu Ile Lys Ala Lys
   325 330 335
   Gly Asp Arg Ile Gln Val Phe Leu Asp Gly Tyr Thr Thr Ala Ala Ile
   340 345 350
   Asp Val Lys Asp Ser Thr Tyr Lys Ser Gly Ser Thr Gly Ile Val Val
   355 360 365
   Lys Lys Gly Thr Ala Tyr Phe Gln Asp Thr Tyr Val Thr Glu Leu Ser
   370 375 380
   Gln Tyr Tyr Asn Glu Ile Tyr Arg Pro Gln Tyr His Tyr Thr Pro Ile
   385 390 395 400
   Arg Gly Ser Ala Ser Asp Pro Asn Gly Leu Val Tyr Phe Glu Gly Glu
   405 410 415
   Tyr His Leu Phe His Gln Asp Gly Gly Thr Trp Ala His Ala Val Ser
   420 425 430
   Lys Asp Met Leu Asn Trp Lys Arg Leu Pro Ile Ala Leu Pro Trp Asn
   435 440 445
   Asp His Gly His Val Trp Ser Gly Ser Ala Val Ala Asp Met Thr Asn
   450 455 460
   Ala Ser Gly Leu Phe Gly Asp Ser Gly Gly Lys Gly Leu Ile Ala Tyr
   465 470 475 480
   Tyr Thr Ser Phe Asn Pro Asp Ser Pro Asn Gly Asn Gln Arg Ile Gly
   485 490 495
   Leu Ala Tyr Ser Lys Asp Gln Gly Arg Thr Trp Glu Tyr Ser Lys Glu
   500 505 510
   Arg Pro Ile Val Ile Glu Asn Pro Gly Lys Ser Gly Asn Glu Ala Gly
   515 520 525
   Asn Trp Asp Phe Arg Asp Pro Lys Val Ile Arg Asp Asp Glu Asn Asn
       530                 535                 540
   Arg Trp Val Met Val Val Ser Gly Gly Asp His Ile Arg Phe Tyr Thr
   545                 550                 555                 560
   Ser Thr Asn Leu Leu Asp Trp Thr Leu Thr Asp Asn Trp Gly Tyr Gly
                   565                 570                 575
   Asp Tyr Val Arg Gly Gly Val Trp Glu Cys Pro Asp Leu Phe Gln Leu
               580                 585                 590
   Pro Val Asp Gly Thr Ser Gln Lys Lys Trp Val Met Met Ile Ser Thr
           595                 600                 605
   Gly Ala Asn Pro Lys Thr Gly Gly Ser Asp Ala Glu Tyr Phe Ile Gly
       610                 615                 620
   His Leu Thr Ala Asp Gly Lys Phe Val Asn Asp Asn Pro Ala Gly Lys
   625                 630                 635                 640
   Val Leu Arg Thr Asp Phe Gly Lys Glu Phe Tyr Ala Ser Met Ser Phe
                   645                 650                 655
   Ala Asn Met Pro Asp His Arg Thr Val Met Met Ala Trp Met Thr Asn
               660                 665                 670
   Trp Asp Tyr Pro Phe Ala Phe Pro Thr Ser Asn Trp Lys Gly Glu Leu
           675                 680                 685
   Thr Ile Pro Arg Glu Val Ser Leu Val Thr Thr Glu Asp Gly Ile Arg
       690                 695                 700
   Met Val Gln Ser Pro Ile Lys Glu Leu Glu Ser Leu Arg Lys Pro Leu
   705                 710                 715                 720
   Tyr Ser Ala Ser Asn Lys Ser Val Ser Pro Ser Ser Gly Asn Leu Leu
                   725                 730                 735
   Lys Gly Ile Ile Ser Gly Ala Tyr Glu Ile Glu Ala Glu Ile Glu Ile
               740                 745                 750
   Pro Glu Thr Ser Thr Val Thr Glu Phe Gly Phe Asn Ile Arg Glu Gly
           755                 760                 765
   Ala Asn Gln Lys Thr Val Val Gly Tyr Lys Ala Ser Asp Ser Arg Met
       770                 775                 780
   Phe Val Asp Arg Thr Ala Ser Gly Glu Thr Asp Phe Ser Asn Leu Phe
   785                 790                 795                 800
   Ser Lys Lys His Glu Ala Pro Thr Gln Met Glu Asn Asn Arg Ile Lys
                   805                 810                 815
   Met Arg Ile Leu Val Asp Glu Ser Ser Val Glu Ala Phe Gly Asn Asp
               820                 825                 830
   Gly Lys Val Val Phe Ser Asp Val Ile Phe Pro Asp Pro Ala Ser Arg
           835                 840                 845
   Ala Met Ser Phe Tyr Val Lys Gly Gly Asn Val Asn Val Val Ser Leu
       850                 855                 860
   Lys Val His Gln Leu Gln Ser Val Trp Asn Ala Asp Ile Pro Ser Lys
   865                 870                 875                 880
   Val Gln Ile Lys Met Asp Thr Ser Val Arg Glu Leu Gly Val Gly Glu
                   885                 890                 895
   Ser Asp Thr Leu Gln Ala Met Val Glu Tyr Gly Pro Gly Leu Gly Val
               900                 905                 910
   Gln Pro Leu Lys Trp Asn Pro Val Ile Thr Thr
           915                 920
<210> 2
   <211> 2769
   <212> DNA
   <213> Paenibacillus amylolyticus
<400> 2
   atgaaagata aatctaaatt cgcaatatgt cttctgatgg caacgggatt aactctgact 60
   tcaaacggta tttttacatc taaatcaatt gcggctagtg ctgtacttgc tgatcaggaa 120
   acaaataaac aggctaagga ggagcttgat atttttgaaa gcgcatccaa gtcaaatacc 180
   aatctgatag gctggcaagt gaaaggaaaa ggaggattag aggatacttc agaaggaatc 240
   ttgctaactt cacagcctaa agaaaatgta atggccatct cagagacagt gtccgatgat 300
   tttatctatg aagctgatgt catgatcagg gatacgaagg cagacgcaac attgttgttt 360
   cgttctaatg aggatggctt tagctcgtac atgctgcaaa tcgttccgga cgcaggtctg 420
   attcggttaa gagatgcaag aactggagat gggaaattaa aggaggagcg taaagtttct 480
   gttgaaatgg ggcaaatcta tcatctcaaa gtgaaggcag tgggttcctc gctaaaagta 540
   tattggggca atcaatataa accattaatt gatgttcaag acatttcgta tcaaagcgga 600
   aagcttggac tccatgtatg ggatggatcc gccttgtttt cgaacatcgt ggtgagcgat 660
   ctgaaaggta atttgggtac ggtgctttct agcaagggaa aatggcagcc tgatatcaac 720
   ggcaaaagag ggacggtaga acaggggagc atagcrcagc aaatctataa caaagaggcg 780
   actgatatgg tctatgaagg gaatattacc cttcgtcctg attctattgc agctctcgca 840
   tttcgatctt caaccgatgg agctgaagga tatgaagcta ctcttacgaa ggaaggagat 900
   caggtccgtg taagtttgac gaatacaaaa ggaacagtaa ttgcaagttc gcaacgtact 960
   tatccgagtc agatgggagc caaacatcat gtggaaatca aggcaaaggg agatcgaatt 1020
   caggtcttcc tggacgggta cactacggcc gcgattgacg tgaaagattc aacctacaag 1080
   agtggaagta ccggaatcgt cgtaaaaaag gggacggctt actttcagga tacttatgtg 1140
   acggaactga gccaatatta caatgagata tatcgtccac aatatcacta cacgccaata 1200
   cgtggttcag cgagtgatcc gaatggactt gtctacttcg aaggagaata tcatctcttc 1260
   catcaggatg gaggaacatg ggcgcatgcg gtaagtaaag atatgctgaa ctggaaacgg 1320
   cttccgattg cacttccctg gaatgatcac ggacatgtct ggtctgggtc ggctgttgcg 1380
   gatatgacaa atgcatctgg tttattcgga gattcaggag gcaagggcct tattgcatac 1440
   tatacttcct ttaatccgga tagcccgaat gggaaccagc gtataggtct agcttacagt 1500
   aaagaccaag gtcgtacttg ggagtattcg aaggagcgcc cgattgtgat cgagaacccg 1560
   ggtaagagcg gaaatgaagc tgggaattgg gatttccgcg atccgaaagt aatccgtgat 1620
   gatgagaata accgatgggt tatggttgtg tccggaggag atcatattcg tttctatact 1680
   tcaacaaatt tacttgactg gacattgaca gataattggg gatatgggga ttacgtccgc 1740
   ggaggagtat gggaatgccc tgatttgttc cagcttccgg tagacggaac gtcacagaaa 1800
   aagtgggtta tgatgatcag tacaggagcg aatcccaaaa caggcggatc agatgccgag 1860
   tattttatcg gtcatttaac agctgatggt aaattcgtaa acgataatcc ggcaggtaag 1920
   gtgttaagaa cagattttgg taaagaattt tacgcttcga tgtcttttgc taacatgcct 1980
   gatcatcgca cagtgatgat ggcgtggatg acgaattggg attatccgtt cgctttccca 2040
   acgtccaatt ggaaaggtga actaaccatt ccgagagaag tatcattggt aacgaccgaa 2100
   gatggaattc ggatggtgca aagtccaatc aaagaattgg aatcactgcg taaacctttg 2160
   tattctgctt ctaacaagtc ggtgagccct tcttccggga atctgctaaa aggtattatt 2220
   tcaggtgctt acgaaattga agctgaaatt gaaatccctg aaaccagcac agtgaccgag 2280
   tttggcttta acattcgtga gggtgcaaat cagaagacgg tcgtgggtta taaggcaagc 2340
   gatagtcgta tgtttgtgga ccgaactgca tccggtgaaa cggatttttc taaccttttc 2400
   agtaagaaac atgaagcgcc tacgcaaatg gagaataatc ggatcaagat gcgtattctt 2460
   gtggatgagt cttctgttga agcctttggt aacgacggca aagtcgtctt ttcagatgtt 2520
   atatttccgg atcctgctag tagagcgatg agtttttacg taaaaggcgg gaatgtgaat 2580
   gttgtttcct tgaaagtgca tcaacttcaa tctgtctgga atgcggacat tccttcaaaa 2640
   gttcaaataa agatggatac gagcgttcgg gaactggggg taggcgagtc ggatacactg 2700
   caggcgatgg tcgagtatgg cccgggtcta ggcgttcaac cgcttaagtg gaatccagta 2760
   ataacgacg                                                         2769
<210> 3
   <211> 1277
   <212> PRT
   <213> Paenibacillus pabuli
<400> 3
   Met Lys Phe Ile Phe Arg Ser Ile Gly Cys Val Leu Leu Ser Cys Val
   1 5 10 15
   Leu Gly Ser Ser Ala Ala Gly Ala Tyr Ser Thr Ala Ser Val Met Glu
   20 25 30
   Thr Asn Glu Ser Gln Arg Met Glu Gln Thr Ser Lys Glu Ser Ala Lys
   35 40 45
   Gln Gly Glu Ala Val Arg Met Leu Thr Lys Val Ser Lys Ala Val Thr
   50 55 60
   Asn Leu Ser Asp Trp Thr Leu Gln Gly Arg Gly Ser Leu Glu Asp Thr
   65 70 75 80
   Asp Glu Gly Leu His Leu Ala Ser Glu Ala Arg Glu Asn Val Met Ala
   85 90 95
   Leu Ser Ala Thr Arg Ala Asp Asn Phe Ile Tyr Glu Ala Asp Leu Met
   100 105 110
   Ile Gln Asp Met Lys Ala Asp Thr Ser Leu Val Phe Arg Ser Asn Asp
   115 120 125
   Thr Gly Trp Ser Ser Tyr Met Leu Gln Val Val Pro Gln Ala Gly Val
   130 135 140
   Ile Arg Leu Arg Asp Ala Ser Gly Gln Pro Gly Thr Leu Asn Val Glu
   145 150 155 160
   His Lys Ala Asn Leu Glu Ser Gly Gly Ile Tyr His Leu Lys Val Lys
   165 170 175
   Ala Asp Gly Glu Ser Leu Gln Val Tyr Trp Asp Asn Arg Tyr Asp Pro
   180 185 190
   Val Ile Asp Val Lys Asp Ser Ala Tyr Ser Ser Gly Arg Leu Gly Leu
   195 200 205
   His Val Trp Asp Gly Ser Ala Leu Phe Gln Asn Val Gln Val Ser Ile
   210 215 220
   Met Asn Gly Asn Ile Gly Lys Pro Ile Ser Ser Val Gly Glu Trp Gln
   225 230 235 240
   Pro Asp Leu Lys Gly Tyr Lys Gly Lys Gly Asn Ala Gln Gly Lys Gly
   245 250 255
   Gln Ile Val Tyr Glu Lys Ala Ala Ser Asp Phe Val Tyr Glu Gly Asn
   260 265 270
   Leu Tyr Leu Ala Asp Ala Ser Thr Ser Ala Ala Leu Leu Phe Arg Ala
   275 280 285
   Ser Thr Asp Gly Thr Lys Gly Tyr Glu Ala Ala Leu Ile Arg Glu Gly
   290 295 300
   Glu Glu Val Arg Val Gln Leu Arg Lys Ala Asp Gly Thr Val Leu Ala
   305 310 315 320
   Ser Ser Asn Arg Lys Val Pro Ser Gln Pro Gly Ala Arg His His Ile
   325 330 335
   Glu Val Ile Ala Ser Gly Ser Leu Ile Gln Val Tyr Val Asp Gly Tyr
   340 345 350
   Thr Pro Ala Ala Val Glu Val Thr Asp Lys Ser Tyr Ala Lys Gly Asn
   355 360 365
   Ala Gly Leu Val Val Gln Gln Gly Met Ala Tyr Phe Gln Asp Ile Tyr
   370 375 380
   Met Thr Glu Glu Ser Met Tyr Tyr Lys Glu Asn Tyr Arg Pro Gln Tyr
   385 390 395 400
   His Tyr Ser Pro Leu Arg Gly Ser Ala Ser Asp Pro Asn Gly Leu Val
   405 410 415
   Tyr Tyr Glu Gly Glu Tyr His Leu Phe His Gln Asp Gly Gly Thr Trp
   420 425 430
   Ala His Ala Val Ser Ser Asp Leu Ile Asn Trp Lys Arg Leu Pro Ile
   435 440 445
   Ala Leu Pro Trp Asn Asp Gln Gly His Val Trp Ser Gly Ser Ala Ile
   450 455 460
   Ala Asp Leu Asn Asn Ala Ser Gly Leu Phe Thr Asp Ser Gly Gly Lys
   465 470 475 480
   Gly Leu Ile Ala Tyr Tyr Thr Ser Tyr His Pro Asp Lys Pro Gly Gly
   485 490 495
   Asn Gln Arg Ile Gly Leu Ala Tyr Ser Thr Asp Gln Gly Arg Asn Trp
   500 505 510
   Gln Tyr Ala Lys Glu Arg Pro Ile Val Ile Asp Asn Pro Gly Lys Asn
   515 520 525
   Gly Asp Asp Pro Gly Ser Trp Asp Phe Arg Asp Pro Lys Val Val Arg
   530 535 540
   Asp Glu Asp His Asn Arg Trp Val Met Val Val Ser Gly Gly Asp His
   545 550 555 560
   Ile Arg Phe Phe Thr Ser Thr Asn Leu Leu Asp Trp Thr Leu Thr Asp
   565 570 575
   Asn Phe Gly Tyr Gly Asp Tyr Val Arg Gly Gly Val Trp Glu Cys Pro
   580 585 590
   Asp Leu Ile Gln Leu Pro Val Asp Gly Thr Gly Gln Arg Lys Trp Val
   595 600 605
   Leu Leu Ile Ser Thr Gly Ala Asn Pro Lys Thr Gln Gly Ser Asp Ala
   610 615 620
   Glu Tyr Phe Val Gly Gln Leu Thr Ala Asp Gly Lys Phe Leu Asn Asp
   625 630 635 640
   His Pro Ala Gly Gln Val Leu Arg Thr Asp Tyr Gly Lys Glu Phe Tyr
   645 650 655
   Ala Ser Met Ser Phe Ala Asn Met Pro Asn Gln Arg Lys Val Met Leu
   660 665 670
   Ala Trp Met Thr Asn Trp Asp Tyr Pro Phe Glu Phe Pro Thr Ser Ser
   675 680 685
   Trp Lys Gly Gln Leu Thr Ile Pro Arg Glu Val Ser Leu Arg Thr Thr
   690 695 700
   Asp Glu Gly Val Arg Leu Val Gln Thr Pro Ile Thr Glu Leu Gln Lys
   705 710 715 720
   Leu Arg His Asn Leu Tyr Ser Ala Gln Gln Met Thr Val Gly Pro Lys
   725 730 735
   Ser Lys Asn Pro Leu Glu Gly Leu Thr Ala Gly Ala Tyr Glu Ile Glu
   740 745 750
   Ala Glu Val Glu Ile Pro Ala Asn Ser Ser Val Thr Glu Phe Gly Phe
   755 760 765
   Gln Leu Arg Gln Arg Glu Gly Gln Lys Thr Thr Val Ala Tyr Arg Val
   770 775 780
   Asp Thr Gln Asn Met Phe Val Asp Arg Thr Thr Ser Gly Asp Val Ser
   785 790 795 800
   Phe Ser Asp Leu Phe Thr Lys Val His Glu Ala Ser Leu Lys Pro Glu
   805 810 815
   Asn Gln Lys Val Lys Leu Arg Ile Phe Val Asp Glu Ser Ser Val Glu
   820 825 830
   Val Phe Gly Asn Asp Gly Lys Val Val Phe Ser Asp Val Ile Phe Pro
   835 840 845
   Asp Pro Ala Gly Arg Ala Met Ala Phe Tyr Ser Leu Gly Gly Glu Val
   850 855 860
   Lys Val Ser Ser Met Lys Val Tyr Ala Leu Asp Asn Ile Trp Arg Lys
   865 870 875 880
   Ser Thr Asp Ser Lys Met Gln Val Phe Ala Asp Met Gln Arg Arg Ile
   885 890 895
   Val Asn Met Gly Gln Thr Gln Thr Leu Tyr Ala Thr Val Glu Gly Arg
   900 905 910
   Ser Gly Lys Gly Ala Ala Gln Pro Val Lys Trp Lys Val Ser Asn Pro
   915 920 925
   Lys Val Ala Gln Ile Val His Ser Asp Lys Thr Lys Ala Thr Val Arg
   930 935 940
   Ala Val Gly Gly Gly Glu Ala Val Val Thr Val Ser Thr Thr Asp Gly
   945 950 955 960
   Lys Ala Ser Ala Gln Ile Pro Met Lys Val Ser Ser Gly Val Phe His
   965 970 975
   Thr Asn Leu Ser Gly Trp Lys Ala Asp Lys Ser Ser Ala Gln Trp Leu
   980 985 990
   Val Ser Glu Gln Gly Ile Gln Gly Gly Tyr Ser Gly Asp Thr Gln Tyr
   995 1000 1005
   Ile Ala Gln Asp Thr Thr Ala Gly Asp Phe Gln Tyr Asp Ala Asp Leu
   1010 1015 1020
   Thr Leu Gly Ala Gln Gly Gly Ala Gly Ser Ile Val Phe Arg Ala Ser
   1025 1030 1035 1040
   Glu Asp Gly Arg Ser Gly Tyr Tyr Leu Asn Ile Asp Pro Asn Leu Lys
   1045 1050 1055
   Ser Ile Arg Leu Phe Tyr Lys Val Asn Gly Lys Phe Glu Asn Arg Gln
   1060 1065 1070
   Val Leu Ala Gln Ile Pro Arg Phe Ile Arg Lys Asp His Met Tyr His
   1075 1080 1085
   Ile Gln Ile Gln Ala Asn Gly Pro Arg Ile Gln Val Gly Val Asp Gly
   1090 1095 1100
   Glu Arg Ile Ile Asp Leu Gln Asp Gly Thr Phe Ala Glu Gly His Phe
   1105 1110 1115 1120
   Gly Ile His Val Phe Gly Gly Ser Ala Ser Phe Gln Asn Val Asn Ala
   1125 1130 1135
   Ile His Thr Lys Lys Ala Glu Leu Asp Gln Ile Ile Ile Arg Asn Ala
   1140 1145 1150
   Gly Ser Pro Val Ala Met Gln Ala Val Pro Ser Glu Ala Gly Glu Met
   1155 1160 1165
   Ile Lys Val Ala Asp Glu Ala Glu Asn Ala Ser Glu Gly Phe Asn Trp
   1170 1175 1180
   Val Leu Val Pro Thr Gly Asp Asp Ser Gly Ser Tyr Ser Ile Arg Thr
   1185 1190 1195 1200
   Leu Ser Gly Met Ser Leu Asp Trp Asp Val Gly Gln Asn Arg Ile Gln
   1205 1210 1215
   Leu Tyr Ser Tyr Leu Gly Tyr Ala Asn Gln Arg Trp Asn Leu Ser Lys
   1220 1225 1230
   Asn Glu Asp Gly Thr Met Arg Ile Thr Ser Ala His Asn Gly His Ala
   1235 1240 1245
   Leu Gly Ile Ser Ala Ser Gly Pro Glu Leu Val Met Asp Glu Phe Arg
   1250 1255 1260
   Gln Glu Asn Glu Tyr Gln Lys Trp Val Ile Lys Gln Gln
   1265 1270 1275
<210> 4
   <211> 3834
   <212> DNA
   <213> Paenibacillus pabuli
<400> 4
   atgaaattca ttttccgatc catcggttgt gttctattat catgtgtcct agggtcttct 60
   gctgctggag cctattccac agcatctgtt atggagacaa atgaatcgca aaggatggag 120
   caaacctcca aggaatcagc aaaacaggga gaggcggttc gaatgttaac caaagtgtcg 180
   aaggcagtta cgaatttgtc ggattggact ttgcagggta gaggcagtct ggaagatacg 240
   gatgaaggac tccatctcgc ttcggaagcc agggagaatg tgatggccct atcagctaca 300
   cgagctgata attttatcta tgaagctgat ttgatgattc aggatatgaa agcggataca 360
   agtcttgttt ttcgatccaa tgatacaggc tggtcttcat atatgctgca agtggttccc 420
   caggctggtg tgatacgtct aagagatgcc agtggacagc cgggaacgtt aaatgtagaa 480
   cataaagcaa acctggaatc tggaggtatc tatcatctga aagtgaaagc ggacggagag 540
   agccttcagg tatattggga taatcgatac gacccggtga ttgatgtgaa agatagtgcc 600
   tactcgtcag ggaggctggg gctccatgtg tgggatgggt cggctttgtt ccagaatgtg 660
   caggtcagta tcatgaatgg caatatcggc aaaccgatta gcagcgtagg tgaatggcag 720
   ccggatttga aagggtataa agggaaagga aacgctcagg gaaaaggaca gatcgtttac 780
   gaaaaggcgg cttccgattt tgtgtatgaa gggaatctat atctggccga tgcgagtacc 840
   tcagcggcgt tgctgttcag agcgagcaca gacgggacga agggatatga agcagccctg 900
   atccgggaag gagaagaagt tcgggtacaa cttcgaaaag cggacggtac tgtgctcgca 960
   agctcgaatc gtaaggttcc aagccaaccg ggagcgaggc atcacatcga agttattgcc 1020
   tcgggcagct tgattcaggt ctatgtggat gggtacacgc ctgcggctgt tgaggtcaca 1080
   gataaaagct acgctaaagg aaatgctgga cttgtcgttc aacaaggcat ggcttatttt 1140
   caggatattt atatgacaga agagtcgatg tactacaaag agaactaccg tcctcagtac 1200
   cattattctc cgcttcgggg atcggcaagt gatccgaacg ggctggtcta ttatgaagga 1260
   gaatatcatc tgtttcatca ggatggaggc acctgggctc atgcagtcag ttcggatctt 1320
   ataaattgga agcgtttacc tattgctttg ccctggaatg atcagggtca tgtatggtca 1380
   ggatcagcaa ttgcagatct gaataatgcc tctggtctgt tcacggattc gggcggaaaa 1440
   ggtctgattg cgtactacac ttcctatcat ccggataaac ctggtggaaa tcagcgcatc 1500
   ggccttgcct atagtaccga tcaaggccgg aattggcagt atgctaaaga acgtccgatt 1560
   gtcattgata accctggcaa gaacggtgac gacccgggaa gctgggattt tcgtgatccg 1620
   aaggtcgtcc gcgatgaaga ccataaccgc tgggtgatgg tggtgtccgg cggggatcac 1680
   atccggttct ttacttccac caatctgctc gattggacct taaccgataa cttcggatat 1740
   ggcgattacg ttcgtggcgg ggtgtgggag tgtcccgatc tgattcaact gcctgtagat 1800
   ggaacgggtc aacgtaagtg ggtgcttctg atcagtacag gggccaatcc gaaaactcag 1860
   ggatctgatg cggaatattt tgtaggtcag ctaactgctg atgggaaatt cctgaacgat 1920
   catccggctg ggcaggtgtt gagaacggac tacggtaagg agttctacgc atcaatgtcc 1980
   tttgcgaata tgccgaacca gcggaaagtt atgcttgcgt ggatgacgaa ctgggattat 2040
   ccgtttgaat tcccaacctc ctcatggaaa ggacaactga ccataccgag agaagtctct 2100
   cttcgaacca cggatgaagg cgtgcgtttg gtgcaaacac caatcactga gcttcagaaa 2160
   ctgcggcata acctatacag tgcacaacaa atgacggttg gcccaaaatc aaaaaatccg 2220
   ctggagggcc taacggcggg agcgtatgag attgaagctg aagtggaaat tccggctaac 2280
   agttctgtaa ccgagtttgg ttttcaactg cgtcagagag aggggcagaa aaccactgta 2340
   gcatacagag tggatacgca gaacatgttc gtggatcgca ccacctcagg tgacgtaagc 2400
   ttctccgact tattcaccaa ggtccatgaa gcctcgttaa aacctgaaaa tcaaaaggtg 2460
   aaactgcgca tatttgtcga tgaatcctct gttgaggtct ttggcaatga tggcaaggtt 2520
   gttttctcgg atgtcatttt tcccgatccg gcgggcaggg cgatggcatt ctatagcctt 2580
   ggtggggaag tgaaggtcag ctccatgaag gtgtacgcct tggacaatat ctggagaaag 2640
   agcacggact ccaagatgca ggtttttgca gatatgcaaa gaagaatagt gaatatgggg 2700
   cagacgcaaa cgctgtacgc cacggtggaa ggcagatcag gtaaaggtgc cgctcaaccg 2760
   gttaaatgga aggtaagcaa tcctaaagtg gcgcaaatcg ttcattctga taaaacgaaa 2820
   gcaaccgttc gtgccgtggg tggaggcgaa gctgtagtaa ccgtatcgac gacagacggc 2880
   aaagcttctg cccaaatacc gatgaaggta tccagcgggg tattccacac caacttatct 2940
   ggctggaagg cagataaatc atctgcccaa tggcttgttt ccgaacaagg catacagggg 3000
   gggtatagtg gagatacaca gtatatcgca caggatacta cggcagggga ttttcaatat 3060
   gatgctgacc tgacgctggg agcacagggg ggagcggggt ctattgtatt ccgtgccagc 3120
   gaggatggac gaagcggcta ctacttgaat atcgatccta acctgaagtc cattcgactg 3180
   ttttataaag taaatggaaa attcgagaat cggcaggtgc tcgctcaaat ccctagattc 3240
   attcgtaagg atcatatgta tcacattcaa attcaggcga acggtcctcg aatccaggtt 3300
   ggtgtggacg gtgagcgaat aatagatctt caggatggga cgtttgctga aggacatttc 3360
   ggtatccatg tgtttggcgg gagtgcttcc ttccagaatg tgaatgcaat ccatacgaag 3420
   aaggcagaat tggaccagat cattattcgt aacgcaggaa gtcctgttgc aatgcaggct 3480
   gttccatctg aagcaggaga aatgataaag gttgctgacg aggcagagaa tgcaagtgaa 3540
   ggcttcaact gggtacttgt accgacaggg gatgattccg gctcttactc cattcggacc 3600
   ttgagcggca tgtctcttga ttgggatgta ggacagaatc gcattcagct ctattcgtat 3660
   cttggttatg cgaatcaacg ctggaacctt tcgaagaatg aggatggaac gatgcgcatc 3720
   acaagtgcac ataacgggca cgcacttggt atatctgcga gtgggccaga actggtaatg 3780
   gatgagtttc gtcaagaaaa cgagtatcaa aagtgggtta ttaagcaaca ataa       3834
<210> 5
   <211> 943
   <212> PRT
   <213> Paenibacillus macerans
<400> 5
   Met Gly Lys Gly Lys Ser Val Leu Ser Ile Leu Leu Ser Ser Ala Leu
    1               5                  10                  15
   Ile Phe Ala Gly Leu Asp Ala Gly Met Val Arg Ala Ala Gly Ala Glu
               20                  25                  30
   Thr Glu Thr Gly Lys Gly Ala Lys Ala Ile Phe Glu Ser Ala Thr Lys
           35                  40                  45
   Leu Glu Thr Asn Leu Thr Gly Trp Arg Ile Glu Gly Lys Gly Arg Met
       50                  55                  60
   Glu Thr Val Glu Gln Gly Leu Leu Leu Val Ser Glu Pro Arg Glu Asn
   65                  70                  75                  80
   Val Met Ala Leu Thr Glu Thr Arg Ala Ala Asp Phe Ile Tyr Glu Ala
                   85                  90                  95
   Asp Val Met Val Thr Glu Arg Gln Ala Asp Ala Thr Leu Val Phe Arg
               100                 105                 110
   Ser Asn Gln Asp Gly Trp Ser Ser Tyr Met Leu Gln Leu Val Pro Asn
   115 120 125
   Ala Gly Leu Ile Arg Leu Lys Asp Ala Ser Glu Asp Gly Lys Leu Arg
   130 135 140
   Glu Glu Arg Arg Val Asp Leu Ala Glu Gly Glu Ile Tyr His Leu Lys
   145 150 155 160
   Val Lys Ala Glu Gly Thr Arg Leu Gln Val Phe Trp Gly Asp Arg Tyr
   165 170 175
   Lys Pro Leu Ile Asp Val His Asp Gly Ala Tyr Ala Ser Gly Tyr Leu
   180 185 190
   Gly Leu Asn Val Trp Asp Gly Ser Ala Leu Phe Gln Asn Val Lys Val
   195 200 205
   Ser Glu Leu Asn Ser Asn Leu Gly Ala Ala Ile Ala Ser Phe Gly Val
   210 215 220
   Trp Gln Pro Asp Leu Arg Gly Glu Leu Gly Thr Ala Asp Gly Thr Gly
   225 230 235 240
   Arg Ala Leu Arg Leu Tyr Gln Gly Ala Glu Arg Asp Leu Val Leu Glu
   245 250 255
   Gly Asn Val Ser Phe Ala Ala Asp Gln Gly Glu Ala Gly Leu Arg Phe
   260 265 270
   Arg Ala Asn Gly Gln Gly Thr Asp Gly Tyr Glu Ala Ala Leu Arg Lys
   275 280 285
   Asp Gly Gly Gln Val Trp Ala Glu Leu Arg Lys Ala Asp Gly Arg Val
   290 295 300
   Ile Ala Ala Ser Glu Arg Ala Tyr Pro Ser Ala Ser Lys Ala Arg His
   305 310 315 320
   His Leu Glu Ile His Ala Leu Gly Ser Arg Ile Gln Val Tyr Val Asp
   325 330 335
   Gly Tyr Ala Glu Pro Ala Val Asp Ala Val Asp Asn Ser Tyr Ala Asp
   340 345 350
   Gly His Ala Gly Phe Ala Val Ser Gly Gly Ala Ala Tyr Phe Gln Asp
   355 360 365
   Val Tyr Leu Thr Ala Ala Asn Asp Tyr Tyr Thr Glu Lys Tyr Arg Pro
   370 375 380
   Asp Tyr His Tyr Ser Pro Ala Arg Gly Ser Ala Ser Asp Pro Asn Gly
   385 390 395 400
   Leu Val Tyr Tyr Glu Gly Glu Tyr His Leu Phe His Gln Asp Gly Gly
   405 410 415
   Thr Trp Ala His Ala Val Ser Thr Asp Leu Val His Trp Lys Arg Leu
   420 425 430
   Pro Ile Ala Leu Pro Trp Asn Asp Leu Gly His Val Trp Ser Gly Ser
   435 440 445
   Ala Val Ala Asp Leu His Asn Ala Ser Gly Leu Phe Ala Asp Ser Gly
   450 455 460
   Gly Lys Gly Leu Ile Ala Tyr Tyr Thr Ser Tyr Asn Pro Asp Arg Pro
   465 470 475 480
   Asn Gly Asn Gln Arg Ile Gly Leu Ala Tyr Ser Lys Asp Arg Gly Arg
   485 490 495
   Thr Trp Glu Tyr Ala Ala Glu Arg Pro Ile Val Ile Glu Asn Pro Gly
   500 505 510
   Lys Gln Gly Asp Asp Pro Gly Gly Trp Asp Phe Arg Asp Pro Lys Val
   515 520 525
   Val Arg Asp Glu Glu His Asn Arg Trp Val Met Val Val Ser Gly Gly
   530 535 540
   Asp His Ile Arg Phe Phe Thr Ser Thr Asn Leu Ile Asp Trp Thr Leu
   545 550 555 560
   Thr Asp Ser Phe Gly Tyr Gly Ala Tyr Val Arg Gly Gly Val Trp Glu
   565 570 575
   Cys Pro Asp Leu Phe Gln Leu Ala Val Asp Asp Thr Gly Glu Lys Lys
   580 585 590
   Trp Val Leu Met Ile Ser Thr Gly Ala Asn Pro Asn Thr Gln Gly Ser
   595 600 605
   Ala Ala Glu Tyr Phe Ile Gly Glu Leu Thr Pro Glu Gly Lys Phe Val
   610 615 620
   Asn Asp Asn Pro Ala Gly Lys Val Leu Ala Thr Asp Tyr Gly Lys Glu
   625 630 635 640
   Tyr Tyr Ala Ser Met Ser Phe Ala Gly Met Pro Asp Gly Arg Arg Val
   645 650 655
   Met Leu Ala Trp Met Thr Asn Trp Asp Tyr Pro Phe Ala Phe Pro Thr
   660 665 670
   Glu Gly Trp Lys Gly Val Leu Ser Ile Pro Arg Glu Leu Thr Leu Gln
   675 680 685
   Lys Thr Asp Lys Gly Ile Arg Leu Ala Gln Thr Pro Ile Arg Glu Leu
   690 695 700
   Glu Ser Leu Arg Gly Gln Leu Leu Phe Ala Ala Ser Asp Arg Arg Val
   705 710 715 720
   Gln Ala Asp Arg Glu Asn Leu Leu Lys Gly Val Ser Ser Gly Val Tyr
   725 730 735
   Glu Ile Glu Ala Glu Ile Glu Ile Pro Gln Ala Ser Asn Val Ser Glu
   740 745 750
   Phe Gly Phe Arg Leu Arg Glu Gly Ala Gly Lys Arg Thr Val Val Gly
   755 760 765
   Tyr Lys Thr Lys Glu Asn Glu Ile Tyr Val Asp Arg Ser Leu Ser Gly
   770 775 780
   Asp Thr Gly Phe Ser Glu Arg Phe Thr Thr Leu His Gln Ala Pro Leu
   785 790 795 800
   Gln Pro Asp Asn Arg Arg Val Lys Leu Arg Ile Phe Val Asp Asp Ser
   805 810 815
   Ser Leu Glu Val Phe Gly Gly Asp Gly Arg Val Val Phe Ser Glu Val
   820 825 830
   Ile Phe Pro Asp Pro Ala His Arg Glu Met Ser Leu Phe Thr Val Gly
   835 840 845
   Gly Glu Val Asn Val Val Ser Leu Lys Val His Ala Leu Thr Asn Val
   850 855 860
   Trp Asn Glu Ala Ala Glu Ser Gln Thr Ser Ile Val Met Asp Thr Ser
   865 870 875 880
   Pro Gln Gln Met Gly Leu Gly Asp Thr Arg Thr Leu Tyr Ala Ala Val
   885 890 895
   Asn Gly Gly Ala Lys Gly Gly Arg Ser Gly Gly Ser Asp Gly Asn Arg
   900 905 910
   Asn Gln Pro Ile Arg Arg Pro Ala Ala Lys Leu Ile Asp Cys Thr Met
   915 920 925
   Pro Lys Thr Ile Ile Arg Lys Asp Arg Glu Thr Phe Asn Ile Leu
   930 935 940
<210> 6
   <211> 2832
   <212> DNA
   <213> Paenibacillus macerans
<400> 6
   atgggcaaag gaaaatccgt attatccatc cttttgtcct ccgcgcttat ttttgccggg 60
   cttgatgctg ggatggtccg ggcggcaggc gcggagacgg aaacgggaaa gggggcaaag 120
   gccatttttg aaagcgcaac caaattggag acgaatttga ccggctggcg aatcgaggga 180
   aaaggccgaa tggagacggt ggagcagggg cttctgctcg tctcggagcc gagggagaac 240
   gtgatggccc tgacggaaac ccgggcggcc gattttatct atgaagccga cgtcatggta 300
   acggagaggc aggcggacgc cacgttggta ttccgctcca atcaggatgg gtggtcctcc 360
   tacatgcttc agctcgtgcc gaacgcggga ttgatccggc taaaggatgc aagcgaagac 420
   ggcaagctgc gggaagaacg gcgggtggac ttggccgaag gggaaatcta tcatctgaaa 480
   gtcaaagccg aaggaacccg gcttcaggta ttttgggggg accgttataa accgttgatc 540
   gacgtccatg acggggctta tgcttccggc tatttgggcc tgaacgtttg ggacggctcc 600
   gcgctgtttc agaacgtgaa ggtcagcgaa ctgaacagca atttaggcgc ggcgatcgcg 660
   agttttggag tctggcagcc ggacctgcgg ggggagctgg gaacggcgga cggtaccggg 720
   cgggcgctgc ggctttatca gggggctgag cgggatctcg tgctggaagg aaacgtgtct 780
   tttgccgcag accaggggga agccggtttg cggttcagag cgaacgggca ggggacggac 840
   ggctacgagg cggctttgcg aaaagacggc ggccaggttt gggcggagct gagaaaagcg 900
   gacggccgcg tcatcgcggc ctcggaacgt gcttacccgt cggcttccaa agcaaggcac 960
   catctggaaa tccatgcgct cgggagccgg attcaggtct atgtggacgg atacgccgaa 1020
   ccggccgtgg atgccgttga caatagctac gcggacggtc acgcgggatt tgcggtatcc 1080
   gggggagcgg catattttca ggacgtctat ttgactgcgg ccaatgatta ttatacggaa 1140
   aaataccgcc ccgactacca ttattcgccc gcccgcgggt cggcaagcga tccgaacgga 1200
   ctggtctatt acgaagggga ataccatctg tttcatcagg acggcggaac ttgggcgcac 1260
   gcggtcagca ccgatctcgt tcattggaag cgcttgccta tcgctttgcc ctggaacgat 1320
   ttgggccatg tctggtcggg ttcggccgtc gccgatctcc ataatgcctc cggcttgttc 1380
   gcggattccg ggggcaaagg cctgatcgcc tattatactt cctataatcc ggaccgcccg 1440
   aacggcaacc agcgcattgg gctggcgtac agcaaggatc gcggccgcac ctgggagtac 1500
   gccgcagagc gcccgatcgt gatcgaaaat ccgggcaagc agggggacga tccgggagga 1560
   tgggattttc gcgatcccaa ggtcgtgcgg gatgaagagc ataaccgctg ggttatggtt 1620
   gtgtccggcg gcgaccatat ccgctttttc acttcgacca acctgatcga ctggacgctg 1680
   accgacagct tcggatacgg ggcttatgtc cgcggcggcg tgtgggagtg ccccgattta 1740
   ttccaactgg ccgtggacga cacgggcgag aaaaaatggg tgctcatgat cagcacgggg 1800
   gccaacccga atacgcaggg ttcggccgcg gagtatttta ttggggagct tactccggaa 1860
   ggtaagttcg ttaacgacaa ccctgccggc aaggtgctgg cgaccgatta cggaaaagag 1920
   tactacgcct ctatgtcttt tgccggtatg ccggacggac gcagggtgat gctggcctgg 1980
   atgacgaact gggattaccc gtttgctttt ccgaccgagg gttggaaggg ggttctaagc 2040
   attccgcgcg agttaacgct gcagaagacg gacaagggaa tccgcctggc acagacgccg 2100
   atccgcgagc tggaatcgct gcgcggccag ctgctgtttg cggcgtcgga ccgccgcgtc 2160
   caagcggata gggagaattt actgaaaggc gtgtcctcag gagtctatga aattgaagcc 2220
   gaaattgaaa ttcctcaggc cagcaacgtg agcgaattcg gtttccgcct ccgcgaggga 2280
   gccggaaagc ggacggtcgt cgggtacaaa acaaaagaga acgagatcta cgtggatcgc 2340
   tccctttccg gcgacaccgg cttctccgag cggttcacca cgcttcatca ggcgccgctt 2400
   cagccggaca accggcgtgt gaagctgcgg attttcgtgg acgattcctc gctggaggta 2460
   ttcggagggg atggccgcgt agtattttcg gaggtgattt ttcccgatcc tgcgcaccgt 2520
   gaaatgagcc tttttactgt aggtggagag gtgaacgtgg tttccttgaa agtgcacgca 2580
   ctaacaaacg tatggaacga ggcggcggag agccaaacct ccatcgtgat ggacacgagt 2640
   ccgcagcaaa tggggctggg agatacccgg accttgtatg cggcggtgaa cggcggcgcc 2700
   aaaggcgggc gcagcggcgg ttcggacggc aaccgaaatc agccgatccg tcgacctgca 2760
   gccaagctta tcgattgcac tatgcctaaa acaattatac gaaaagatcg cgaaactttc 2820
   aatatactct ga 2832